# EUROPEAN PATENT SPECIFICATION

(11) **EP 3 394 260 B1**
(45) Date of publication and mention of the grant of the patent: **17.02.2021**
(21) Application number: 16831921.8
(22) Date of filing: 22.12.2016
(51) Int. Cl.: C12N 15/113, C12N 15/90, A61K 48/00

(54) **MATERIALS AND METHODS FOR TREATMENT OF AMYOTROPHIC LATERAL SCLEROSIS AND/OR FRONTAL TEMPORAL LOBULAR DEGENERATION**
MATERIALIEN UND VERFAHREN ZUR BEHANDLUNG VON AMYOTHROPHISCHER LATERALER SKLEROSE UND/ODER FRONTOTEMPORALER DEMENZ
MATÉRIAUX ET PROCÉDÉS DE TRAITEMENT DE LA SCLÉROSE LATÉRALE AMYOTROPHIQUE ET/OU DE DÉGÉNÉRESCENCE LOBAIRE FRONTO-TEMPORALE

(30) Priority: 23.12.2015 US 201562387424 P; 18.04.2016 US 201662324281 P
(43) Date of publication of application: 31.10.2018
(73) Proprietor: CRISPR Therapeutics AG, CH-6300 (CH)
(72) Inventor: COWAN, Chad Albert, Cambridge, MA 02142 (US); KABADI, Ami Meda, Cambridge, MA 02142 (US); LUNDBERG, Ante Sven, Cambridge, MA 02142 (US)
(74) Representative: D Young & Co LLP
(86) International application number: PCT/IB2016/057958
(87) International publication number: WO 2017/109757

(56) References cited:
- WO-A1-2015/059265
- WO-A1-2015/089351
- WO-A1-2016/196185
- WO-A1-2018/064600
- WO-A2-2015/153760
- WO-A2-2016/011080
- WO-A2-2016/210372
- US-A1- 2011 023 144
- HRUSCHA ALEXANDER ET AL: "Efficient CRISPR/Cas9 genome editing with low off-target effects in zebrafish", DEVELOPMENT, THE COMPANY OF BIOLOGISTS LTD, GB, vol. 140, no. 24, 1 December 2013 (2013-12-01), pages 4982-4987, XP009177513, ISSN: 0950-1991, DOI: 10.1242/DEV.099085 [retrieved on 2013-11-20] & Alexander Hruscha ET AL: "Supplementary Material: Efficient CRISPR/Cas9 genome editing with low off-target effects in zebrafish", , 3 December 2013 (2013-12-03), XP055354021, Retrieved from the Internet: URL:http://dev.biologists.org/highwire/fil estream/1204063/field_highwire_adjunct_fil es/0/DEV099085.pdf [retrieved on 2017-03-13]
- C. A. ROSS ET AL: "Human-induced pluripotent stem cells: potential for neurodegenerative diseases", HUMAN MOLECULAR GENETICS, vol. 23, no. R1, 13 May 2014 (2014-05-13), pages R17-R26, XP055354140, gb ISSN: 0964-6906, DOI: 10.1093/hmg/ddu204
- GUNNAR HARGUS ET AL: "Human stem cell models of neurodegeneration: a novel approach to study mechanisms of disease development", ACTA NEUROPATHOLOGICA., vol. 127, no. 2, 1 February 2014 (2014-02-01), pages 151-173, XP055354137, BERLIN, DE ISSN: 0001-6322, DOI: 10.1007/s00401-013-1222-6
- HONG CHEN ET AL: "Modeling ALS with iPSCs Reveals that Mutant SOD1 Misregulates Neurofilament Balance in Motor Neurons", CELL STEM CELL, vol. 14, no. 6, 1 June 2014 (2014-06-01), pages 796-809, XP055354252, AMSTERDAM, NL ISSN: 1934-5909, DOI: 10.1016/j.stem.2014.02.004
- J Amick ET AL: "C9orf72 binds SMCR8, localizes to lysosomes, and regulates mTORC1 signaling", Molecular Biology of the Cell, 15 October 2016 (2016-10-15), pages 3040-3051, XP055354101, DOI: 10.1091/mbc.E16-01-0003 Retrieved from the Internet: URL:https://www.ncbi.nlm.nih.gov/pmc/artic les/PMC5063613/pdf/3040.pdf [retrieved on 2017-03-13]
- Joseph Amick ET AL: "Supplemental Materials for: C9orf72 binds SMCR8, localizes to lysosomes and regulates mTORC1 signaling", Molecular Biology of the Cell, 15 October 2016 (2016-10-15), XP055610218, Retrieved from the Internet: URL:https://www.ncbi.nlm.nih.gov/pmc/artic les/PMC5063613/bin/supp_E16-01-0003v1_supp lemental091616.pdf [retrieved on 2019-08-01]
- Luke a. Gilbert ET AL: "Genome-Scale CRISPR-Mediated Control of Gene Repression and Activation", CELL, vol. 159, no. 3, 23 October 2014 (2014-10-23), pages 647-661, XP055247644, AMSTERDAM, NL ISSN: 0092-8674, DOI: 10.1016/j.cell.2014.09.029
- Max A Horlbeck ET AL: "Compact and highly active next-generation libraries for CRISPR-mediated gene repression and activation", eLife, vol. 5, 23 September 2016 (2016-09-23), XP055610557, DOI: 10.7554/eLife.19760

## Description

### Field

The present application provides materials and methods for treating a patient with Amyotrophic Lateral Sclerosis (ALS) and/or Frontaltemporal Lobular Degeneration (FTLD), both *ex vivo* and *in vivo.* In addition, the present application provides materials and methods for editing to modulate the expression, function or activity of the C9ORF72 gene in a cell by genome editing.

### Background

Amyotrophic lateral sclerosis (ALS) is a fatal neurodegenerative disease characterized clinically by progressive paralysis leading to death from respiratory failure, typically within two to three years of symptom onset (Rowland and Shneider, N. Engl. J. Med., 2001, 344, 1688-1700). ALS is the third most common neurodegenerative disease in the Western world (Hirtz et al., Neurology, 2007, 68, 326-337). Approximately 10% of cases are familial in nature, whereas the bulk of patients diagnosed with the disease are classified as sporadic, as they appear to occur randomly throughout the population (Chio et al., Neurology, 2008, 70, 533-537). There is growing recognition, based on clinical, genetic, and epidemiological data that ALS and Frontotemporal Lobular Dementia represent an overlapping continuum of disease, characterized pathologically by the presence of TDP-43 positive inclusions throughout the central nervous system (Lillo and Hodges, J. Clin. Neurosci, 2009, 16, 1131-1135; Neumann et al., Science, 2006, 314, 130-133).

To date, a number of genes have been discovered as causative for classical familial ALS, for example, SOD1, TARDBP, FUS, OPTN, and VCP (Johnson et al., Neuron, 2010, 68, 857-864; Kwiatkowski et al., Science, 2009, 323, 1205-1208; Maruyama et al., Nature, 2010, 465, 223-226; Rosen et al., Nature, 1993, 362, 59-62; Sreedharan et al., Science, 2008, 319, 1668-1672; Vance et al., Brain, 2009, 129, 868-876). Over the past 10 years, linkage analysis of kindreds involving multiple cases of ALS, FTD, and ALS-FTD identified an important locus for the disease on the short arm of chromosome 9, which is now known as C9orf72 (Boxer et al., J. Neurol. Neurosurg. Psychiatry, 2011, 82, 196-203; Morita et al., Neurology, 2006, 66, 839-844; Pearson et al. J. Neurol., 2011, 258, 647-655; Vance et al., Brain, 2006, 129, 868-876).

Frontotemporal lobar degeneration (FTLD) is a type of neurodegenerative disease involving degeneration of gray matter in the frontal lobe and anterior portion of the temporal lobe of the cerebrum, with sparing of the parietal and occipital lobes. FTLD is the second most common form of dementia after Alzheimer's disease, and is therefore a major cause of neurological problems in the elderly. The syndrome of FTLD encompasses the clinical subgroups of frontotemporal dementia (FTD), FTD with motor neuron disease (MND), semantic dementia and primary progressive aphasia, and is characterized by changes in behavior, personality and language, with relative preservation of memory and perception.

Pathologically, there are two main histological profiles associated with FTLD. One of these is tauopathy, the accumulation of hyperphosphorylated tau in neurons, and occasionally in glia. However, the most common neuropathology associated with FTLD, accounting for well over half of all cases, is that known as FTLD-U, in which there are neuronal cytoplasmic inclusions and neurites that are immunoreactive for ubiquitin (ub-ir), but not for tau. FTLD pathology of this type was first described in patients with motor neuron disease (MND) and dementia, but has subsequently been recognized as a common neuropathological feature of FTLD in patients without motor symptoms. This ub-ir pathology is characteristically found in granule cells of dentate fascia of the hippocampus and in neurons of layer 2 of the frontal and temporal neocortex.

Currently, there is only one FDA approved drug on the market for the treatment of ALS, Rilutek (riluzole). However, the mechanism of action is poorly understood. The drug is thought to slow the disease's progression in some people by reducing levels of glutamate in the brain.

C9orf72 (chromosome 9 open reading frame 72) is a protein which, in humans, is encoded by the gene C9ORF72. The human C9ORF72 gene is located on the short (p) arm of chromosome 9 open reading frame 72, from base pair 27,546,542 to base pair 27,573,863. Its cytogenetic location is at 9p21.2. The protein is found in many regions of the brain, in the cytoplasm of neurons, as well as in presynaptic terminals. Disease causing mutations in the gene were first discovered by two independent research teams in 2011 (DeJesus-Hernandez et al. (2011) Neuron 72 (2): 245-56; Renton et al. (2011). Neuron 72 (2): 257-68). The mutation in C9ORF72 is significant because it is the first pathogenic mechanism identified to be a genetic link between FTLD and ALS. As of 2012, it is the most common mutation identified that is associated with familial FTLD and/or ALS.

The mutation of C9ORF72 is a hexanucleotide repeat expansion of the six letter string of nucleotides GGGGCC. In healthy individuals, there are few repeats of this hexanucleotide, typically 30, but in people with the diseased phenotype, the repeat can occur in the order of hundreds (Fong et al. (2012) Alzheimers Res Ther 4 (4): 27). The hexanucleotide expansion event in the C9ORF72 gene is present in approximately 40% of familial ALS and 8-10% of sporadic ALS patients. The hexanucleotide expansion occurs in an alternatively spliced Intron 1 of the C9ORF72 gene, and as such does not alter the coding sequence or resulting protein. Three alternatively spliced variants of C9ORF72 (V1, V2 and V3) are normally produced. The expanded nucleotide repeat has been shown to reduce the transcription of V1, however the total amount of protein produced was unaffected (DeJesus-Hernandez et al. (2011), Neuron 72 (2): 245-56). Overall, reduced protein levels of C9ORF72 have been observed in brain autopsies from ALS patients (Waite (2014) Neurobiol Aging, 35 1779 e1775-1779 e1713) suggesting haploinsufficiency as a cause of ALS/FTD. However, the physiological function of the C9orf72 protein is poorly understood and neuron specific knockout mice develop no disease like pathology (Koppers (2015), Ann Neurol, 78:426-438) making it unlikely that haploinsufficiency significantly contributes to the disease mechanism.

In addition to haploinsufficiency, there are other theories about the way in which the C9ORF72 hexanucleotide expansion causes FTD and/or ALS. Another theory is that accumulation of GC rich RNA in the nucleus and cytoplasm becomes toxic, and RNA binding protein sequestration occurs. A common feature of non-coding repeat expansion disorders, which has gained increased attention in recent years, is the accumulation of RNA fragments composed of the repeated nucleotides as RNA foci in the nucleus and/or cytoplasm of affected cells (Todd and Paulson, 2010, Ann. Neurol. 67, 291-300). In several disorders, the RNA foci have been shown to sequester RNA-binding proteins, leading to dysregulation of alternative mRNA splicing. A hallmark of such RNA foci is TDP-43 positive inclusions throughout the central nervous system (Lillo and Hodges, J. Clin. Neurosci, 2009, 16, 1131-1135; Neumann et al., Science, 2006, 314, 130-133).

An additional theory is that RNA transcribed from the C9ORF72 gene containing expanded hexanucleotide repeats is translated through a non-ATG initiated mechanism. This drives the formation and accumulation of dipeptide repeat proteins corresponding to multiple ribosomal reading frames on the mutation. The repeat is translated into dipeptide repeat (DPR) proteins that cause repeat-induced toxicity. DPRs inhibit the proteasome and sequester other proteins. GGGGCC repeat expansion in C9ORF72 may compromise nucleocytoplasmic transport through several possible mechanisms (Edbauer, Current Opinion in Neurobiology 2016, 36:99-106).

Traditionally, familial and sporadic cases of ALS have been clinically indistinguishable, which has made diagnosis difficult. The identification of this gene will therefore help in the future diagnosis of familial ALS. Slow diagnosis is also common for FTD, which can often take up to a year with many patients initially misdiagnosed with another condition. Testing for a specific gene that is known to cause the diseases would help with faster diagnoses. Most importantly, this hexanucleotide repeat expansion is an extremely promising future target for developing therapies to treat both familial FTD and familial ALS.

Genome engineering refers to the strategies and techniques for the targeted, specific modification of the genetic information (genome) of living organisms. Genome engineering is a very active field of research because of the wide range of possible applications, particularly in the areas of human health; the correction of a gene carrying a harmful mutation, for example, or to explore the function of a gene. Early technologies developed to insert a transgene into a living cell were often limited by the random nature of the insertion of the new sequence into the genome. Random insertions into the genome may result in disrupting normal regulation of neighboring genes leading to severe unwanted effects. Furthermore, random integration technologies offer little reproducibility, as there is no guarantee that the sequence would be inserted at the same place in two different cells. Recent genome engineering strategies, such as ZFNs, TALENs, HEs and MegaTALs, enable a specific area of the DNA to be modified, thereby increasing the precision of the correction or insertion compared to early technologies. These newer platforms offer a much larger degree of reproducibility.

Despite efforts from researchers and medical professionals worldwide who have been trying to address ALS and FTLD, and despite the promise of genome engineering approaches, there still remains a critical need for developing safe and effective treatments for ALS and FTLD.

Currently, all therapies for addressing ALS and FTLD aim at slowing the progression of the disease, rather than curing the disease. In contrast, the present invention presents an approach to correct the genetic basis of the disease. By using genome engineering tools to create permanent changes to the genome that can restore the C9ORF72 hexanucleotide repeat expansion with a single treatment, the resulting therapy should stop the disease progression completely.

Amick et al., 2016, Molecular Biology of the Cell, 27(20), pages 3040-3051 relates to genome-editing strategies to investigate C9orf72 interactions, subcellular localization, and knockout (KO) phenotypes.

WO 2016/196185 A1 relates to a non-human animal model for neurodegenerative and/or inflammatory diseases, which non-human animal comprises a disruption in a C9ORF72 locus.

WO 2016/011080 A2 relates to methods, compositions, and kits for CRISPR/Cas mediated transcriptional modulation.

Gilbert et al., 2014, Cell, 159(3), pages 647-661 relates to genome-scale CRISPR-mediated control of gene repression and activation.

Horlbeck et al., 2016, eLife, 5:e19760 relates to compact next-generation libraries for CRISPR-mediated gene repression and activation.

### Summary

The invention provides an *ex vivo* method for editing the C9ORF72 gene in a human cell by genome editing comprising introducing into the cell one or more Cas9 endonucleases and one or more guide ribonucleic acids (gRNAs) to effect one or more single-strand breaks (SSBs) or double-strand breaks (DSBs) within or near the C9ORF72 gene or other DNA sequences that encode regulatory elements of the C9ORF72 gene that results in permanent deletion or correction of an expanded hexanucleotide repeat within or near the C9ORF72 gene and results in restoration of C9ORF72 protein activity, wherein the one or more gRNAs comprise a spacer sequence selected from the group consisting of SEQ ID NOs 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558, and 3592, and wherein the gRNAs induce a cutting efficiency of 80% or higher.

The invention also provides a composition comprising one or more Cas9 endonucleases and one or more guide ribonucleic acids (gRNAs) for use in a therapeutic method for editing the C9ORF72 gene in a human cell comprising introducing the composition into the cell to effect one or more single-strand breaks (SSBs) or double-strand breaks (DSBs) within or near the C9ORF72 gene or other DNA sequences that encode regulatory elements of the C9ORF72 gene that results in permanent deletion or correction of an expanded hexanucleotide repeat within or near the C9ORF72 gene and results in restoration of C9ORF72 protein activity, wherein the one or more gRNAs comprise a spacer sequence selected from the group consisting of SEQ ID NOs 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558, and 3592, and wherein the gRNAs induce a cutting efficiency of 80% or higher.

The invention also provides one or more guide ribonucleic acids (gRNAs) comprising a spacer sequence selected from SEQ ID NOs: 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558, and 3592.

The invention also provides the one or more gRNAs of the invention for use in treating amyotrophic lateral sclerosis (ALS) or frontotemporal lobular dementia (FTLD) in a subject by editing the C9ORF72 gene in a cell from the subject.

Disclosed herein are cellular, *ex vivo* and *in vivo* methods for creating permanent changes to the genome by deleting, inserting or correcting the expanded hexanucleotide repeat within or near the C9ORF72 gene or other DNA sequences that encode regulatory elements of the C9ORF72 gene or knocking in C9ORF72 cDNA or minigene into a safe harbor locus by genome editing and restoring C9orf72 protein activity, which can be used to treat amyotrophic lateral sclerosis (ALS) or frontotemporal lobular dementia (FTLD), as well as components, kits and compositions for performing such methods, and cells produced by them.

Disclosed herein is a method for editing the C9ORF72 gene in a human cell by genome editing, the method comprising the step of introducing into the human cell one or more deoxyribonucleic acid (DNA) endonucleases to effect one or more single-strand breaks (SSBs) or one or more double-strand breaks (DSBs) within or near the C9ORF72 gene or other DNA sequences that encode regulatory elements of the C9ORF72 gene that results in at least one of a permanent insertion, correction, or modulation of expression or function of one or more mutations or exons within or near or affecting the expression or function of the C9ORF72 gene, or within or near a safe harbor locus that results in a permanent insertion of the C9ORF72 gene and results in restoration of C9orf72 protein activity.

Also disclosed herein is a method for inserting the C9ORF72 gene in a human cell by genome editing, the method comprising the step of: introducing into the human cell one or more deoxyribonucleic acid (DNA) endonucleases to effect one or more single-strand breaks (SSBs) or double-strand breaks (DSBs) within or near a safe harbor locus that results in a permanent insertion of the C9ORF72 gene or minigene, and results in restoration of C9orf72 protein activity.

In one aspect, disclosed herein is a method for editing the C9ORF72 gene in a human cell by genome editing comprising introducing into the cell one or more deoxyribonucleic acid (DNA) endonucleases to effect one or more double-strand breaks (DSBs) within or near the C9ORF72 gene or other DNA sequences that encode regulatory elements of the C9ORF72 gene that results in permanent deletion, insertion, or correction of the expanded hexanucleotide repeat within or near the C9ORF72 gene or within or near a safe harbor locus that results in permanent insertion of the C9ORF72 gene or minigene, and restoration of C9orf72 protein activity.

In another aspect, disclosed herein is an *ex vivo* method for treating a patient with amyotrophic lateral sclerosis (ALS) or frontotemporal lobular dementia (FTLD) comprising the steps of: creating a patient specific induced pluripotent stem cell (iPSC); editing within or near the C9ORF72 gene of the iPSC or other DNA sequences that encode regulatory elements of the C9ORF72 gene of the iPSC or editing within or near a safe harbor locus of the iPSC; differentiating the genome edited iPSC into a hematopoietic progenitor cell, a neural progenitor cell, or a neural cell; and implanting the hematopoietic progenitor cell, neural progenitor cell, or neural cell into the patient.

In some embodiments, the step of creating a patient specific induced pluripotent stem cell comprises: isolating a somatic cell from the patient; and introducing a set of pluripotency-associated genes into the somatic cell to induce the cell to become a pluripotent stem cell. In some embodiments, the somatic cell is a fibroblast. In some embodiments, the set of pluripotency-associated genes is one or more of the genes selected from the group consisting of OCT4, SOX2, KLF4, Lin28, NANOG and cMYC.

The step of editing within or near the C9ORF72 gene or other DNA sequences that encode regulatory elements of the C9ORF72 gene or of editing within or near a safe harbor locus or of editing within or near a locus of the first exon of the C9ORF72 gene can comprise introducing into the iPSC one or more deoxyribonucleic acid (DNA) endonucleases to effect one or more single-strand breaks (SSBs) or double-strand breaks (DSBs) within or near the C9ORF72 gene or other DNA sequences that encode regulatory elements of the C9ORF72 gene that results in a permanent insertion, correction, or modulation of expression or function of one or more mutations or exons within or near or affecting the expression or function of the C9ORF72 gene or within or near a safe harbor locus that results in a permanent insertion of the C9ORF72 gene and results in restoration of C9orf72 protein activity. The safe harbor locus can be selected from the group consisting of: AAVS1 (PPP1 R12C), ALB, Angptl3, ApoC3, ASGR2, CCR5, FIX (F9), G6PC, Gys2, HGD, Lp(a), Pcsk9, Serpina1, TF, and TTR. The safe harbor locus can be selected from the group consisting of: exon 1-2 of AAVS1 (PPP1 R12C), exon 1-2 of ALB, exon 1-2 of Angptl3, exon 1-2 of ApoC3, exon 1-2 of ASGR2, exon 1-2 of CCR5, exon 1-2 of FIX (F9), exon 1-2 of G6PC, exon 1-2 of Gys2, exon 1-2 of HGD, exon 1-2 of Lp(a), exon 1-2 of Pcsk9, exon 1-2 of Serpina1, exon 1-2 of TF, and exon 1-2 of TTR.

In some embodiments, the step of editing the C9ORF72 gene of the iPSC comprises introducing into the iPSC one or more deoxyribonucleic acid (DNA) endonucleases to effect one or more double-strand breaks (DSBs) within or near the C9ORF72 gene that results in permanent deletion, insertion, or correction of the expanded hexanucleotide repeat within or near the C9ORF72 gene.

In some embodiments, the step of differentiating the genome edited iPSC into hematopoietic progenitor cell, a neural progenitor cell, or neural cell comprises one or more of the following: treatment with a combination of small molecules or delivery of master transcription factors.

In some embodiments, the step of implanting the hematopoietic progenitor cell, neural progenitor cell, or neural cell into the patient comprises implanting the hematopoietic progenitor cell, neural progenitor cell, or neural cell into the patient by transplantation, local injection, systemic infusion, or combinations thereof.

In another aspect, disclosed herein is an *ex vivo* method for treating a patient with amyotrophic lateral sclerosis (ALS) or frontotemporal lobular dementia (FTLD) comprising the steps of: isolating a white blood cell from the patient; editing the C9ORF72 gene of the white blood cell; and implanting the edited white blood cell into the patient.

In some embodiments, step of isolating a white blood cell comprises: cell differential centrifugation and cell culturing.

The step of editing within or near the C9ORF72 gene or other DNA sequences that encode regulatory elements of the C9ORF72 gene or of editing within or near a safe harbor locus or of editing within or near a locus of the first exon of the C9ORF72 gene can comprise introducing into the white blood cell one or more deoxyribonucleic acid (DNA) endonucleases to effect one or more single-strand breaks (SSBs) or double-strand breaks (DSBs) within or near the C9ORF72 gene or other DNA sequences that encode regulatory elements of the C9ORF72 gene that results in a permanent insertion, correction, or modulation of expression or function of one or more mutations or exons within or near or affecting the expression or function of the C9ORF72 gene or within or near a safe harbor locus that results in a permanent insertion of the C9ORF72 gene and results in restoration of C9orf72 protein activity. The safe harbor locus can be selected from the group consisting of: AAVS1 (PPP1 R12C), ALB, Angptl3, ApoC3, ASGR2, CCR5, FIX (F9), G6PC, Gys2, HGD, Lp(a), Pcsk9, Serpina1, TF, and TTR. The safe harbor locus can be selected from the group consisting of: exon 1-2 of AAVS1 (PPP1 R12C), exon 1-2 of ALB, exon 1-2 of Angptl3, exon 1-2 of ApoC3, exon 1-2 of ASGR2, exon 1-2 of CCR5, exon 1-2 of FIX (F9), exon 1-2 of G6PC, exon 1-2 of Gys2, exon 1-2 of HGD, exon 1-2 of Lp(a), exon 1-2 of Pcsk9, exon 1-2 of Serpina1, exon 1-2 of TF, and exon 1-2 of TTR.

In some embodiments, the step of editing the C9ORF72 gene of the neural cell comprises introducing into the neural cell one or more deoxyribonucleic acid (DNA) endonucleases to effect one or more double-strand breaks (DSBs) within or near the C9ORF72 gene that results in permanent deletion, insertion, or correction of the expanded hexanucleotide repeat within or near the C9ORF72 gene.

In some embodiments, the step of implanting the white blood cell into the patient comprises implanting the white blood cell into the patient by transplantation, local injection, systemic infusion, or combinations thereof.

In an aspect, disclosed herein is an *ex vivo* method for treating a patient with amyotrophic lateral sclerosis (ALS) or frontotemporal lobular dementia (FTLD) comprising the steps of: optionally, performing a biopsy of the patient's bone marrow; isolating a mesenchymal stem cell; editing within or near the C9ORF72 gene of the stem cell or other DNA sequences that encode regulatory elements of the C9ORF72 gene or editing within or near a safe harbor locus of the C9ORF72 gene; differentiating the stem cell into a hematopoietic progenitor cell, neural progenitor cell, or neural cell; and implanting the hematopoietic progenitor cell, neural progenitor cell, or neural cell into the patient.

In some embodiments, the step of isolating a mesenchymal stem cell comprises: aspiration of bone marrow and isolation of mesenchymal cells by density centrifugation using Percoll™.

The step of editing within or near the C9ORF72 gene or other DNA sequences that encode regulatory elements of the C9ORF72 gene or of editing within or near a safe harbor locus or of editing within or near a locus of the first exon of the C9ORF72 gene can comprise introducing into the mesenchymal stem cell one or more deoxyribonucleic acid (DNA) endonucleases to effect one or more single-strand breaks (SSBs) or double-strand breaks (DSBs) within or near the C9ORF72 gene or other DNA sequences that encode regulatory elements of the C9ORF72 gene that results in a permanent insertion, correction, or modulation of expression or function of one or more mutations or exons within or near or affecting the expression or function of the C9ORF72 gene or within or near a safe harbor locus that results in a permanent insertion of the C9ORF72 gene and results in restoration of C9orf72 protein activity. The safe harbor locus can be selected from the group consisting of: AAVS1 (PPP1 R12C), ALB, Angptl3, ApoC3, ASGR2, CCR5, FIX (F9), G6PC, Gys2, HGD, Lp(a), Pcsk9, Serpina1, TF, and TTR. The safe harbor locus can be selected from the group consisting of: exon 1-2 of AAVS1 (PPP1 R12C), exon 1-2 of ALB, exon 1-2 of Angptl3, exon 1-2 of ApoC3, exon 1-2 of ASGR2, exon 1-2 of CCR5, exon 1-2 of FIX (F9), exon 1-2 of G6PC, exon 1-2 of Gys2, exon 1-2 of HGD, exon 1-2 of Lp(a), exon 1-2 of Pcsk9, exon 1-2 of Serpina1, exon 1-2 of TF, and exon 1-2 of TTR.

In some embodiments, the step of editing the C9ORF72 gene of the stem cell comprises introducing into the stem cell one or more deoxyribonucleic acid (DNA) endonucleases to effect one or more double-strand breaks (DSBs) within or near the C9ORF72 gene that results in permanent deletion, insertion, or correction of the expanded hexanucleotide repeat within or near the C9ORF72 gene.

In some embodiments, the step of differentiating the stem cell into a hematopoietic progenitor cell, neural progenitor cell, or neural cell comprises one or more of the following to differentiate the genome edited stem cell into a neural cell: treatment with a combination of small molecules or delivery of master transcription factors.

In some embodiments, step of implanting the cell into the patient comprises implanting the cell into the patient by transplantation, local injection, systemic infusion, or combinations thereof.

In another aspect, disclosed herein is an *ex vivo* method for treating a patient with amyotrophic lateral sclerosis (ALS) or frontotemporal lobular dementia (FTLD) comprising the steps of: optionally treating the patient with granulocyte colony stimulating factor (GCSF); isolating a hematopoietic progenitor cell from the patient; editing within or near the C9ORF72 gene of the hematopoietic progenitor cell or other DNA sequences that encode regulatory elements of the C9ORF72 gene or editing within or near a safe harbor locus of the C9ORF72 gene of the hematopoietic progenitor cell; and implanting the cell into the patient.

In some embodiments, the step of treating the patient with granulocyte colony stimulating factor (GCSF) is performed in combination with Plerixaflor.

In some embodiments, the step of isolating a hematopoietic progenitor cell from the patient comprises isolating CD34+ cells.

The step of editing within or near the C9ORF72 gene or other DNA sequences that encode regulatory elements of the C9ORF72 gene or of editing within or near a safe harbor locus or of editing within or near a locus of the first exon of the C9ORF72 gene can comprise introducing into the hematopoietic progenitor cell one or more deoxyribonucleic acid (DNA) endonucleases to effect one or more single-strand breaks (SSBs) or double-strand breaks (DSBs) within or near the C9ORF72 gene or other DNA sequences that encode regulatory elements of the C9ORF72 gene that results in a permanent insertion, correction, or modulation of expression or function of one or more mutations or exons within or near or affecting the expression or function of the C9ORF72 gene or within or near a safe harbor locus that results in a permanent insertion of the C9ORF72 gene and results in restoration of C9orf72 protein activity. The safe harbor locus can be selected from the group consisting of: AAVS1 (PPP1 R12C), ALB, Angptl3, ApoC3, ASGR2, CCR5, FIX (F9), G6PC, Gys2, HGD, Lp(a), Pcsk9, Serpina1, TF, and TTR. The safe harbor locus can be selected from the group consisting of: exon 1-2 of AAVS1 (PPP1 R12C), exon 1-2 of ALB, exon 1-2 of Angptl3, exon 1-2 of ApoC3, exon 1-2 of ASGR2, exon 1-2 of CCR5, exon 1-2 of FIX (F9), exon 1-2 of G6PC, exon 1-2 of Gys2, exon 1-2 of HGD, exon 1-2 of Lp(a), exon 1-2 of Pcsk9, exon 1-2 of Serpina1, exon 1-2 of TF, and exon 1-2 of TTR.

In some embodiments, the step of editing the C9ORF72 gene of the hematopoietic progenitor cell comprises introducing into the stem cell one or more deoxyribonucleic acid (DNA) endonucleases to effect one or more double-strand breaks (DSBs) within or near the C9ORF72 gene or other DNA sequences that encode regulatory elements of the C9ORF72 gene or editing within or near a safe harbor locus of the C9ORF72 gene that results in permanent deletion, insertion, or correction of the expanded hexanucleotide repeat within or near the C9ORF72 gene and restoration of C9orf72 protein activity.

In some embodiments, the step of implanting the cell into the patient comprises implanting the neural cell into the patient by transplantation, local injection, systemic infusion, or combinations thereof.

In another aspect, disclosed herein is an *in vivo* method for treating a patient with amyotrophic lateral sclerosis (ALS) or frontotemporal lobular dementia (FTLD) comprising the step of editing the C9ORF72 gene in a cell of the patient.

The step of editing within or near the C9ORF72 gene or other DNA sequences that encode regulatory elements of the C9ORF72 gene or of editing within or near a safe harbor locus or of editing within or near a locus of the first exon of the C9ORF72 gene can comprise introducing into the cell one or more deoxyribonucleic acid (DNA) endonucleases to effect one or more single-strand breaks (SSBs) or double-strand breaks (DSBs) within or near the C9ORF72 gene or other DNA sequences that encode regulatory elements of the C9ORF72 gene that results in a permanent insertion, correction, or modulation of expression or function of one or more mutations or exons within or near or affecting the expression or function of the C9ORF72 gene or within or near a safe harbor locus that results in a permanent insertion of the C9ORF72 gene and results in restoration of C9orf72 protein activity. The safe harbor locus can be selected from the group consisting of: AAVS1 (PPP1 R12C), ALB, Angptl3, ApoC3, ASGR2, CCR5, FIX (F9), G6PC, Gys2, HGD, Lp(a), Pcsk9, Serpina1, TF, and TTR. The safe harbor locus can be selected from the group consisting of: exon 1-2 of AAVS1 (PPP1 R12C), exon 1-2 of ALB, exon 1-2 of Angptl3, exon 1-2 of ApoC3, exon 1-2 of ASGR2, exon 1-2 of CCR5, exon 1-2 of FIX (F9), exon 1-2 of G6PC, exon 1-2 of Gys2, exon 1-2 of HGD, exon 1-2 of Lp(a), exon 1-2 of Pcsk9, exon 1-2 of Serpina1, exon 1-2 of TF, and exon 1-2 of TTR.

In some embodiments, the step of editing the C9ORF72 gene in a cell of the patient comprises introducing into the cell one or more deoxyribonucleic acid (DNA) endonucleases to effect one or more double-strand breaks (DSBs) within or near the C9ORF72 gene that results in permanent deletion, insertion, or correction of the expanded hexanucleotide repeat within or near the C9ORF72 gene.

In some *in vivo* embodiments, the cell is a neural cell, a bone marrow cell, a hematopoietic progenitor cell, or a CD34+ cell.

In some embodiments, the one or more DNA endonucleases is a Cas1, Cas1B, Cas2, Cas3, Cas4, Cas5, Cas6, Cas7, Cas8, Cas9 (also known as Csn1 and Csx12), Cas100, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, or Cpf1 endonuclease; or a homolog, codon-optimized, or modified version thereof.

In some embodiments, the method comprises introducing into the cell one or more polynucleotides encoding the one or more DNA endonucleases. In some embodiments, the method comprises introducing into the cell one or more ribonucleic acids (RNAs) encoding the one or more DNA endonucleases. In some embodiments, the one or more polynucleotides or one or more RNAs is one or more modified polynucleotides or one or more modified RNAs.

In some embodiments, the method further comprises introducing into the cell one or more DNA endonucleases, wherein the DNA endonuclease is a protein or polypeptide.

In some embodiments, the method further comprises introducing into the cell one or more guide ribonucleic acids (gRNAs). In some embodiments, the one or more gRNAs are single-molecule guide RNA (sgRNAs). In some embodiments, the one or more gRNAs or one or more sgRNAs is one or more modified gRNAs or one or more modified sgRNAs.

In some embodiments, the one or more DNA endonucleases is pre-complexed with one or more gRNAs or one or more sgRNAs.

In some embodiments, the method further comprises introducing into the cell a polynucleotide donor template comprising a part of the wild-type C9ORF72 gene or cDNA. In some embodiments, the part of the wild-type C9ORF72 gene or cDNA is the entire C9ORF72 gene or cDNA, or the cDNA of natural Variant 1, Variant 2, or Variant 3. In some embodiments, the donor template is either single or double stranded. In some embodiments, the donor template has homologous arms to the 9p21.2 region.

In some embodiments, the method further comprises introducing into the cell one guide ribonucleic acid (gRNA) and a polynucleotide donor template comprising at least a portion of the wild-type C9ORF72 gene. In some embodiments, the method further comprises introducing into the cell one guide ribonucleic acid (gRNA) and a polynucleotide donor template comprising at least a portion of a codon-optimized or modified C9ORF72 gene. In some embodiments, the one or more DNA endonucleases is one or more Cas9 or Cpf1 endonucleases that effect one single-strand break (SSB) or one double-strand break (DSB) at a locus within or near the C9ORF72 gene or other DNA sequences that encode regulatory elements of the C9ORF72 gene that facilitates insertion of a new sequence from the polynucleotide donor template into the chromosomal DNA at the DSB locus that results in permanent insertion or correction of a part of the chromosomal DNA of the C9ORF72 gene proximal to the locus or safe harbor locus, and wherein the gRNA comprises a spacer sequence that is complementary to a segment of the locus or safe harbor locus and restoration of C9orf72 protein activity. In some embodiments, proximal means nucleotides both upstream and downstream of the DSB locus.

In some embodiments, the method further comprises introducing into the cell two guide ribonucleic acid (gRNAs) and a polynucleotide donor template comprising at least a portion of the wild-type C9ORF72 gene, and wherein the one or more DNA endonucleases is two or more Cas9 or Cpf1 endonucleases that effect or create at least two (e.g., a pair) of single-strand breaks (SSBs) or a pair of double-strand breaks (DSBs), the first at a 5' locus and the second at a 3' locus, within or near the C9ORF72 gene or other DNA sequences that encode regulatory elements of the C9ORF72 gene or within or near a safe harbor locus that facilitates insertion of a new sequence from the polynucleotide donor template into the chromosomal DNA between the 5' locus and the 3' locus that results in permanent insertion or correction of the chromosomal DNA between the 5' locus and the 3' locus within or near the C9ORF72 gene or other DNA sequences that encode regulatory elements of the C9ORF72 gene or within or near a safe harbor locus, and wherein the first guide RNA comprises a spacer sequence that is complementary to a segment of the 5' locus and the second guide RNA comprises a spacer sequence that is complementary to a segment of the 3' locus.

In some embodiments, the one or two gRNAs are one or two single-molecule guide RNA (sgRNAs). In some embodiments, the one or two gRNAs or one or two sgRNAs is one or two modified gRNAs or one or two modified sgRNAs.

In some embodiments, the one or more DNA endonucleases is pre-complexed with one or two gRNAs or one or two sgRNAs.

In some embodiments, the part of the wild-type C9ORF72 gene or cDNA is the entire C9ORF72 gene or cDNA; the cDNA of natural Variant 1, Variant 2, or Variant 3; or about 30 hexanucleotide repeats.

In some embodiments, the donor template is either single or double stranded. In some embodiments, the donor template has homologous arms to the 9p21.2 region.

In some embodiments, the DSB, or 5' DSB and 3' DSB are in the first intron of the C9ORF72 gene.

The donor template can be either single or double stranded polynucleotide. The donor template can have homologous arms to a safe harbor locus, such as, for example, exon 1-2 of AAVS1 (PPP1R12C), exon 1-2 of ALB, exon 1-2 of Angptl3, exon 1-2 of ApoC3, exon 1-2 of ASGR2, exon 1-2 of CCR5, exon 1-2 of FIX (F9), exon 1-2 of G6PC, exon 1-2 of Gys2, exon 1-2 of HGD, exon 1-2 of Lp(a), exon 1-2 of Pcsk9, exon 1-2 of Serpina1, exon 1-2 of TF, and exon 1-2 of TTR.

In some embodiments, the insertion or correction is by homology directed repair (HDR).

In some embodiments, the method further comprises introducing into the cell two guide ribonucleic acid (gRNAs), and wherein the one or more DNA endonucleases is two or more Cas9 or Cpf1 endonucleases that effect a pair of double-strand breaks (DSBs), the first at a 5' DSB locus and the second at a 3' DSB locus, within or near the C9ORF72 gene that causes a deletion of the chromosomal DNA between the 5' DSB locus and the 3' DSB locus that results in permanent deletion of the chromosomal DNA between the 5' DSB locus and the 3' DSB locus within or near the C9ORF72 gene, and wherein the first guide RNA comprises a spacer sequence that is complementary to a segment of the 5' DSB locus and the second guide RNA comprises a spacer sequence that is complementary to a segment of the 3' DSB locus.

In some embodiments, the two gRNAs are two single-molecule guide RNA (sgRNAs). In some embodiments, the two gRNAs or two sgRNAs are two modified gRNAs or two modified sgRNAs.

In some embodiments, the one or more DNA endonucleases is pre-complexed with one or two gRNAs or one or two sgRNAs.

In some embodiments, both the 5' DSB and 3' DSB are in or near either the first exon, first intron, or second exon of the C9ORF72 gene.

In some embodiments, the deletion is a deletion of the expanded hexanucleotide repeat.

In some embodiments, the Cas9 or Cpf1 mRNA, gRNA, and donor template are either each formulated separately into lipid nanoparticles or all co-formulated into a lipid nanoparticle.

In some embodiments, the Cas9 or Cpf1 mRNA, gRNA, and donor template are formulated into separate exosomes or are co-formulated into an exosome.

In some embodiments, the Cas9 or Cpf1 mRNA is formulated into a lipid nanoparticle, and both the gRNA and donor template are delivered to the cell by a viral vector. In some embodiments, the viral vector is an adeno-associated virus (AAV) vector. In some embodiments, the AAV vector is an AAV6 vector.

The Cas9 or Cpf1 mRNA can be formulated into a lipid nanoparticle, and the gRNA can be delivered to the cell by electroporation and donor template can be delivered to the cell by a viral vector. The viral vector can be an adeno-associated virus (AAV) vector. The AAV vector can be an AAV6 vector.

In some embodiments, the C9ORF72 gene is located on Chromosome 9: 27,546,542 - 27,573,863 (Genome Reference Consortium - GRCh38/hg38).

In some embodiments, the restoration of C9orf72 protein activity is compared to wild-type or normal C9orf72 protein activity.

In another aspect, disclosed herein is one or more guide ribonucleic acids (gRNAs) comprising a spacer sequence selected from the group consisting of the nucleic acid sequences in SEQ ID NOs: 1-18807 for editing the C9ORF72 gene in a cell from a patient with amyotrophic lateral sclerosis (ALS) or frontotemporal lobular dementia (FTLD). In some embodiments, the one or more gRNAs are one or more single-molecule guide RNAs (sgRNAs). In some embodiments, the one or more gRNAs or one or more sgRNAs is one or more modified gRNAs or one or more modified sgRNAs.

Also disclosed herein is one or more guide ribonucleic acids (gRNAs) comprising a spacer sequence selected from the group consisting of the nucleic acid sequences in SEQ ID NOs: 18810-73668 for editing a safe harbor locus in a cell from a patient with amyotrophic lateral sclerosis (ALS) or frontotemporal lobular dementia (FTLD), wherein the safe harbor locus is selected from the group consisting of AAVS1 (PPP1 R12C), ALB, Angptl3, ApoC3, ASGR2, CCR5, FIX (F9), G6PC, Gys2, HGD, Lp(a), Pcsk9, Serpina1, TF, and TTR. In some embodiments, the one or more gRNAs are one or more single-molecule guide RNAs (sgRNAs). In some embodiments, the one or more gRNAs or one or more sgRNAs is one or more modified gRNAs or one or more modified sgRNAs.

Also disclosed herein are cells that have been modified by the preceding methods to permanently correct one or more mutations within the C9ORF72 gene, and restore C9orf72 protein activity. Further disclosed herein are methods for ameliorating amyotrophic lateral sclerosis (ALS) or frontotemporal lobular dementia (FTLD) by the administration of cells that have been modified by the preceding methods to an ALS or FTLD patient.

In some embodiments, the methods and compositions of the disclosure comprise one or more modified guide ribonucleic acids (gRNAs). Non-limiting examples of modifications can comprise one or more nucleotides modified at the 2' position of the sugar, in some embodiments a 2'-O-alkyl, 2'-O-alkyl-O-alkyl, or 2'-fluoro-modified nucleotide. In some embodiments, RNA modifications include 2'-fluoro, 2'-amino or 2' O-methyl modifications on the ribose of pyrimidines, abasic residues, desoxy nucleotides, or an inverted base at the 3' end of the RNA.

In some embodiments, the one or more modified guide ribonucleic acids (gRNAs) comprise a modification that makes the modified gRNA more resistant to nuclease digestion than the native oligonucleotide. Non-limiting examples of such modifications include those comprising modified backbones, for example, phosphorothioates, phosphorothyos, phosphotriesters, methyl phosphonates, short chain alkyl or cycloalkyl intersugar linkages or short chain heteroatomic or heterocyclic intersugar linkages.

In another aspect, disclosed herein is a method for treating a patient with amyotrophic lateral sclerosis (ALS) or frontotemporal lobular dementia (FTLD) comprising transplanting the bone marrow from a donor to the patient.

Various other aspects are described and exemplified herein.

### Brief Description of the Drawings

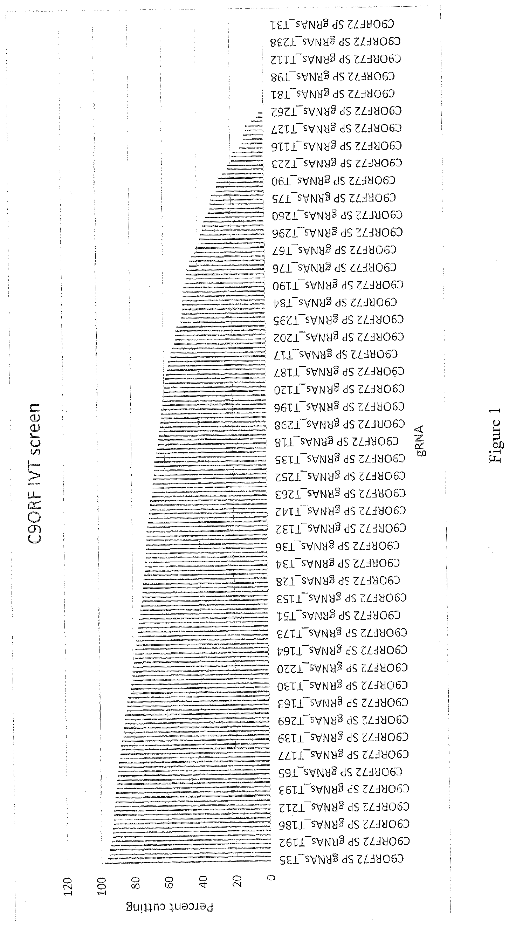

Figure 1 is a graph showing the percent cutting of the guide RNAs subjected to tracking of indels by decomposition (TIDE) analysis.

### Brief Description of the Sequence Listing

SEQ ID NOs: 1-6148 are 20 bp spacer sequences for targeting the C9ORF72 gene with a *S. pyogenes* Cas9 endonuclease.

SEQ ID NOs: 6149-6892 are 20 bp spacer sequences for targeting the C9ORF72 gene with a *S. aureus* Cas9 endonuclease.

SEQ ID NOs: 7760-8097 are 20 bp spacer sequences for targeting the C9ORF72 gene with a *S. thermophilus* Cas9 endonuclease.

SEQ ID NOs: 8098-8261 are 20 bp spacer sequences for targeting the C9ORF72 gene with a *T. denticola* Cas9 endonuclease.

SEQ ID NOs: 6893-7759 are 20 bp spacer sequences for the C9ORF72 gene with a *N. meningitides* Cas9 endonuclease.

SEQ ID NOs: 8262-18807 are 22 bp spacer sequences for targeting the C9ORF72 gene with an *Acidominococcus, Lachnospiraceae,* and *Francisella novicida* Cpf1 endonuclease.

SEQ ID NOs: 18810-20841 are 20 bp spacer sequences for targeting exons 1-2 of an AAVS1 (PPP1 R12C) gene with a *S. pyogenes* Cas9 endonuclease.

SEQ ID NOs: 20842-21012 are 20 bp spacer sequences for targeting exons 1-2 of an AAVS1 (PPP1 R12C) gene with a *S. aureus* Cas9 endonuclease.

SEQ ID NOs: 21013-21030 are 20 bp spacer sequences for targeting exons 1-2 of an AAVS1 (PPP1 R12C) gene with a *S. thermophilus* Cas9 endonuclease.

SEQ ID NOs: 21031-21039 are 20 bp spacer sequences for targeting exons 1-2 of an AAVS1 (PPP1R12C) gene with a *T. denticola* Cas9 endonuclease.

SEQ ID NOs: 21040-21114 are 20 bp spacer sequences for targeting exons 1-2 of an AAVS1 (PPP1 R12C) gene with a *N. meningitides* Cas9 endonuclease.

SEQ ID NOs: 21115-22290 are 22 bp spacer sequences for targeting exons 1-2 of an AAVS1 (PPP1R12C) gene with an *Acidominococcus, Lachnospiraceae,* and *Francisella novicida* Cpf1 endonuclease.

SEQ ID NOs: 22291-22458 are 20 bp spacer sequences for targeting exons 1-2 of an Alb gene with a *S. pyogenes* Cas9 endonuclease.

SEQ ID NOs: 22459-22486 are 20 bp spacer sequences for targeting exons 1-2 of an Alb gene with a *S. aureus* Cas9 endonuclease.

SEQ ID NOs: 22487-22504 are 20 bp spacer sequences for targeting exons 1-2 of an Alb gene with a *S. thermophilus* Cas9 endonuclease.

SEQ ID NOs: 22505-22509 are 20 bp spacer sequences for targeting exons 1-2 of an Alb gene with a *T. denticola* Cas9 endonuclease.

SEQ ID NOs: 22510-22533 are 20 bp spacer sequences for targeting exons 1-2 of an Alb gene with a *N. meningitides* Cas9 endonuclease.

SEQ ID NOs: 22534-22912 are 22 bp spacer sequences for targeting exons 1-2 of an Alb gene with an *Acidominococcus, Lachnospiraceae,* and *Francisella novicida* Cpf1 endonuclease.

SEQ ID NOs: 22913-23257 are 20 bp spacer sequences for targeting exons 1-2 of an Angpt13 gene with a *S. pyogenes* Cas9 endonuclease.

SEQ ID NOs: 23258-23293 are 20 bp spacer sequences for targeting exons 1-2 of an Angpt13 gene with a *S. aureus* Cas9 endonuclease.

SEQ ID NOs: 23294-23316 are 20 bp spacer sequences for targeting exons 1-2 of an Angpt13 gene with a *S. thermophilus* Cas9 endonuclease.

SEQ ID NOs: 23317-23329 are 20 bp spacer sequences for targeting exons 1-2 of an Angpt13 gene with a *T. denticola* Cas9 endonuclease.

SEQ ID NOs: 23330-23392 are 20 bp spacer sequences for targeting exons 1-2 of an Angpt13 gene with a *N. meningitides* Cas9 endonuclease.

SEQ ID NOs: 23393-24240 are 22 bp spacer sequences for targeting exons 1-2 of an Angpt13 gene with an *Acidominococcus, Lachnospiraceae,* and *Francisella novicida* Cpf1 endonuclease.

SEQ ID NOs: 24241-24643 are 20 bp spacer sequences for targeting exons 1-2 of an ApoC3 gene with a *S. pyogenes* Cas9 endonuclease.

SEQ ID NOs: 24644-24668 are 20 bp spacer sequences for targeting exons 1-2 of an ApoC3 gene with a *S. aureus* Cas9 endonuclease.

SEQ ID NOs: 24669-24671 are 20 bp spacer sequences for targeting exons 1-2 of an ApoC3 gene with a *S. thermophilus* Cas9 endonuclease.

SEQ ID NOs: 24672-24673 are 20 bp spacer sequences for targeting exons 1-2 of an ApoC3 gene with a *T. denticola* Cas9 endonuclease.

SEQ ID NOs: 24674-24685 are 20 bp spacer sequences for targeting exons 1-2 of an ApoC3 gene with a *N. meningitides* Cas9 endonuclease.

SEQ ID NOs: 24686-24917 are 22 bp spacer sequences for targeting exons 1-2 of an ApoC3 gene with an *Acidominococcus, Lachnospiraceae,* and *Francisella novicida* Cpf1 endonuclease.

SEQ ID NOs: 24918-26685 are 20 bp spacer sequences for targeting exons 1-2 of an ASGR2 gene with a *S. pyogenes* Cas9 endonuclease.

SEQ ID NOs: 26686-26891 are 20 bp spacer sequences for targeting exons 1-2 of an ASGR2 gene with a *S. aureus* Cas9 endonuclease.

SEQ ID NOs: 26892-26915 are 20 bp spacer sequences for targeting exons 1-2 of an ASGR2 gene with a *S. thermophilus* Cas9 endonuclease.

SEQ ID NOs: 26916-26927 are 20 bp spacer sequences for targeting exons 1-2 of an ASGR2 gene with a *T. denticola* Cas9 endonuclease.

SEQ ID NOs: 26928-27010 are 20 bp spacer sequences for targeting exons 1-2 of an ASGR2 gene with a *N. meningitides* Cas9 endonuclease.

SEQ ID NOs: 27011-28450 are 22 bp spacer sequences for targeting exons 1-2 of an ASGR2 gene with an *Acidominococcus, Lachnospiraceae,* and *Francisella novicida* Cpf1 endonuclease.

SEQ ID NOs: 28451-28653 are 20 bp spacer sequences for targeting exons 1-2 of a CCR5 gene with a *S. pyogenes* Cas9 endonuclease.

SEQ ID NOs: 28654-28685 are 20 bp spacer sequences for targeting exons 1-2 of a CCR5 gene with a *S. aureus* Cas9 endonuclease.

SEQ ID NOs: 28686-28699 are 20 bp spacer sequences for targeting exons 1-2 of a CCR5 gene with a *S. thermophilus* Cas9 endonuclease.

SEQ ID NOs: 28700-28701 are 20 bp spacer sequences for targeting exons 1-2 of a CCR5 gene with a *T. denticola* Cas9 endonuclease.

SEQ ID NOs: 28702-28729 are 20 bp spacer sequences for targeting exons 1-2 of a CCR5 gene with a *N. meningitides* Cas9 endonuclease.

SEQ ID NOs: 28730-29029 are 22 bp spacer sequences for targeting exons 1-2 of a CCR5 gene with an *Acidominococcus, Lachnospiraceae,* and *Francisella novicida* Cpf1 endonuclease.

SEQ ID NOs: 29030-30495 are 20 bp spacer sequences for targeting exons 1-2 of an F9 gene with a *S. pyogenes* Cas9 endonuclease.

SEQ ID NOs: 30496-30658 are 20 bp spacer sequences for targeting exons 1-2 of an F9 gene with a *S. aureus* Cas9 endonuclease.

SEQ ID NOs: 30659-30719 are 20 bp spacer sequences for targeting exons 1-2 of an F9 gene with a *S. thermophilus* Cas9 endonuclease.

SEQ ID NOs: 30720-30744 are 20 bp spacer sequences for targeting exons 1-2 of an F9 gene with a *T. denticola* Cas9 endonuclease.

SEQ ID NOs: 30745-30897 are 20 bp spacer sequences for targeting exons 1-2 of an F9 gene with a *N. meningitides* Cas9 endonuclease.

SEQ ID NOs: 30898-33038 are 22 bp spacer sequences for targeting exons 1-2 of an F9 gene with an *Acidominococcus, Lachnospiraceae,* and *Francisella novicida* Cpf1 endonuclease.

SEQ ID NOs: 33039-34054 are 20 bp spacer sequences for targeting exons 1-2 of a G6PC gene with a *S. pyogenes* Cas9 endonuclease.

SEQ ID NOs: 34055-34171 are 20 bp spacer sequences for targeting exons 1-2 of a G6PC gene with a *S. aureus* Cas9 endonuclease.

SEQ ID NOs: 34172-34195 are 20 bp spacer sequences for targeting exons 1-2 of a G6PC gene with a *S. thermophilus* Cas9 endonuclease.

SEQ ID NOs: 34196-34204 are 20 bp spacer sequences for targeting exons 1-2 of a G6PC gene with a *T. denticola* Cas9 endonuclease.

SEQ ID NOs: 34205-34294 are 20 bp spacer sequences for targeting exons 1-2 of a G6PC gene with a *N. meningitides* Cas9 endonuclease.

SEQ ID NOs: 34295-35389 are 22 bp spacer sequences for targeting exons 1-2 of a G6PC gene with an *Acidominococcus, Lachnospiraceae,* and *Francisella novicida* Cpf1 endonuclease.

SEQ ID NOs: 35390-40882 are 20 bp spacer sequences for targeting exons 1-2 of a Gys2 gene with a *S. pyogenes* Cas9 endonuclease.

SEQ ID NOs: 40883-41558 are 20 bp spacer sequences for targeting exons 1-2 of a Gys2 gene with a *S. aureus* Cas9 endonuclease.

SEQ ID NOs: 41559-41836 are 20 bp spacer sequences for targeting exons 1-2 of a Gys2 gene with a *S. thermophilus* Cas9 endonuclease.

SEQ ID NOs: 41837-41950 are 20 bp spacer sequences for targeting exons 1-2 of a Gys2 gene with a *T. denticola* Cas9 endonuclease.

SEQ ID NOs: 41951-42630 are 20 bp spacer sequences for targeting exons 1-2 of a Gys2 gene with a *N. meningitides* Cas9 endonuclease.

SEQ ID NOs: 42631-51062 are 22 bp spacer sequences for targeting exons 1-2 of a Gys2 gene with an *Acidominococcus, Lachnospiraceae,* and *Francisella novicida* Cpf1 endonuclease.

SEQ ID NOs: 51063-52755 are 20 bp spacer sequences for targeting exons 1-2 of an HGD gene with a *S. pyogenes* Cas9 endonuclease.

SEQ ID NOs: 52756-52969 are 20 bp spacer sequences for targeting exons 1-2 of an HGD gene with a *S. aureus* Cas9 endonuclease.

SEQ ID NOs: 52970-53052 are 20 bp spacer sequences for targeting exons 1-2 of an HGD gene with a *S. thermophilus* Cas9 endonuclease.

SEQ ID NOs: 53053-53071 are 20 bp spacer sequences for targeting exons 1-2 of an HGD gene with a *T. denticola* Cas9 endonuclease.

SEQ ID NOs: 53072-53272 are 20 bp spacer sequences for targeting exons 1-2 of an HGD gene with a *N. meningitides* Cas9 endonuclease.

SEQ ID NOs: 53273-55597 are 22 bp spacer sequences for targeting exons 1-2 of an HGD gene with an *Acidominococcus, Lachnospiraceae,* and *Francisella novicida* Cpf1 endonuclease.

SEQ ID NOs: 55598-59392 are 20 bp spacer sequences for targeting exons 1-2 of an Lp(a) gene with a *S. pyogenes* Cas9 endonuclease.

SEQ ID NOs: 59393-59802 are 20 bp spacer sequences for targeting exons 1-2 of an Lp(a) gene with a *S. aureus* Cas9 endonuclease.

SEQ ID NOs: 59803-59938 are 20 bp spacer sequences for targeting exons 1-2 of an Lp(a) gene with a *S. thermophilus* Cas9 endonuclease.

SEQ ID NOs: 59939-59973 are 20 bp spacer sequences for targeting exons 1-2 of an Lp(a) gene with a *T. denticola* Cas9 endonuclease.

SEQ ID NOs: 59974-60341 are 20 bp spacer sequences for targeting exons 1-2 of an Lp(a) gene with a *N. meningitides* Cas9 endonuclease.

SEQ ID NOs: 60342-64962 are 22 bp spacer sequences for targeting exons 1-2 of an Lp(a) gene with an *Acidominococcus, Lachnospiraceae,* and *Francisella novicida* Cpf1 endonuclease.

SEQ ID NOs: 64963-66982 are 20 bp spacer sequences for targeting exons 1-2 of a PCSK9 gene with a *S. pyogenes* Cas9 endonuclease.

SEQ ID NOs: 66983-67169 are 20 bp spacer sequences for targeting exons 1-2 of a PCSK9 gene with a *S. aureus* Cas9 endonuclease.

SEQ ID NOs: 67170-67205 are 20 bp spacer sequences for targeting exons 1-2 of a PCSK9 gene with a *S. thermophilus* Cas9 endonuclease.

SEQ ID NOs: 67206-67219 are 20 bp spacer sequences for targeting exons 1-2 of a PCSK9 gene with a *T. denticola* Cas9 endonuclease.

SEQ ID NOs: 67220-67359 are 20 bp spacer sequences for targeting exons 1-2 of a PCSK9 gene with a *N. meningitides* Cas9 endonuclease.

SEQ ID NOs: 67360-69153 are 22 bp spacer sequences for targeting exons 1-2 of a PCSK9 gene with an *Acidominococcus, Lachnospiraceae,* and *Francisella novicida* Cpf1 endonuclease.

SEQ ID NOs: 69154-70291 are 20 bp spacer sequences for targeting exons 1-2 of a Serpina1 gene with a *S. pyogenes* Cas9 endonuclease.

SEQ ID NOs: 70292-70384 are 20 bp spacer sequences for targeting exons 1-2 of a Serpina1 gene with a *S. aureus* Cas9 endonuclease.

SEQ ID NOs: 70385-70396 are 20 bp spacer sequences for targeting exons 1-2 of a Serpina1 gene with a *S. thermophilus* Cas9 endonuclease.

SEQ ID NOs: 70397-70399 are 20 bp spacer sequences for targeting exons 1-2 of a Serpina1 gene with a *T. denticola* Cas9 endonuclease.

SEQ ID NOs: 70400-70450 are 20 bp spacer sequences for targeting exons 1-2 of a Serpina1 gene with a *N. meningitides* Cas9 endonuclease.

SEQ ID NOs: 70451-71254 are 22 bp spacer sequences for targeting exons 1-2 of a Serpina1 gene with an *Acidominococcus, Lachnospiraceae,* and *Francisella novicida* Cpf1 endonuclease.

SEQ ID NOs: 71255-72086 are 20 bp spacer sequences for targeting exons 1-2 of a TF gene with a *S. pyogenes* Cas9 endonuclease.

SEQ ID NOs: 72087-72172 are 20 bp spacer sequences for targeting exons 1-2 of a TF gene with a *S. aureus* Cas9 endonuclease.

SEQ ID NOs: 72173-72184 are 20 bp spacer sequences for targeting exons 1-2 of a TF gene with a *S. thermophilus* Cas9 endonuclease.

SEQ ID NOs: 72185-72191 are 20 bp spacer sequences for targeting exons 1-2 of a TF gene with a *T. denticola* Cas9 endonuclease.

SEQ ID NOs: 72192-72235 are 20 bp spacer sequences for targeting exons 1-2 of a TF gene with a *N. meningitides* Cas9 endonuclease.

SEQ ID NOs: 72236-72871 are 22 bp spacer sequences for targeting exons 1-2 of a TF gene with an *Acidominococcus, Lachnospiraceae,* and *Francisella novicida* Cpf1 endonuclease.

SEQ ID NOs: 72872-73171 are 20 bp spacer sequences for targeting exons 1-2 of a TTR gene with a *S. pyogenes* Cas9 endonuclease.

SEQ ID NOs: 73172-73212 are 20 bp spacer sequences for targeting exons 1-2 of a TTR gene with a *S. aureus* Cas9 endonuclease.

SEQ ID NOs: 73213-73229 are 20 bp spacer sequences for targeting exons 1-2 of a TTR gene with a *S. thermophilus* Cas9 endonuclease.

SEQ ID NOs: 73230-73231 are 20 bp spacer sequences for targeting exons 1-2 of a TTR gene with a *T. denticola* Cas9 endonuclease.

SEQ ID NOs: 73232-73266 are 20 bp spacer sequences for targeting exons 1-2 of a TTR gene with a *N. meningitides* Cas9 endonuclease.

SEQ ID NOs: 73267-73668 are 22 bp spacer sequences for targeting exons 1-2 of a TTR gene with an *Acidominococcus, Lachnospiraceae,* and *Francisella novicida* Cpf1 endonuclease.

### Detailed Description

### C9ORF72

The human C9ORF72 gene is located on the short (p) arm of chromosome 9 open reading frame 72, from base pair 27,546,542 to base pair 27,573,863. Its cytogenetic location is at 9p21.2. The mutation of C9ORF72 is a hexanucleotide repeat expansion of the six letter string of nucleotides GGGGCC. In healthy individuals, there are few repeats of this hexanucleotide, typically 30 or less, but in people with the diseased phenotype, the repeat can occur in the order of hundreds. The hexanucleotide expansion event in the C9ORF72 gene is present in approximately 40% of familial ALS and 8-10% of sporadic ALS.

The hexanucleotide expansion occurs in an alternatively spliced Intron 1 of the C9ORF72 gene, and as such does not alter the coding sequence or resulting protein. Three alternatively spliced variants of C9ORF72 (V1, V2 and V3) are normally produced. The expanded nucleotide repeat has been shown to reduce the transcription of V1, however the total amount of protein produced was unaffected.

The term "hexanucleotide repeat expansion" means a series of six bases (for example, GGGGCC, GGGGGG, GGGGCG, or GGGGGC) repeated at least twice. In certain embodiments, the hexanucleotide repeat expansion may be located in intron 1 of a C9ORF72 nucleic acid. In certain embodiments, a pathogenic hexanucleotide repeat expansion includes at least 30 repeats of GGGGCC, GGGGGG, GGGGCG, or GGGGGC in a C9ORF72 nucleic acid and is associated with disease. In other embodiments, a pathogenic hexanucleotide repeat expansion includes at least 31, 32, 33, 34, 35, 40, 45, 50, 60, 70, 80, 90, 100, 150, 200, 250, 300 or more repeats. In certain embodiments, the repeats are consecutive. In certain embodiments, the repeats are interrupted by 1 or more nucleobases. In certain embodiments, a wild-type hexanucleotide repeat expansion includes 29 or fewer repeats of GGGGCC, GGGGGG, GGGGCG, or GGGGGC in a C9ORF72 nucleic acid. In other embodiments, a wild-type hexanucleotide repeat expansion includes 28, 27, 26, 25, 24, 23, 22, 21, 20, 19, 18, 17, 16, 15, 14, 13, 12, 11, 10, 9, 8, 7, 6, 5, 4, 3, 2, or 1 repeat. In certain embodiments, the entire hexanucleotide repeat expansion is deleted. In certain embodiments, the repeats are consecutive. In certain embodiments, the repeats are interrupted by 1 or more nucleobases.

### Therapeutic approach

Disclosed herein are cellular, *ex vivo* and *in vivo* methods for using genome engineering tools to create permanent changes to the genome by: 1) completely deleting the expanded hexanucleotide repeat within or near the C9ORF72 gene, 2) correcting/replacing the expanded hexanucleotide repeat within or near the C9ORF72 gene with a wild-type or similar number of hexanucleotide repeats, or 3) deletion of the hexanucleotide repeat region and knocking-in C9ORF72 cDNA into the gene locus or a safe harbor locus. Such methods use endonucleases, such as CRISPRassociated (Cas9, Cpf1 and the like) nucleases, to permanently delete or replace the expanded hexanucleotide repeat or portions thereof or insert in the genomic locus of the C9ORF72 gene. In this way, the present invention restores the wild-type or similar C9ORF72 intronic sequence or deletes the expanded hexanucleotide repeat with a single treatment (rather than deliver potential therapies for the lifetime of the patient).

Disclosed herein are methods for treating a patient with amyotrophic lateral sclerosis (ALS) or frontotemporal lobular dementia (FTLD). An embodiment of such method is an *ex vivo* cell based therapy. For example, a patient specific induced pluripotent stem cell (iPSC) is created. Then, the chromosomal DNA of these iPS cells is corrected using the materials and methods described herein. Next, the corrected iPSCs are differentiated into hematopoietic progenitor cells, neural progenitor cells, or neural cells. Finally, the hematopoietic progenitor cells, neural progenitor cells, or neural cells are implanted into the patient.

Disclosed herein are methods of restoring correct expression of a C9ORF72 gene using CRISPR-Cas9 or CRISPR-Cpf1, together with donor template to insert a wildtype C9ORF72 cDNA into an C9ORF72 locus, or to a safe harbor locus selected from the group consisting of: AAVS1 (PPP1R12C), ALB, Angptl3, ApoC3, ASGR2, CCR5, FIX (F9), G6PC, Gys2, HGD, Lp(a), Pcsk9, Serpina1, TF, and TTR. In some embodiments, such method is the use of a cDNA that can be knocked in and that contains the exons affected. In addition to partial cDNA approach, a full length cDNA can be knocked into translation start site. In either case, the expression of inserted cDNA is under the control of a C9ORF72 promoter, which renders the precise regulation of C9ORF72 protein level.

Also disclosed herein are methods of restoring correct expression of a C9ORF72 gene by inserting C9ORF72 cDNA into a safe harbor locus selected from the group consisting of: exon 1-2 of AAVS1 (PPP1R12C), exon 1-2 of ALB, exon 1-2 of Angptl3, exon 1-2 of ApoC3, exon 1-2 of ASGR2, exon 1-2 of CCR5, exon 1-2 of FIX (F9), exon 1-2 of G6PC, exon 1-2 of Gys2, exon 1-2 of HGD, exon 1-2 of Lp(a), exon 1-2 of Pcsk9, exon 1-2 of Serpina1, exon 1-2 of TF, and exon 1-2 of TTR.

Another embodiment of such method is an *ex vivo* cell based therapy. For example, a white blood cell is isolated from the patient. Next, the chromosomal DNA of these white blood cells is corrected using the materials and methods described herein. Finally, the edited white blood cells are implanted into the patient.

Yet another embodiment of such method is an *ex vivo* cell based therapy. For example, a biopsy of the patient's bone marrow is optionally performed. Then, a mesenchymal stem cell is isolated from the biopsied material. Next, the chromosomal DNA of these stem cells is corrected using the materials and methods described herein. Next, the stem cells are differentiated into hematopoietic progenitor cells, neural progenitor cells, or neural cells. Finally, these hematopoietic progenitor cells, neural progenitor cells, or neural cells are implanted into the patient.

A further embodiments of such method is an *ex vivo* cell based therapy. For example, a patient is optionally treated with granulocyte colony stimulating factor. Then, a hematopoietic progenitor cell is isolated from the patient. Next, the chromosomal DNA of these cells is corrected using the materials and methods described herein. Finally, the cells are implanted into the patient.

One advantage of an *ex vivo* cell therapy approach is the ability to conduct a comprehensive analysis of the therapeutic prior to administration. All nuclease based therapeutics have some level of off-target effects. Performing gene correction *ex vivo* allows one to fully characterize the corrected cell population prior to implantation. Aspects of the invention include sequencing the entire genome of the corrected cells to ensure that the off-target cuts, if any, are in genomic locations associated with minimal risk to the patient. Furthermore, populations of specific cells, including clonal populations, can be isolated prior to implantation.

Another advantage of *ex vivo* cell therapy relates to genetic correction in iPSCs compared to other primary cell sources. iPSCs are prolific, making it easy to obtain the large number of cells that will be required for a cell based therapy. Furthermore, iPSCs are an ideal cell type for performing clonal isolations. This allows screening for the correct genomic correction, without risking a decrease in viability. In contrast, other primary cells are viable for only a few passages and difficult to clonally expand. Thus, manipulation of iPSCs for the treatment of ALS or FTLD will be much easier, and will shorten the amount of time needed to make the desired genetic correction.

Another embodiment of such method is an *in vivo* based therapy. In this method, the chromosomal DNA of the cells in the patient is corrected using the materials and methods described herein. Preferably, the cells are neural cells, bone marrow cells, hematopoietic progenitor cells, or CD34+ cells.

An advantage of *in vivo* gene therapy is the ease of therapeutic production and administration. The same therapeutic approach and therapy will have the potential to be used to treat more than one patient, for example a number of patients who share the same or similar genotype or allele. In contrast, *ex vivo* cell therapy typically requires using a patient's own cells, which are isolated, manipulated and returned to the same patient.

Also disclosed herein is a cellular method for editing the C9ORF72 gene in a cell by genome editing. For example, a cell is isolated from a patient or animal. Then, the chromosomal DNA of the cell is corrected using the materials and methods described herein.

The methods of the disclosure, regardless of whether a cellular or *ex vivo* or *in vivo* method, involves one or a combination of the following: deleting the entire expanded hexanucleotide repeat or a portion thereof in or near the C9ORF72 gene, correcting the expanded hexanucleotide repeat in or near the C9ORF72 gene to wildtype level or similar levels, or introducing exogenous C9ORF72 DNA or cDNA sequence or a fragment thereof into the locus of the gene or at a heterologous location in the genome (such as a safe harbor site, such as a safe harbor locus selected from the group consisting of: AAVS1 (PPP1R12C), ALB, Angptl3, ApoC3, ASGR2, CCR5, FIX (F9), G6PC, Gys2, HGD, Lp(a), Pcsk9, Serpina1, TF, and TTR). Both the correction and knock-in strategies utilize a donor DNA template in Homology-Directed Repair (HDR). HDR in either strategy may be accomplished by making one or more double-stranded breaks (DSBs) at specific sites in the genome by using one or more endonucleases. Assessment of efficiency of HDR mediated knock-in of cDNA into the first exon can utilize cDNA knock-in into "safe harbor" sites such as: single-stranded or double-stranded DNA having homologous arms to one of the following regions, for example: ApoC3 (chr11:116,829,908-116,833,071), Angptl3 (chr1:62,597,487-62,606,305), Serpina1 (chr14:94,376,747-94,390,692), Lp(a) (chr6:160,531,483-160,664,259), Pcsk9 (chr1:55,039,475-55,064,852), FIX (chrX:139,530,736-139,563,458), ALB (chr4:73,404,254-73,421,411), TTR (chr18:31,591,766-31,599,023), TF (chr3:133,661,997-133,779,005), G6PC (17:42,900,796-42,914,432), Gys2 (chr12:21,536,188-21,604,857), AAVS1(PPP1R12C) (chr19:55,090,912-55,117,599), HGD (chr3:120,628,167-120,682,570), CCR5 (chr3:46,370,854-46,376,206), ASGR2 (chr17:7,101,322-7,114,310). Both the correction and knock-in strategies utilize a donor DNA template in Homology-Directed Repair (HDR). HDR in either strategy may be accomplished by making one or more double-stranded breaks (DSBs) at specific sites in the genome by using one or more endonucleases.

For example, the correction strategy involves correcting the expanded hexanucleotide repeat in the C9ORF72 gene by inducing one double stranded break in the gene of interest with one or more Cas9 and a sg RNA, or two or more double stranded breaks in the gene of interest using two or more appropriate sgRNAs, in the presence of a donor DNA template introduced exogenously to direct the cellular DSB response to Homology-Directed Repair (the donor DNA template can be a short single stranded oligonucleotide, a short double stranded oligonucleotide, a long single or double stranded DNA molecule). This approach requires development and optimization of gRNAS and donor DNA molecules for heterogeneous hexanucleotide repeat expansions of the C9ORF72 gene.

For example, the knock-in strategy involves knocking-in C9ORF72 cDNA into the locus of the gene using a sgRNA or a pair of sgRNAs targeting upstream of or in the first or other exon and/or intron of the C9ORF72 gene, or in a safe harbor site (such as, e.g., exon 1-2 of a safe harbor locus selected from the group consisting of: AAVS1 (PPP1R12C), ALB, Angptl3, ApoC3, ASGR2, CCR5, FIX (F9), G6PC, Gys2, HGD, Lp(a), Pcsk9, Serpina1, TF, and TTR)). The donor DNA will be single or double stranded DNA having homologous arms to the 9p21.2 region. The donor DNA can be single or double stranded DNA having homologous arms to the target safe harbor locus. The donor template can be single or double stranded DNA having homologous arms to a safe harbor locus selected from the group consisting of: AAVS1 (PPP1R12C), ALB, Angptl3, ApoC3, ASGR2, CCR5, FIX (F9), G6PC, Gys2, HGD, Lp(a), Pcsk9, Serpina1, TF, and TTR.

For example, the deletion strategy involves deleting one or more, and preferably all, hexanucleotide repeats in the C9ORF72 gene using one or more endonucleases and two or more gRNAs or sgRNAs.

The advantages for the above strategies (correction and knock-in and deletion) are similar, including in principle both short and long term beneficial clinical and laboratory effects. The knock-in approach does provide one advantage over the correction or deletion approach - the ability to treat all patients versus only a subset of patients. The other issue with gene editing in this manner is the need for a DNA donor for HDR.

In addition to the above genome editing strategies, another strategy involves modulating expression, function, or activity of C9orf72 by editing in the gene other than the expanded hexanucleotide repeat region or by editing in the regulatory sequence.

A CRISPR/Cas9 endonuclease can be used to target single-stranded RNA targets when the PAM is presented in trans as a DNA oligo (PAMer) (O'Connell, M.R. et al. Nature 516, 263-266 (2014)). Manipulation of this system can be utilized to specifically induce breaks in transcribed RNA while leaving the genomic locus intact. This is a useful strategy for specifically knocking down expression from deleterious RNAs in the cell. In the context of treating ALS, one approach would be to deliver gRNAs that specifically target the hexanucleotide repeat in C9ORF72 pre-RNA along with a DNA PAMer to induce cutting. This would lead to reduced expression of the deleterious C9ORF72 mRNA.

Another method of the disclosure involves treating a patient with amyotrophic lateral sclerosis (ALS) or frontotemporal lobular dementia (FTLD) comprising transplanting the bone marrow from a donor to the patient.

A number of types of genomic target sites are present in addition to mutations in the coding and splicing sequences.

The regulation of transcription and translation implicates a number of different classes of sites that interact with cellular proteins or nucleotides. Often the DNA binding sites of transcription factors or other proteins can be targeted for mutation or deletion to study the role of the site, though they can also be targeted to change gene expression. Sites can be added through non-homologous end joining (NHEJ) or direct genome editing by homology directed repair (HDR). Increased use of genome sequencing, RNA expression and genome-wide studies of transcription factor binding have increased our ability to identify how the sites lead to developmental or temporal gene regulation. These control systems may be direct or may involve extensive cooperative regulation that can require the integration of activities from multiple enhancers. Transcription factors typically bind 6-12 bp-long degenerate DNA sequences. The low level of specificity provided by individual sites suggests that complex interactions and rules are involved in binding and the functional outcome. Binding sites with less degeneracy may provide simpler means of regulation. Artificial transcription factors can be designed to specify longer sequences that have less similar sequences in the genome and have lower potential for off-target cleavage. Any of these types of binding sites can be mutated, deleted or even created to enable changes in gene regulation or expression (Canver, M.C. et al., Nature (2015)).

Another class of gene regulatory regions having these features is microRNA (miRNA) binding sites. miRNAs are non-coding RNAs that play key roles in post-transcriptional gene regulation. miRNA may regulate the expression of 30% of all mammalian protein-encoding genes. Specific and potent gene silencing by double stranded RNA (RNAi) was discovered, plus additional small noncoding RNA (Canver, M.C. et al., Nature (2015)). The largest class of noncoding RNAs important for gene silencing are miRNAs. In mammals, miRNAs are first transcribed as a long RNA transcripts, which can be separate transcriptional units, part of protein introns, or other transcripts. The long transcripts are called primary miRNA (pri-miRNA) that include imperfectly base-paired hairpin structures. These pri-miRNA are cleaved into one or more shorter precursor miRNAs (pre-miRNAs) by Microprocessor, a protein complex in the nucleus, involving Drosha.

Pre-miRNAs are short stem loops ∼70 nucleotides in length with a 2-nucleotide 3'-overhang that are exported, into the mature 19-25 nucleotide miRNA:miRNA* duplexes. The miRNA strand with lower base pairing stability (the guide strand) is loaded onto the RNA-induced silencing complex (RISC). The passenger guide strand (marked with *), may be functional, but is usually degraded. The mature miRNA tethers RISC to partly complementary sequence motifs in target mRNAs predominantly found within the 3' untranslated regions (UTRs) and induces posttranscriptional gene silencing (Bartel, D.P. Cell 136, 215-233 (2009); Saj, A. & Lai, E.C. Curr Opin Genet Dev 21, 504-510 (2011)).

miRNAs are important in development, differentiation, cell cycle and growth control, and in virtually all biological pathways in mammals and other multicellular organisms. miRNAs are also involved in cell cycle control, apoptosis and stem cell differentiation, hematopoiesis, hypoxia, muscle development, neurogenesis, insulin secretion, cholesterol metabolism, aging, viral replication and immune responses.

A single miRNA can target hundreds of different mRNA transcripts, while an individual transcript can be targeted by many different miRNAs. More than 28645 microRNAs have been annotated in the latest release of miRBase (v.21). Some miRNAs are encoded by multiple loci, some of which are expressed from tandemly co-transcribed clusters. The features allow for complex regulatory networks with multiple pathways and feedback controls. miRNAs are integral parts of these feedback and regulatory circuits and can help regulate gene expression by keeping protein production within limits (Herranz, H. & Cohen, S.M. Genes Dev 24, 1339-1344 (2010); Posadas, D.M. & Carthew, R.W. Curr Opin Genet Dev 27, 1-6 (2014)).

miRNA are also important in a large number of human diseases that are associated with abnormal miRNA expression. This association underscores the importance of the miRNA regulatory pathway. Recent miRNA deletion studies have linked miRNA with regulation of the immune responses (Stern-Ginossar, N. et al., Science 317, 376-381 (2007)).

miRNA also have a strong link to cancer and may play a role in different types of cancer. miRNAs have been found to be downregulated in a number of tumors. miRNA are important in the regulation of key cancer-related pathways, such as cell cycle control and the DNA damage response, and are therefore used in diagnosis and are being targeted clinically. MicroRNAs delicately regulate the balance of angiogenesis, such that experiments depleting all microRNAs suppresses tumor angiogenesis (Chen, S. et al., Genes Dev 28, 1054-1067 (2014)).

As has been shown for protein coding genes, miRNA genes are also subject to epigenetic changes occurring with cancer. Many miRNA loci are associated with CpG islands increasing their opportunity for regulation by DNA methylation (Weber, B., Stresemann, C., Brueckner, B. & Lyko, F. Cell Cycle 6, 1001-1005 (2007)). The majority of studies have used treatment with chromatin remodeling drugs to reveal epigenetically silenced miRNAs.

In addition to their role in RNA silencing, miRNA can also activate translation (Posadas, D.M. & Carthew, R.W. Curr Opin Genet Dev 27, 1-6 (2014)). Knocking out these sites may lead to decreased expression of the targeted gene, while introducing these sites may increase expression.

Individual miRNA can be knocked out most effectively by mutating the seed sequence (bases 2-8 of the microRNA), which is important for binding specificity. Cleavage in this region, followed by mis-repair by NHEJ can effectively abolish miRNA function by blocking binding to target sites. miRNA could also be inhibited by specific targeting of the special loop region adjacent to the palindromic sequence. Catalytically inactive Cas9 can also be used to inhibit shRNA expression (Zhao, Y. et al., Sci Rep 4, 3943 (2014)). In addition to targeting the miRNA, the binding sites can also be targeted and mutated to prevent the silencing by miRNA.

### Human Cells

For ameliorating ALS or FTLD, as described and illustrated herein, the principal targets for gene editing are human cells. For example, in the *ex vivo* methods, the human cells are somatic cells, which after being modified using the techniques as described, can give rise to neural cells or progenitor cells. For example, in the *in vivo* methods, the human cells are neural cells.

By performing gene editing in autologous cells that are derived from and therefore already completely matched with the patient in need, it is possible to generate cells that can be safely re-introduced into the patient, and effectively give rise to a population of cells that will be effective in ameliorating one or more clinical conditions associated with the patient's disease.

Progenitor cells (also referred to as stem cells herein) are capable of both proliferation and giving rise to more progenitor cells, these in turn having the ability to generate a large number of mother cells that can in turn give rise to differentiated or differentiable daughter cells. The daughter cells themselves can be induced to proliferate and produce progeny that subsequently differentiate into one or more mature cell types, while also retaining one or more cells with parental developmental potential. The term "stem cell" refers then, to a cell with the capacity or potential, under particular circumstances, to differentiate to a more specialized or differentiated phenotype, and which retains the capacity, under certain circumstances, to proliferate without substantially differentiating. In one embodiment, the term progenitor or stem cell refers to a generalized mother cell whose descendants (progeny) specialize, often in different directions, by differentiation, *e*.*g*., by acquiring completely individual characters, as occurs in progressive diversification of embryonic cells and tissues. Cellular differentiation is a complex process typically occurring through many cell divisions. A differentiated cell may derive from a multipotent cell that itself is derived from a multipotent cell, and so on. While each of these multipotent cells may be considered stem cells, the range of cell types that each can give rise to may vary considerably. Some differentiated cells also have the capacity to give rise to cells of greater developmental potential. Such capacity may be natural or may be induced artificially upon treatment with various factors. In many biological instances, stem cells are also "multipotent" because they can produce progeny of more than one distinct cell type, but this is not required for "stem-ness."

Self-renewal is another important aspect of the stem cell. In theory, self-renewal can occur by either of two major mechanisms. Stem cells may divide asymmetrically, with one daughter retaining the stem state and the other daughter expressing some distinct other specific function and phenotype. Alternatively, some of the stem cells in a population can divide symmetrically into two stems, thus maintaining some stem cells in the population as a whole, while other cells in the population give rise to differentiated progeny only. Generally, "progenitor cells" have a cellular phenotype that is more primitive (*i.e.,* is at an earlier step along a developmental pathway or progression than is a fully differentiated cell). Often, progenitor cells also have significant or very high proliferative potential. Progenitor cells can give rise to multiple distinct differentiated cell types or to a single differentiated cell type, depending on the developmental pathway and on the environment in which the cells develop and differentiate.

In the context of cell ontogeny, the adjective "differentiated," or "differentiating" is a relative term. A "differentiated cell" is a cell that has progressed further down the developmental pathway than the cell to which it is being compared. Thus, stem cells can differentiate into lineage-restricted precursor cells (such as a myocyte progenitor cell), which in turn can differentiate into other types of precursor cells further down the pathway (such as a myocyte precursor), and then to an end-stage differentiated cell, such as a myocyte, which plays a characteristic role in a certain tissue type, and may or may not retain the capacity to proliferate further.

The term "hematopoietic progenitor cell" refers to cells of a stem cell lineage that give rise to all the blood cell types, including erythroid (erythrocytes or red blood cells (RBCs)), myeloid (monocytes and macrophages, neutrophils, basophils, eosinophils, megakaryocytes / platelets, and dendritic cells), and lymphoid (T-cells, B-cells, NK-cells).

A "cell of the erythroid lineage" indicates that the cell being contacted is a cell that undergoes erythropoiesis, such that upon final differentiation it forms an erythrocyte or red blood cell. Such cells originate from bone marrow hematopoietic progenitor cells. Upon exposure to specific growth factors and other components of the hematopoietic microenvironment, hematopoietic progenitor cells can mature through a series of intermediate differentiation cellular types, all intermediates of the erythroid lineage, into RBCs. Thus, cells of the "erythroid lineage" comprise hematopoietic progenitor cells, rubriblasts, prorubricytes, erythroblasts, metarubricytes, reticulocytes, and erythrocytes.

In some embodiments, the hematopoietic progenitor cell expresses at least one of the following cell surface markers characteristic of hematopoietic progenitor cells: CD34+, CD59+, Thyl/CD90+, CD381o/-, and C-kit/CDI 17+. In some embodiments, the hematopoietic progenitors are CD34+.

In some embodiments, the hematopoietic progenitor cell is a peripheral blood stem cell obtained from the patient after the patient has been treated with one or more factors such as granulocyte colony stimulating factor (optionally in combination with Plerixaflor). In illustrative embodiments, CD34+ cells are enriched using CliniMACS® Cell Selection System (Miltenyi Biotec). In some embodiments, CD34+ cells are stimulated in serum-free medium (e.g., CellGrow SCGM media, CellGenix) with cytokines (e.g., SCF, rhTPO, rhFLT3) before genome editing. In some embodiments, addition of SR1 and dmPGE2 and/or other factors is contemplated to improve long-term engraftment.

In some embodiments, the hematopoietic progenitor cells of the erythroid lineage have a cell surface marker characteristic of the erythroid lineage: such as CD71 and Terl 19.

ALS and FTLD are both diseases of motor neurons, the large cells of the spinal cord that send nerve fibers out to control the muscles. Also, motor neurons in the part of the brain governing voluntary movements are destroyed in ALS and FTLD. These so called upper motor neurons send nerve fibers down from the brain to control the lower motor neurons in the spinal cord. *In vivo* methods could target the motor neurons directly. *In vivo* methods could also target the glia, astrocytes and other supporting cells.

In unpublished data, it has been shown that mice harboring a homozygous C9ORF72 mutation are viable. However these mice are characterized by a decreased life expectancy, splenomegaly, neutrophilia, thrombocytopenia, and elevated levels of inflammatory cytokines, suggesting that there may be an immune component to the pathology of ALS. Interestingly, a bone marrow transplant is able to delay the lethality and level of cytokines observed in C9ORF72 homozygous mutant mice. Building on this work, it is hypothesized that correction of the C9orf72 locus in cells of the immune system using CRISPR/Cas9/Cpf1 technologies may be a new approach for the treatment of ALS.

### Induced Pluripotent Stem Cells

In some embodiments, the genetically engineered human cells described herein are induced pluripotent stem cells (iPSCs). An advantage of using iPSCs is that the cells can be derived from the same subject to which the progenitor cells are to be administered. That is, a somatic cell can be obtained from a subject, reprogrammed to an induced pluripotent stem cell, and then re-differentiated into a progenitor cell to be administered to the subject (*e*.*g*., autologous cells). Because the progenitors are essentially derived from an autologous source, the risk of engraftment rejection or allergic response is reduced compared to the use of cells from another subject or group of subjects. In addition, the use of iPSCs negates the need for cells obtained from an embryonic source. Thus, in one embodiment, the stem cells used in the disclosed methods are not embryonic stem cells.

Although differentiation is generally irreversible under physiological contexts, several methods have been recently developed to reprogram somatic cells to iPSCs. Exemplary methods are known to those of skill in the art and are described briefly herein below.

The term "reprogramming" refers to a process that alters or reverses the differentiation state of a differentiated cell *(e.g.,* a somatic cell). Stated another way, reprogramming refers to a process of driving the differentiation of a cell backwards to a more undifferentiated or more primitive type of cell. It should be noted that placing many primary cells in culture can lead to some loss of fully differentiated characteristics. Thus, simply culturing such cells included in the term differentiated cells does not render these cells non-differentiated cells (*e.g.,* undifferentiated cells) or pluripotent cells. The transition of a differentiated cell to pluripotency requires a reprogramming stimulus beyond the stimuli that lead to partial loss of differentiated character in culture. Reprogrammed cells also have the characteristic of the capacity of extended passaging without loss of growth potential, relative to primary cell parents, which generally have capacity for only a limited number of divisions in culture.

The cell to be reprogrammed can be either partially or terminally differentiated prior to reprogramming. In some embodiments, reprogramming encompasses complete reversion of the differentiation state of a differentiated cell (*e.g.,* a somatic cell) to a pluripotent state or a multipotent state. In some embodiments, reprogramming encompasses complete or partial reversion of the differentiation state of a differentiated cell (*e.g.,* a somatic cell) to an undifferentiated cell (*e*.*g*., an embryonic-like cell). Reprogramming can result in expression of particular genes by the cells, the expression of which further contributes to reprogramming. In certain embodiments described herein, reprogramming of a differentiated cell (*e.g.,* a somatic cell) causes the differentiated cell to assume an undifferentiated state (*e*.*g*., is an undifferentiated cell). The resulting cells are referred to as "reprogrammed cells," or "induced pluripotent stem cells (iPSCs or iPS cells)."

Reprogramming can involve alteration, *e*.*g*., reversal, of at least some of the heritable patterns of nucleic acid modification (*e*.*g*., methylation), chromatin condensation, epigenetic changes, genomic imprinting, etc., that occur during cellular differentiation. Reprogramming is distinct from simply maintaining the existing undifferentiated state of a cell that is already pluripotent or maintaining the existing less than fully differentiated state of a cell that is already a multipotent cell (*e.g.,* a myogenic stem cell). Reprogramming is also distinct from promoting the self-renewal or proliferation of cells that are already pluripotent or multipotent, although the compositions and methods described herein can also be of use for such purposes, in some embodiments.

Many methods are known in the art that can be used to generate pluripotent stem cells from somatic cells. Any such method that reprograms a somatic cell to the pluripotent phenotype would be appropriate for use in the methods described herein.

Reprogramming methodologies for generating pluripotent cells using defined combinations of transcription factors have been described. Mouse somatic cells can be converted to ES cell-like cells with expanded developmental potential by the direct transduction of Oct4, Sox2, Klf4, and c-Myc; see, *e*.*g*., Takahashi and Yamanaka, Cell 126(4): 663-76 (2006). iPSCs resemble ES cells, as they restore the pluripotency-associated transcriptional circuitry and much of the epigenetic landscape. In addition, mouse iPSCs satisfy all the standard assays for pluripotency: specifically, *in vitro* differentiation into cell types of the three germ layers, teratoma formation, contribution to chimeras, germline transmission [see, *e*.*g*., Maherali and Hochedlinger, Cell Stem Cell. 3(6):595-605 (2008)], and tetraploid complementation.

Human iPSCs can be obtained using similar transduction methods, and the transcription factor trio, OCT4, SOX2, and NANOG, has been established as the core set of transcription factors that govern pluripotency; see, *e*.*g*., Budniatzky and Gepstein, Stem Cells Transl Med. 3(4):448-57 (2014); Barrett et al., Stem Cells Trans Med 3:1-6 sctm.2014-0121 (2014); Focosi et al., Blood Cancer Journal 4: e211 (2014); and references cited therein. The production of iPSCs can be achieved by the introduction of nucleic acid sequences encoding stem cell-associated genes into an adult, somatic cell, historically using viral vectors.

iPSCs can be generated or derived from terminally differentiated somatic cells, as well as from adult stem cells, or somatic stem cells. That is, a non-pluripotent progenitor cell can be rendered pluripotent or multipotent by reprogramming. In such instances, it may not be necessary to include as many reprogramming factors as required to reprogram a terminally differentiated cell. Further, reprogramming can be induced by the non-viral introduction of reprogramming factors, *e*.*g*., by introducing the proteins themselves, or by introducing nucleic acids that encode the reprogramming factors, or by introducing messenger RNAs that upon translation produce the reprogramming factors (see *e*.*g*., Warren et al., Cell Stem Cell, 7(5):618-30 (2010). Reprogramming can be achieved by introducing a combination of nucleic acids encoding stem cell-associated genes, including, for example, Oct-4 (also known as Oct-3/4 or Pouf51), SoxI, Sox2, Sox3, Sox 15, Sox 18, NANOG, Klfl, Klf2, Klf4, Klf5, NR5A2, c-Myc, 1-Myc, n-Myc, Rem2, Tert, and LIN28. In one embodiment, reprogramming using the methods and compositions described herein can further comprise introducing one or more of Oct-3/4, a member of the Sox family, a member of the Klf family, and a member of the Myc family to a somatic cell. In one embodiment, the methods and compositions described herein further comprise introducing one or more of each of Oct-4, Sox2, Nanog, c-MYC and Klf4 for reprogramming. As noted above, the exact method used for reprogramming is not necessarily critical to the methods and compositions described herein. However, where cells differentiated from the reprogrammed cells are to be used in, *e.g.,* human therapy, in one embodiment the reprogramming is not effected by a method that alters the genome. Thus, in such embodiments, reprogramming is achieved, *e*.*g*., without the use of viral or plasmid vectors.

The efficiency of reprogramming (*i.e.,* the number of reprogrammed cells) derived from a population of starting cells can be enhanced by the addition of various agents, *e.g.,* small molecules, as shown by Shi et al., Cell-Stem Cell 2:525-528 (2008); Huangfu et al., Nature Biotechnology 26(7):795-797 (2008) and Marson et al., Cell-Stem Cell 3: 132-135 (2008). Thus, an agent or combination of agents that enhance the efficiency or rate of induced pluripotent stem cell production can be used in the production of patient-specific or disease-specific iPSCs. Some non-limiting examples of agents that enhance reprogramming efficiency include soluble Wnt, Wnt conditioned media, BIX-01294 (a G9a histone methyltransferase), PD0325901 (a MEK inhibitor), DNA methyltransferase inhibitors, histone deacetylase (HDAC) inhibitors, valproic acid, 5'-azacytidine, dexamethasone, suberoylanilide, hydroxamic acid (SAHA), vitamin C, and trichostatin (TSA), among others.

Other non-limiting examples of reprogramming enhancing agents include: Suberoylanilide Hydroxamic Acid (SAHA (*e*.*g*., MK0683, vorinostat) and other hydroxamic acids), BML-210, Depudecin (*e*.*g*., (-)-Depudecin), HC Toxin, Nullscript (4-(1,3-Dioxo-1H,3H-benzo[de]isoquinolin-2-yl)-N-hydroxybutanamide), Phenylbutyrate (*e*.*g*., sodium phenylbutyrate) and Valproic Acid ((VP A) and other short chain fatty acids), Scriptaid, Suramin Sodium, Trichostatin A (TSA), APHA Compound 8, Apicidin, Sodium Butyrate, pivaloyloxymethyl butyrate (Pivanex, AN-9), Trapoxin B, Chlamydocin, Depsipeptide (also known as FR901228 or FK228), benzamides (*e.g.,* CI-994 (*e.g.,* N-acetyl dinaline) and MS-27-275), MGCD0103, NVP-LAQ-824, CBHA (m-carboxycinnaminic acid bishydroxamic acid), JNJ16241199, Tubacin, A-161906, proxamide, oxamflatin, 3-Cl-UCHA (*e*.*g*., 6-(3-chlorophenylureido)caproic hydroxamic acid), AOE (2-amino-8-oxo-9, 10-epoxydecanoic acid), CHAP31 and CHAP 50. Other reprogramming enhancing agents include, for example, dominant negative forms of the HDACs (*e.g.,* catalytically inactive forms), siRNA inhibitors of the HDACs, and antibodies that specifically bind to the HDACs. Such inhibitors are available, *e.g.,* from BIOMOL International, Fukasawa, Merck Biosciences, Novartis, Gloucester Pharmaceuticals, Titan Pharmaceuticals, MethylGene, and Sigma Aldrich.

To confirm the induction of pluripotent stem cells for use with the methods described herein, isolated clones can be tested for the expression of a stem cell marker. Such expression in a cell derived from a somatic cell identifies the cells as induced pluripotent stem cells. Stem cell markers are selected from the non-limiting group including SSEA3, SSEA4, CD9, Nanog, Fbxl5, Ecatl, Esgl, Eras, Gdf3, Fgf4, Cripto, Daxl, Zpf296, Slc2a3, Rexl, Utfl, and Natl. In one embodiment, a cell that expresses Oct4 or Nanog is identified as pluripotent. Methods for detecting the expression of such markers can include, for example, RT-PCR and immunological methods that detect the presence of the encoded polypeptides, such as Western blots or flow cytometric analyses. In some embodiments, detection involves not only RT-PCR, but also includes detection of protein markers. Intracellular markers may be best identified via RT-PCR, or protein detection methods such as immunocytochemistry, while cell surface markers are readily identified, *e.g.,* by immunocytochemistry.

The pluripotent stem cell character of isolated cells can be confirmed by tests evaluating the ability of the iPSCs to differentiate into cells of each of the three germ layers. As one example, teratoma formation in nude mice can be used to evaluate the pluripotent character of the isolated clones. The cells are introduced into nude mice and histology and/or immunohistochemistry is performed on a tumor arising from the cells. The growth of a tumor comprising cells from all three germ layers, for example, further indicates that the cells are pluripotent stem cells.

### Neural cells

The human nervous system is estimated to have approximately 360 billion non-neural glial cells and 90 billion nerve cells. There are hundreds of different types of neurons based on morphology alone. Often, neurons that look similar have strikingly different properties. For example, they utilize and respond to different neurotransmitters.

Neural stem cells (NSCs) are self-renewing, multipotent cells that generate the main phenotype of the nervous system. Stem cells are characterized by their capability to differentiate into multiple cell types via exogenous stimuli from their environment. They undergo asymmetric cell division into two daughter cells, one non-specialized and one specialized. NSCs primarily differentiate into neurons, astrocytes, and oligodendrocytes.

The typical neuron transmits electrical signals from one cell to another. Neurons contain a cell body, dendrite, axon hillock, axon, nerve ending and neuromuscular junction. Neurons may be named according to shape or the nature of the dendritic tree.

The most numerous cellular constituents of the central nervous system are the non-neuronal, neuroglial ("nerve glue") cells that occupy the space between neurons. It has been estimated that there are roughly 360 billion glial cells, which comprise 80-90% of the cells in the CNS. Neuroglia differ from neurons in several general ways in that they: do not form synapses, have essentially only one type of process, retain the ability to divide, and are less electrically excitable than neurons. Neuroglia are classified based on size and shape of their neucleus and distinguished from neurons, at the light microscopic level. Neuroglia are divided into two major categories based on size, the macroglia and the microglia. The macroglia are of ectodermal origin and consist of astrocytes, oligodendrocytes and ependymal cells. Microglia cells are probably of mesodermal origin.

### Creating patient specific iPSCs

One step of the *ex vivo* methods of the disclosure involves creating a patient specific iPS cell, patient specific iPS cells, or a patient specific iPS cell line. There are many established methods in the art for creating patient specific iPS cells, as described in Takahashi and Yamanaka 2006; Takahashi, Tanabe *et al.* 2007. For example, the creating step comprises: a) isolating a somatic cell, such as a skin cell or fibroblast, from the patient; and b) introducing a set of pluripotency-associated genes into the somatic cell in order to induce the cell to become a pluripotent stem cell. In some embodiments, the set of pluripotency-associated genes is one or more of the genes selected from the group consisting of OCT4, SOX2, KLF4, Lin28, NANOG, and cMYC.

### Performing a biopsy of the patient's bone marrow

A biopsy is a sample of tissue or fluid taken from the body. There are many different kinds of biopsies. Nearly all of them involve using a sharp tool to remove a small amount of tissue. If the biopsy will be on the skin or other sensitive area, numbing medicine is applied first. A biopsy may be performed according to any of the known methods in the art. For example, in a bone marrow biopsy, a large needle is used to enter the pelvis bone to collect bone marrow.

### Isolating a white blood cell

White blood cells may be isolated according to any method known in the art. For example, white blood cells may be isolated from a liquid sample by centrifugation.

### Isolating a mesenchymal stem cell

Mesenchymal stem cells may be isolated according to any method known in the art. For example, marrow aspirate is collected into a syringe with heparin. Cells are washed and centrifuged on a Percoll. The cells are cultured in Dulbecco's modified Eagle's medium (DMEM) (low glucose) containing 10% fetal bovine serum (FBS) (Pittinger MF, Mackay AM, Beck SC et al., Science 1999; 284:143-147).

### Treating a patient with GCSF

A patient may optionally be treated with granulocyte colony stimulating factor (GCSF) in accordance with any method known in the art. In some embodiments, the GCSF is administered in combination with Plerixaflor.

### Isolating a hematopoietic progenitor cell from a patient

A hematopoietic progenitor cell may be isolated from a patient by any method known in the art. CD34+ cells are enriched using CliniMACS® Cell Selection System (Miltenyi Biotec). In some embodiments, CD34+ cells are weakly stimulated in serum-free medium (e.g., CellGrow SCGM media, CellGenix) with cytokines (e.g., SCF, rhTPO, rhFLT3) before genome editing.

### Genome Editing

Genome editing generally refers to the process of modifying the nucleotide sequence of a genome, preferably in a precise or pre-determined manner. Examples of methods of genome editing described herein include methods of using site-directed nucleases to cut deoxyribonucleic acid (DNA) at precise target locations in the genome, thereby creating double-strand or single-strand DNA breaks at particular locations within the genome. Such breaks can be and regularly are repaired by natural, endogenous cellular processes, such as homology-directed repair (HDR) and non-homologous end-joining (NHEJ), as recently reviewed in Cox et al., Nature Medicine 21 (2), 121-31 (2015). NHEJ directly joins the DNA ends resulting from a double-strand break, sometimes with the loss or addition of nucleotide sequence, which may disrupt or enhance gene expression. HDR utilizes a homologous sequence, or donor sequence, as a template for inserting a defined DNA sequence at the break point. The homologous sequence may be in the endogenous genome, such as a sister chromatid. Alternatively, the donor may be an exogenous nucleic acid, such as a plasmid, a single-strand oligonucleotide, a double-stranded oligonucleotide, a duplex oligonucleotide or a virus, that has regions of high homology with the nuclease-cleaved locus, but which may also contain additional sequence or sequence changes including deletions that can be incorporated into the cleaved target locus. A third repair mechanism is microhomology-mediated end joining (MMEJ), also referred to as "Alternative NHEJ", in which the genetic outcome is similar to NHEJ in that small deletions and insertions can occur at the cleavage site. MMEJ makes use of homologous sequences of a few basepairs flanking the DNA break site to drive a more favored DNA end joining repair outcome, and recent reports have further elucidated the molecular mechanism of this process; see, *e.g.,* Cho and Greenberg, Nature 518, 174-76 (2015); Kent et al., Nature Structural and Molecular Biology, Adv. Online doi:10.1038/nsmb.2961 (2015); Mateos-Gomez et al., Nature 518, 254-57 (2015); Ceccaldi et al., Nature 528, 258-62 (2015). In some instances it may be possible to predict likely repair outcomes based on analysis of potential microhomologies at the site of the DNA break.

Each of these genome editing mechanisms can be used to create desired genomic alterations. A step in the genome editing process is to create one or two DNA breaks, the latter as double-strand breaks or as two single-stranded breaks, in the target locus as close as possible to the site of intended mutation. This can be achieved via the use of site-directed polypeptides, as described and illustrated herein.

Site-directed polypeptides, such as a DNA endonuclease, can introduce double-strand breaks or single-strand breaks in nucleic acids, e.g., genomic DNA. The double-strand break can stimulate a cell's endogenous DNA-repair pathways (e.g., homology-dependent repair or non-homologous end joining or alternative non-homologous end joining (A-NHEJ) or microhomology-mediated end joining). NHEJ can repair cleaved target nucleic acid without the need for a homologous template. This can sometimes result in small deletions or insertions (indels) in the target nucleic acid at the site of cleavage, and can lead to disruption or alteration of gene expression. HDR can occur when a homologous repair template, or donor, is available. The homologous donor template comprises sequences that are homologous to sequences flanking the target nucleic acid cleavage site. The sister chromatid is generally used by the cell as the repair template. However, for the purposes of genome editing, the repair template is often supplied as an exogenous nucleic acid, such as a plasmid, duplex oligonucleotide, single-strand oligonucleotide, double-stranded oligonucleotide, or viral nucleic acid. With exogenous donor templates, it is common to introduce an additional nucleic acid sequence (such as a transgene) or modification (such as a single or multiple base change or a deletion) between the flanking regions of homology so that the additional or altered nucleic acid sequence also becomes incorporated into the target locus. MMEJ results in a genetic outcome that is similar to NHEJ in that small deletions and insertions can occur at the cleavage site. MMEJ makes use of homologous sequences of a few basepairs flanking the cleavage site to drive a favored end-joining DNA repair outcome. In some instances it may be possible to predict likely repair outcomes based on analysis of potential microhomologies in the nuclease target regions.

Thus, in some cases, homologous recombination is used to insert an exogenous polynucleotide sequence into the target nucleic acid cleavage site. An exogenous polynucleotide sequence is termed a donor polynucleotide (or donor or donor sequence or polynucleotide donor template) herein. In some embodiments, the donor polynucleotide, a portion of the donor polynucleotide, a copy of the donor polynucleotide, or a portion of a copy of the donor polynucleotide is inserted into the target nucleic acid cleavage site. In some embodiments, the donor polynucleotide is an exogenous polynucleotide sequence, *i.e.,* a sequence that does not naturally occur at the target nucleic acid cleavage site.

The modifications of the target DNA due to NHEJ and/or HDR can lead to, for example, mutations, deletions, alterations, integrations, gene correction, gene replacement, gene tagging, transgene insertion, nucleotide deletion, gene disruption, translocations and/or gene mutation. The processes of deleting genomic DNA and integrating non-native nucleic acid into genomic DNA are examples of genome editing.

### CRISPR Endonuclease System

A CRISPR (Clustered Regularly Interspaced Short Palindromic Repeats) genomic locus can be found in the genomes of many prokaryotes (*e*.*g*., bacteria and archaea). In prokaryotes, the CRISPR locus encodes products that function as a type of immune system to help defend the prokaryotes against foreign invaders, such as virus and phage. There are three stages of CRISPR locus function: integration of new sequences into the locus, biogenesis of CRISPR RNA (crRNA), and silencing of foreign invader nucleic acid. Five types of CRISPR systems (*e*.*g*., Type I, Type II, Type III, Type U, and Type V) have been identified.

A CRISPR locus includes a number of short repeating sequences referred to as "repeats." The repeats can form hairpin structures and/or comprise unstructured single-stranded sequences. The repeats usually occur in clusters and frequently diverge between species. The repeats are regularly interspaced with unique intervening sequences referred to as "spacers," resulting in a repeat-spacer-repeat locus architecture. The spacers are identical to or have high homology with known foreign invader sequences. A spacer-repeat unit encodes a crisprRNA (crRNA), which is processed into a mature form of the spacer-repeat unit. A crRNA comprises a "seed" or spacer sequence that is involved in targeting a target nucleic acid (in the naturally occurring form in prokaryotes, the spacer sequence targets the foreign invader nucleic acid). A spacer sequence is located at the 5' or 3' end of the crRNA.

A CRISPR locus also comprises polynucleotide sequences encoding CRISPR Associated (Cas) genes. Cas genes encode endonucleases involved in the biogenesis and the interference stages of crRNA function in prokaryotes. Some Cas genes comprise homologous secondary and/or tertiary structures.

### Type II CRISPR Systems

crRNA biogenesis in a Type II CRISPR system in nature requires a transactivating CRISPR RNA (tracrRNA). The tracrRNA is modified by endogenous RNaselll, and then hybridizes to a crRNA repeat in the pre-crRNA array. Endogenous RNaseIII is recruited to cleave the pre-crRNA. Cleaved crRNAs are subjected to exoribonuclease trimming to produce the mature crRNA form (*e*.*g*., 5' trimming). The tracrRNA remains hybridized to the crRNA, and the tracrRNA and the crRNA associate with a site-directed polypeptide (*e*.*g*., Cas9). The crRNA of the crRNA-tracrRNA-Cas9 complex guides the complex to a target nucleic acid to which the crRNA can hybridize. Hybridization of the crRNA to the target nucleic acid activates Cas9 for targeted nucleic acid cleavage. The target nucleic acid in a Type II CRISPR system is referred to as a protospacer adjacent motif (PAM). In nature, the PAM is essential to facilitate binding of a site-directed polypeptide (*e*.*g*., Cas9) to the target nucleic acid. Type II systems (also referred to as Nmeni or CASS4) are further subdivided into Type II-A (CASS4) and II-B (CASS4a). Jinek et al., Science, 337(6096):816-821 (2012) showed that the CRISPR/Cas9 system is useful for RNA-programmable genome editing, and international patent application publication number WO2013/176772 provides numerous examples and applications of the CRISPR/Cas endonuclease system for site-specific gene editing.

### Type V CRISPR Systems

Type V CRISPR systems have several important differences from Type II systems. For example, Cpf1 is a single RNA-guided endonuclease that, in contrast to Type II systems, lacks tracrRNA. In fact, Cpf1-associated CRISPR arrays are processed into mature crRNAS without the requirement of an additional transactivating tracrRNA. The Type V CRISPR array is processed into short mature crRNAs of 42-44 nucleotides in length, with each mature crRNA beginning with 19 nucleotides of direct repeat followed by 23-25 nucleotides of spacer sequence. In contrast, mature crRNAs in Type II systems start with 20-24 nucleotides of spacer sequence followed by about 22 nucleotides of direct repeat. Also, Cpf1 utilizes a T-rich protospacer-adjacent motif such that Cpf1-crRNA complexes efficiently cleave target DNA preceded by a short T-rich PAM, which is in contrast to the G-rich PAM following the target DNA for Type II systems. Thus, Type V systems cleave at a point that is distant from the PAM, while Type II systems cleave at a point that is adjacent to the PAM. In addition, in contrast to Type II systems, Cpf1 cleaves DNA via a staggered DNA double-stranded break with a 4 or 5 nucleotide 5' overhang. Type II systems cleave via a blunt double-stranded break. Similar to Type II systems, Cpf1 contains a predicted RuvC-like endonuclease domain, but lacks a second HNH endonuclease domain, which is in contrast to Type II systems.

### Cas Genes/Polypeptides and Protospacer Adjacent Motifs

Exemplary CRISPR/Cas polypeptides include the Cas9 polypeptides in Fig. 1 of Fonfara et al., Nucleic Acids Research, 42:2577-2590 (2014). The CRISPR/Cas gene naming system has undergone extensive rewriting since the Cas genes were discovered. Fig. 5 of Fonfara, *supra,* provides PAM sequences for the Cas9 polypeptides from various species.

### Site-Directed Polypeptides

A site-directed polypeptide is a nuclease used in genome editing to cleave DNA. The site-directed may be administered to a cell or a patient as either: one or more polypeptides, or one or more mRNAs encoding the polypeptide.

In the context of a CRISPR/Cas or CRISPR/Cpf1 system, the site-directed polypeptide can bind to a guide RNA that, in turn, specifies the site in the target DNA to which the polypeptide is directed. In embodiments of CRISPR/Cas or CRISPR/Cpf1 systems herein, the site-directed polypeptide is an endonuclease, such as a DNA endonuclease.

In some embodiments, a site-directed polypeptide comprises a plurality of nucleic acid-cleaving (*i.e.,* nuclease) domains. Two or more nucleic acid-cleaving domains can be linked together via a linker. In some embodiments, the linker comprises a flexible linker. Linkers may comprise 1, 2, 3, 4, 5, 6, 7, 8, 9, 10, 11, 12, 13, 14, 15, 16, 17, 18, 19, 20, 21, 22, 23, 24, 25, 30, 35, 40 or more amino acids in length.

Naturally-occurring wild-type Cas9 enzymes comprise two nuclease domains, a HNH nuclease domain and a RuvC domain. Herein, the "Cas9" refers to both naturally-occurring and recombinant Cas9s. Cas9 enzymes contemplated herein comprises a HNH or HNH-like nuclease domain, and/or a RuvC or RuvC-like nuclease domain.

HNH or HNH-like domains comprise a McrA-like fold. HNH or HNH-like domains comprises two antiparallel β-strands and an α-helix. HNH or HNH-like domains comprises a metal binding site (*e*.*g*., a divalent cation binding site). HNH or HNH-like domains can cleave one strand of a target nucleic acid (*e*.*g*., the complementary strand of the crRNA targeted strand).

RuvC or RuvC-like domains comprise an RNaseH or RNaseH-like fold. RuvC/RNaseH domains are involved in a diverse set of nucleic acid-based functions including acting on both RNA and DNA. The RNaseH domain comprises 5 β-strands surrounded by a plurality of α-helices. RuvC/RNaseH or RuvC/RNaseH-like domains comprise a metal binding site (*e*.*g*., a divalent cation binding site). RuvC/RNaseH or RuvC/RNaseH-like domains can cleave one strand of a target nucleic acid (*e.g*., the non-complementary strand of a double-stranded target DNA).

Site-directed polypeptides can introduce double-strand breaks or single-strand breaks in nucleic acids, *e.g.,* genomic DNA. The double-strand break can stimulate a cell's endogenous DNA-repair pathways (*e*.*g*., homology-dependent repair (HDR) or non-homologous end joining (NHEJ) or alternative non-homologous end joining (A-NHEJ) or microhomology-mediated end joining (MMEJ)). NHEJ can repair cleaved target nucleic acid without the need for a homologous template. This can sometimes result in small deletions or insertions (indels) in the target nucleic acid at the site of cleavage, and can lead to disruption or alteration of gene expression. HDR can occur when a homologous repair template, or donor, is available. The homologous donor template comprises sequences that are homologous to sequences flanking the target nucleic acid cleavage site. The sister chromatid is generally used by the cell as the repair template. However, for the purposes of genome editing, the repair template is often supplied as an exogenous nucleic acid, such as a plasmid, duplex oligonucleotide, single-strand oligonucleotide or viral nucleic acid. With exogenous donor templates, it is common to introduce an additional nucleic acid sequence (such as a transgene) or modification (such as a single or multiple base change or a deletion) between the flanking regions of homology so that the additional or altered nucleic acid sequence also becomes incorporated into the target locus. MMEJ results in a genetic outcome that is similar to NHEJ in that small deletions and insertions can occur at the cleavage site. MMEJ makes use of homologous sequences of a few basepairs flanking the cleavage site to drive a favored end-joining DNA repair outcome. In some instances it may be possible to predict likely repair outcomes based on analysis of potential microhomologies in the nuclease target regions.

Thus, in some cases, homologous recombination is used to insert an exogenous polynucleotide sequence into the target nucleic acid cleavage site. An exogenous polynucleotide sequence is termed a donor polynucleotide (or donor or donor sequence) herein. In some embodiments, the donor polynucleotide, a portion of the donor polynucleotide, a copy of the donor polynucleotide, or a portion of a copy of the donor polynucleotide is inserted into the target nucleic acid cleavage site. In some embodiments, the donor polynucleotide is an exogenous polynucleotide sequence, *i.e.,* a sequence that does not naturally occur at the target nucleic acid cleavage site.

The modifications of the target DNA due to NHEJ and/or HDR can lead to, for example, mutations, deletions, alterations, integrations, gene correction, gene replacement, gene tagging, transgene insertion, nucleotide deletion, gene disruption, translocations and/or gene mutation. The processes of deleting genomic DNA and integrating non-native nucleic acid into genomic DNA are examples of genome editing.

In some embodiments, the site-directed polypeptide comprises an amino acid sequence having at least 10%, at least 15%, at least 20%, at least 30%, at least 40%, at least 50%, at least 60%, at least 70%, at least 75%, at least 80%, at least 85%, at least 90%, at least 95%, at least 99%, or 100% amino acid sequence identity to a wild-type exemplary site-directed polypeptide [*e*.*g*., Cas9 from *S. pyogenes,* US2014/0068797 Sequence ID No. 8 or Sapranauskas et al., Nucleic Acids Res, 39(21): 9275-9282 (2011)], and various other site-directed polypeptides).

In some embodiments, the site-directed polypeptide comprises an amino acid sequence having at least 10%, at least 15%, at least 20%, at least 30%, at least 40%, at least 50%, at least 60%, at least 70%, at least 75%, at least 80%, at least 85%, at least 90%, at least 95%, at least 99%, or 100% amino acid sequence identity to the nuclease domain of a wild-type exemplary site-directed polypeptide (*e.g.,* Cas9 from *S. pyogenes, supra*).

In some embodiments, a site-directed polypeptide comprises at least 70, 75, 80, 85, 90, 95, 97, 99, or 100% identity to a wild-type site-directed polypeptide (*e.g.,* Cas9 from *S. pyogenes, supra*) over 10 contiguous amino acids. In some embodiments, a site-directed polypeptide comprises at most: 70, 75, 80, 85, 90, 95, 97, 99, or 100% identity to a wild-type site-directed polypeptide (*e.g*., Cas9 from *S*. *pyogenes, supra*) over 10 contiguous amino acids. In some embodiments, a site-directed polypeptide comprises at least: 70, 75, 80, 85, 90, 95, 97, 99, or 100% identity to a wild-type site-directed polypeptide (*e.g.,* Cas9 from *S. pyogenes, supra*) over 10 contiguous amino acids in a HNH nuclease domain of the site-directed polypeptide. In some embodiments, a site-directed polypeptide comprises at most: 70, 75, 80, 85, 90, 95, 97, 99, or 100% identity to a wild-type site-directed polypeptide (*e.g.,* Cas9 from *S. pyogenes, supra*) over 10 contiguous amino acids in a HNH nuclease domain of the site-directed polypeptide. In some embodiments, a site-directed polypeptide comprises at least: 70, 75, 80, 85, 90, 95, 97, 99, or 100% identity to a wild-type site-directed polypeptide (*e.g.,* Cas9 from *S. pyogenes, supra*) over 10 contiguous amino acids in a RuvC nuclease domain of the site-directed polypeptide. In some embodiments, a site-directed polypeptide comprises at most: 70, 75, 80, 85, 90, 95, 97, 99, or 100% identity to a wild-type site-directed polypeptide (*e.g.,* Cas9 from *S. pyogenes, supra*) over 10 contiguous amino acids in a RuvC nuclease domain of the site-directed polypeptide.

In some embodiments, the site-directed polypeptide comprises a modified form of a wild-type exemplary site-directed polypeptide. The modified form of the wild- type exemplary site-directed polypeptide comprises a mutation that reduces the nucleic acid-cleaving activity of the site-directed polypeptide. In some embodiments, the modified form of the wild-type exemplary site-directed polypeptide has less than 90%, less than 80%, less than 70%, less than 60%, less than 50%, less than 40%, less than 30%, less than 20%, less than 10%, less than 5%, or less than 1% of the nucleic acid-cleaving activity of the wild-type exemplary site-directed polypeptide (*e.g.,* Cas9 from *S. pyogenes, supra*). The modified form of the site-directed polypeptide can have no substantial nucleic acid-cleaving activity. When a site-directed polypeptide is a modified form that has no substantial nucleic acid-cleaving activity, it is referred to herein as "enzymatically inactive."

In some embodiments, the modified form of the site-directed polypeptide comprises a mutation such that it can induce a single-strand break (SSB) on a target nucleic acid (*e.g.,* by cutting only one of the sugar-phosphate backbones of a double-strand target nucleic acid). In some embodiments, the mutation results in less than 90%, less than 80%, less than 70%, less than 60%, less than 50%, less than 40%, less than 30%, less than 20%, less than 10%, less than 5%, or less than 1% of the nucleic acid-cleaving activity in one or more of the plurality of nucleic acid-cleaving domains of the wild-type site directed polypeptide (*e.g.,* Cas9 from S. *pyogenes, supra*). In some embodiments, the mutation results in one or more of the plurality of nucleic acid-cleaving domains retaining the ability to cleave the complementary strand of the target nucleic acid, but reducing its ability to cleave the non-complementary strand of the target nucleic acid. In some embodiments, the mutation results in one or more of the plurality of nucleic acid-cleaving domains retaining the ability to cleave the non-complementary strand of the target nucleic acid, but reducing its ability to cleave the complementary strand of the target nucleic acid. For example, residues in the wild-type exemplary *S. pyogenes* Cas9 polypeptide, such as Asp10, His840, Asn854 and Asn856, are mutated to inactivate one or more of the plurality of nucleic acid-cleaving domains (*e.g.,* nuclease domains). In some embodiments, the residues to be mutated correspond to residues Asp10, His840, Asn854 and Asn856 in the wild-type exemplary S. *pyogenes* Cas9 polypeptide (*e.g.,* as determined by sequence and/or structural alignment). Non-limiting examples of mutations include D10A, H840A, N854A or N856A. One skilled in the art will recognize that mutations other than alanine substitutions are suitable.

In some embodiments, a D10A mutation is combined with one or more of H840A, N854A, or N856A mutations to produce a site-directed polypeptide substantially lacking DNA cleavage activity. In some embodiments, a H840A mutation is combined with one or more of D10A, N854A, or N856A mutations to produce a site-directed polypeptide substantially lacking DNA cleavage activity. In some embodiments, a N854A mutation is combined with one or more of H840A, D10A, or N856A mutations to produce a site-directed polypeptide substantially lacking DNA cleavage activity. In some embodiments, a N856A mutation is combined with one or more of H840A, N854A, or D10A mutations to produce a site-directed polypeptide substantially lacking DNA cleavage activity. Site-directed polypeptides that comprise one substantially inactive nuclease domain are referred to as "nickases".

Nickase variants of RNA-guided endonucleases, for example Cas9, can be used to increase the specificity of CRISPR-mediated genome editing. Wild type Cas9 is typically guided by a single guide RNA designed to hybridize with a specified -20 nucleotide sequence in the target sequence (such as an endogenous genomic locus). However, several mismatches can be tolerated between the guide RNA and the target locus, effectively reducing the length of required homology in the target site to, for example, as little as 13 nt of homology, and thereby resulting in elevated potential for binding and double-strand nucleic acid cleavage by the CRISPR/Cas9 complex elsewhere in the target genome - also known as off-target cleavage. Because nickase variants of Cas9 each only cut one strand, in order to create a double-strand break it is necessary for a pair of nickases to bind in close proximity and on opposite strands of the target nucleic acid, thereby creating a pair of nicks, which is the equivalent of a double-strand break. This requires that two separate guide RNAs - one for each nickase - must bind in close proximity and on opposite strands of the target nucleic acid. This requirement essentially doubles the minimum length of homology needed for the double-strand break to occur, thereby reducing the likelihood that a double-strand cleavage event will occur elsewhere in the genome, where the two guide RNA sites - if they exist - are unlikely to be sufficiently close to each other to enable the double-strand break to form. As described in the art, nickases can also be used to promote HDR versus NHEJ. HDR can be used to introduce selected changes into target sites in the genome through the use of specific donor sequences that effectively mediate the desired changes. Descriptions of various CRISPR/Cas systems for use in gene editing can be found, *e.g.,* in international patent application publication number WO2013/176772, and in Nature Biotechnology 32, 347-355 (2014), and references cited therein.

Mutations contemplated include substitutions, additions, and deletions, or any combination thereof. In some embodiments, the mutation converts the mutated amino acid to alanine. In some embodiments, the mutation converts the mutated amino acid to another amino acid (*e.g.,* glycine, serine, threonine, cysteine, valine, leucine, isoleucine, methionine, proline, phenylalanine, tyrosine, tryptophan, aspartic acid, glutamic acid, asparagines, glutamine, histidine, lysine, or arginine). In some embodiments, the mutation converts the mutated amino acid to a non-natural amino acid (*e.g.,* selenomethionine). In some embodiments, the mutation converts the mutated amino acid to amino acid mimics (*e.g.,* phosphomimics). In some embodiments, the mutation is a conservative mutation. For example, the mutation can convert the mutated amino acid to amino acids that resemble the size, shape, charge, polarity, conformation, and/or rotamers of the mutated amino acids (*e.g.,* cysteine/serine mutation, lysine/asparagine mutation, histidine/phenylalanine mutation). In some embodiments, the mutation causes a shift in reading frame and/or the creation of a premature stop codon. In some embodiments, mutations cause changes to regulatory regions of genes or loci that affect expression of one or more genes.

In some embodiments, the site-directed polypeptide (*e.g.,* variant, mutated, enzymatically inactive and/or conditionally enzymatically inactive site-directed polypeptide) targets nucleic acid. In some embodiments, the site-directed polypeptide (*e.g.,* variant, mutated, enzymatically inactive and/or conditionally enzymatically inactive endoribonuclease) targets RNA. In some embodiments, the site-directed polypeptide (*e.g.,* variant, mutated, enzymatically inactive and/or conditionally enzymatically inactive endoribonuclease) targets DNA.

In some embodiments, the site-directed polypeptide comprises one or more non-native sequences (*e.g.,* the site-directed polypeptide is a fusion protein).

In some embodiments, the site-directed polypeptide comprises an amino acid sequence comprising at least 15% amino acid identity to a Cas9 from a bacterium (*e.g., S. pyogenes*), a nucleic acid binding domain, and two nucleic acid cleaving domains (*i.e.,* a HNH domain and a RuvC domain).

In some embodiments, the site-directed polypeptide comprises an amino acid sequence comprising at least 15% amino acid identity to a Cas9 from a bacterium (*e.g., S. pyogenes*), and two nucleic acid cleaving domains (*i.e.,* a HNH domain and a RuvC domain).

In some embodiments, the site-directed polypeptide comprises an amino acid sequence comprising at least 15% amino acid identity to a Cas9 from a bacterium (*e.g., S. pyogenes*), and two nucleic acid cleaving domains, wherein one or both of the nucleic acid cleaving domains comprise at least 50% amino acid identity to a nuclease domain from Cas9 from a bacterium (*e.g*., *S. pyogenes*).

In some embodiments, the site-directed polypeptide comprises an amino acid sequence comprising at least 15% amino acid identity to a Cas9 from a bacterium (*e.g., S. pyogenes*), two nucleic acid cleaving domains (*i.e.,* a HNH domain and a RuvC domain), and non-native sequence (for example, a nuclear localization signal) or a linker linking the site-directed polypeptide to a non-native sequence.

In some embodiments, the site-directed polypeptide comprises an amino acid sequence comprising at least 15% amino acid identity to a Cas9 from a bacterium (*e.g., S. pyogenes*), two nucleic acid cleaving domains (*i.e.,* a HNH domain and a RuvC domain), wherein the site-directed polypeptide comprises a mutation in one or both of the nucleic acid cleaving domains that reduces the cleaving activity of the nuclease domains by at least 50%.

In some embodiments, the site-directed polypeptide comprises an amino acid sequence comprising at least 15% amino acid identity to a Cas9 from a bacterium (*e.g., S. pyogenes*), and two nucleic acid cleaving domains (*i.e.,* a HNH domain and a RuvC domain), wherein one of the nuclease domains comprises mutation of aspartic acid 10, and/or wherein one of the nuclease domains comprises mutation of histidine 840, and wherein the mutation reduces the cleaving activity of the nuclease domain(s) by at least 50%.

In some embodiments of the disclosure, the one or more site-directed polypeptides, e.g. DNA endonucleases, include two nickases that together effect one double-strand break at a specific locus in the genome, or four nickases that together effect two double-strand breaks at specific loci in the genome. Alternatively, one site-directed polypeptide, e.g. DNA endonuclease, effects one double-strand break at a specific locus in the genome.

### Genome-targeting Nucleic Acid

The present disclosure provides a genome-targeting nucleic acid that can direct the activities of an associated polypeptide (*e.g.,* a site-directed polypeptide) to a specific target sequence within a target nucleic acid. In some embodiments, the genome-targeting nucleic acid is an RNA. A genome-targeting RNA is referred to as a "guide RNA" or "gRNA" herein. A guide RNA comprises at least a spacer sequence that hybridizes to a target nucleic acid sequence of interest, and a CRISPR repeat sequence. In Type II systems, the gRNA also comprises a tracrRNA sequence. In the Type II guide RNA, the CRISPR repeat sequence and tracrRNA sequence hybridize to each other to form a duplex. In the Type V guide RNA, the crRNA forms a duplex. In both systems, the duplex binds a site-directed polypeptide, such that the guide RNA and site-direct polypeptide form a complex. The genome-targeting nucleic acid provides target specificity to the complex by virtue of its association with the site-directed polypeptide. The genome-targeting nucleic acid thus directs the activity of the site-directed polypeptide.

Exemplary guide RNAs include the spacer sequences in SEQ ID NOs: 1-18807, shown with the genome location of their target sequence and the associated Cas9 cut site, wherein the genome location is based on the GRCh38/hg38 human genome assembly. As is understood by the person of ordinary skill in the art, each guide RNA is designed to include a spacer sequence complementary to its genomic target sequence. For example, each of the spacer sequences in SEQ ID NOs: 1-18807 may be put into a single RNA chimera or a crRNA (along with a corresponding tracrRNA). See Jinek et al., Science, 337, 816-821 (2012) and Deltcheva et al., Nature, 471, 602-607 (2011).

In some embodiments, the genome-targeting nucleic acid is a double-molecule guide RNA. In some embodiments, the genome-targeting nucleic acid is a single-molecule guide RNA.

A double-molecule guide RNA comprises two strands of RNA. The first strand comprises in the 5' to 3' direction, an optional spacer extension sequence, a spacer sequence and a minimum CRISPR repeat sequence. The second strand comprises a minimum tracrRNA sequence (complementary to the minimum CRISPR repeat sequence), a 3' tracrRNA sequence and an optional tracrRNA extension sequence.

A single-molecule guide RNA in a Type II system comprises, in the 5' to 3' direction, an optional spacer extension sequence, a spacer sequence, a minimum CRISPR repeat sequence, a single-molecule guide linker, a minimum tracrRNA sequence, a 3' tracrRNA sequence and an optional tracrRNA extension sequence. The optional tracrRNA extension may comprise elements that contribute additional functionality (*e.g.,* stability) to the guide RNA. The single-molecule guide linker links the minimum CRISPR repeat and the minimum tracrRNA sequence to form a hairpin structure. The optional tracrRNA extension comprises one or more hairpins.

A single-molecule guide RNA in a Type V system comprises, in the 5' to 3' direction, a minimum CRISPR repeat sequence and a spacer sequence.

By way of illustration, guide RNAs used in the CRISPR/Cas system, or other smaller RNAs can be readily synthesized by chemical means, as illustrated below and described in the art. While chemical synthetic procedures are continually expanding, purifications of such RNAs by procedures such as high performance liquid chromatography (HPLC, which avoids the use of gels such as PAGE) tends to become more challenging as polynucleotide lengths increase significantly beyond a hundred or so nucleotides. One approach used for generating RNAs of greater length is to produce two or more molecules that are ligated together. Much longer RNAs, such as those encoding a Cas9 endonuclease, are more readily generated enzymatically. Various types of RNA modifications can be introduced during or after chemical synthesis and/or enzymatic generation of RNAs, *e.g.,* modifications that enhance stability, reduce the likelihood or degree of innate immune response, and/or enhance other attributes, as described in the art.

### Spacer Extension Sequence

In some embodiments of genome-targeting nucleic acids, a spacer extension sequence can provide stability and/or provide a location for modifications of a genome-targeting nucleic acid. A spacer extension sequence can modify on- or off-target activity or specificity. In some embodiments, a spacer extension sequence is provided. A spacer extension sequence may have a length of more than 1, 5, 10, 15, 20, 25, 30, 35, 40, 45, 50, 60, 70, 80, 90, 100, 120, 140, 160, 180, 200, 220, 240, 260, 280, 300, 320, 340, 360, 380, 400, 1000, 2000, 3000, 4000, 5000, 6000, or 7000 or more nucleotides. A spacer extension sequence may have a length of less than 1, 5, 10, 15, 20, 25, 30, 35, 40, 45, 50, 60, 70, 80, 90, 100, 120, 140, 160, 180, 200, 220, 240, 260, 280, 300, 320, 340, 360, 380, 400, 1000, 2000, 3000, 4000, 5000, 6000, 7000 or more nucleotides. In some embodiments, a spacer extension sequence is less than 10 nucleotides in length. In some embodiments, a spacer extension sequence is between 10-30 nucleotides in length. In some embodiments, a spacer extension sequence is between 30-70 nucleotides in length.

In some embodiments, the spacer extension sequence comprises another moiety (*e.g.,* a stability control sequence, an endoribonuclease binding sequence, a ribozyme). In some embodiments, the moiety increases the stability of a nucleic acid targeting nucleic acid. In some embodiments, the moiety is a transcriptional terminator segment (*i.e.,* a transcription termination sequence). In some embodiments, the moiety functions in a eukaryotic cell. In some embodiments, the moiety functions in a prokaryotic cell. In some embodiments, the moiety functions in both eukaryotic and prokaryotic cells. Non-limiting examples of suitable moieties include: a 5' cap (*e.g.,* a 7-methylguanylate cap (m7 G)), a riboswitch sequence (*e.g.,* to allow for regulated stability and/or regulated accessibility by proteins and protein complexes), a sequence that forms a dsRNA duplex (*i.e.,* a hairpin), a sequence that targets the RNA to a subcellular location (*e.g.,* nucleus, mitochondria, chloroplasts, and the like), a modification or sequence that provides for tracking (e.g., direct conjugation to a fluorescent molecule, conjugation to a moiety that facilitates fluorescent detection, a sequence that allows for fluorescent detection, etc.), and/or a modification or sequence that provides a binding site for proteins (*e.g.,* proteins that act on DNA, including transcriptional activators, transcriptional repressors, DNA methyltransferases, DNA demethylases, histone acetyltransferases, histone deacetylases, and the like).

### Spacer Sequence

The spacer sequence hybridizes to a sequence in a target nucleic acid of interest. The spacer of a genome-targeting nucleic acid interacts with a target nucleic acid in a sequence-specific manner via hybridization (*i.e.,* base pairing). The nucleotide sequence of the spacer thus varies depending on the sequence of the target nucleic acid of interest.

In a CRISPR/Cas system herein, the spacer sequence is designed to hybridize to a target nucleic acid that is located 5' of a PAM of the Cas9 enzyme used in the system. The spacer may perfectly match the target sequence or may have mismatches. Each Cas9 enzyme has a particular PAM sequence that it recognizes in a target DNA. For example, *S. pyogenes* recognizes in a target nucleic acid a PAM that comprises the sequence 5'-NRG-3', where R comprises either A or G, where N is any nucleotide and N is immediately 3' of the target nucleic acid sequence targeted by the spacer sequence.

In some embodiments, the target nucleic acid sequence comprises 20 nucleotides. In some embodiments, the target nucleic acid comprises more than 20 nucleotides. In some embodiments, the target nucleic acid comprises less than 20 nucleotides. In some embodiments, the target nucleic acid comprises at least: 5, 10, 15, 16, 17, 18, 19, 20, 21, 22, 23, 24, 25, 30 or more nucleotides. In some embodiments, the target nucleic acid comprises at most: 5, 10, 15, 16, 17, 18, 19, 20, 21, 22, 23, 24, 25, 30 or more nucleotides. In some embodiments, the target nucleic acid sequence comprises 20 bases immediately 5' of the first nucleotide of the PAM. For example, in a sequence comprising 5'-NNNNNNNNNNNNNNNNNNNNNRG-3' (SEQ ID NO: 18808), the target nucleic acid comprises the sequence that corresponds to the Ns, wherein N is any nucleotide , and the underlined NRG sequence is the *S. pyogenes* PAM.

In some embodiments, the spacer sequence that hybridizes to the target nucleic acid has a length of at least about 6 nucleotides (nt). The spacer sequence can be at least about 6 nt, at least about 10 nt, at least about 15 nt, at least about 18 nt, at least about 19 nt, at least about 20 nt, at least about 25 nt, at least about 30 nt, at least about 35 nt or at least about 40 nt, from about 6 nt to about 80 nt, from about 6 nt to about 50 nt, from about 6 nt to about 45 nt, from about 6 nt to about 40 nt, from about 6 nt to about 35 nt, from about 6 nt to about 30 nt, from about 6 nt to about 25 nt, from about 6 nt to about 20 nt, from about 6 nt to about 19 nt, from about 10 nt to about 50 nt, from about 10 nt to about 45 nt, from about 10 nt to about 40 nt, from about 10 nt to about 35 nt, from about 10 nt to about 30 nt, from about 10 nt to about 25 nt, from about 10 nt to about 20 nt, from about 10 nt to about 19 nt, from about 19 nt to about 25 nt, from about 19 nt to about 30 nt, from about 19 nt to about 35 nt, from about 19 nt to about 40 nt, from about 19 nt to about 45 nt, from about 19 nt to about 50 nt, from about 19 nt to about 60 nt, from about 20 nt to about 25 nt, from about 20 nt to about 30 nt, from about 20 nt to about 35 nt, from about 20 nt to about 40 nt, from about 20 nt to about 45 nt, from about 20 nt to about 50 nt, or from about 20 nt to about 60 nt. In some embodiments, the spacer sequence comprises 20 nucleotides. In some embodiments, the spacer comprises 19 nucleotides.

In some embodiments, the percent complementarity between the spacer sequence and the target nucleic acid is at least about 30%, at least about 40%, at least about 50%, at least about 60%, at least about 65%, at least about 70%, at least about 75%, at least about 80%, at least about 85%, at least about 90%, at least about 95%, at least about 97%, at least about 98%, at least about 99%, or 100%. In some embodiments, the percent complementarity between the spacer sequence and the target nucleic acid is at most about 30%, at most about 40%, at most about 50%, at most about 60%, at most about 65%, at most about 70%, at most about 75%, at most about 80%, at most about 85%, at most about 90%, at most about 95%, at most about 97%, at most about 98%, at most about 99%, or 100%. In some embodiments, the percent complementarity between the spacer sequence and the target nucleic acid is 100% over the six contiguous 5'-most nucleotides of the target sequence of the complementary strand of the target nucleic acid. In some embodiments, the percent complementarity between the spacer sequence and the target nucleic acid is at least 60% over about 20 contiguous nucleotides. The length of the spacer sequence and the target nucleic acid can differ by 1 to 6 nucleotides, which may be thought of as a bulge or bulges.

In some embodiments, a spacer sequence is designed or chosen using a computer program. The computer program can use variables, such as predicted melting temperature, secondary structure formation, predicted annealing temperature, sequence identity, genomic context, chromatin accessibility, % GC, frequency of genomic occurrence (e.g., of sequences that are identical or are similar but vary in one or more spots as a result of mismatch, insertion or deletion), methylation status, presence of SNPs, and the like.

### Minimum CRISPR Repeat Sequence

In some embodiments, a minimum CRISPR repeat sequence is a sequence with at least about 30%, about 40%, about 50%, about 60%, about 65%, about 70%, about 75%, about 80%, about 85%, about 90%, about 95%, or 100% sequence identity to a reference CRISPR repeat sequence (*e.g.,* crRNA from S. *pyogenes*).

A minimum CRISPR repeat sequence comprises nucleotides that can hybridize to a minimum tracrRNA sequence in a cell. The minimum CRISPR repeat sequence and a minimum tracrRNA sequence form a duplex, *i.e.* a base-paired double-stranded structure. Together, the minimum CRISPR repeat sequence and the minimum tracrRNA sequence bind to the site-directed polypeptide. At least a part of the minimum CRISPR repeat sequence hybridizes to the minimum tracrRNA sequence. In some embodiments, at least a part of the minimum CRISPR repeat sequence comprises at least about 30%, about 40%, about 50%, about 60%, about 65%, about 70%, about 75%, about 80%, about 85%, about 90%, about 95%, or 100% complementary to the minimum tracrRNA sequence. In some embodiments, at least a part of the minimum CRISPR repeat sequence comprises at most about 30%, about 40%, about 50%, about 60%, about 65%, about 70%, about 75%, about 80%, about 85%, about 90%, about 95%, or 100% complementary to the minimum tracrRNA sequence.

The minimum CRISPR repeat sequence can have a length from about 7 nucleotides to about 100 nucleotides. For example, the length of the minimum CRISPR repeat sequence is from about 7 nucleotides (nt) to about 50 nt, from about 7 nt to about 40 nt, from about 7 nt to about 30 nt, from about 7 nt to about 25 nt, from about 7 nt to about 20 nt, from about 7 nt to about 15 nt, from about 8 nt to about 40 nt, from about 8 nt to about 30 nt, from about 8 nt to about 25 nt, from about 8 nt to about 20 nt, from about 8 nt to about 15 nt, from about 15 nt to about 100 nt, from about 15 nt to about 80 nt, from about 15 nt to about 50 nt, from about 15 nt to about 40 nt, from about 15 nt to about 30 nt, or from about 15 nt to about 25 nt. In some embodiments, the minimum CRISPR repeat sequence is approximately 9 nucleotides in length. In some embodiments, the minimum CRISPR repeat sequence is approximately 12 nucleotides in length.

In some embodiments, the minimum CRISPR repeat sequence is at least about 60% identical to a reference minimum CRISPR repeat sequence (*e.g.,* wild-type crRNA from *S. pyogenes)* over a stretch of at least 6, 7, or 8 contiguous nucleotides. For example, the minimum CRISPR repeat sequence is at least about 65% identical, at least about 70% identical, at least about 75% identical, at least about 80% identical, at least about 85% identical, at least about 90% identical, at least about 95% identical, at least about 98% identical, at least about 99% identical or 100% identical to a reference minimum CRISPR repeat sequence over a stretch of at least 6, 7, or 8 contiguous nucleotides.

### Minimum tracrRNA Sequence

In some embodiments, a minimum tracrRNA sequence is a sequence with at least about 30%, about 40%, about 50%, about 60%, about 65%, about 70%, about 75%, about 80%, about 85%, about 90%, about 95%, or 100% sequence identity to a reference tracrRNA sequence (*e.g.,* wild type tracrRNA from S. *pyogenes*).

A minimum tracrRNA sequence comprises nucleotides that hybridize to a minimum CRISPR repeat sequence in a cell. A minimum tracrRNA sequence and a minimum CRISPR repeat sequence form a duplex, *i.e.* a base-paired double-stranded structure. Together, the minimum tracrRNA sequence and the minimum CRISPR repeat bind to a site-directed polypeptide. At least a part of the minimum tracrRNA sequence can hybridize to the minimum CRISPR repeat sequence. In some embodiments, the minimum tracrRNA sequence is at least about 30%, about 40%, about 50%, about 60%, about 65%, about 70%, about 75%, about 80%, about 85%, about 90%, about 95%, or 100% complementary to the minimum CRISPR repeat sequence.

The minimum tracrRNA sequence can have a length from about 7 nucleotides to about 100 nucleotides. For example, the minimum tracrRNA sequence can be from about 7 nucleotides (nt) to about 50 nt, from about 7 nt to about 40 nt, from about 7 nt to about 30 nt, from about 7 nt to about 25 nt, from about 7 nt to about 20 nt, from about 7 nt to about 15 nt, from about 8 nt to about 40 nt, from about 8 nt to about 30 nt, from about 8 nt to about 25 nt, from about 8 nt to about 20 nt, from about 8 nt to about 15 nt, from about 15 nt to about 100 nt, from about 15 nt to about 80 nt, from about 15 nt to about 50 nt, from about 15 nt to about 40 nt, from about 15 nt to about 30 nt or from about 15 nt to about 25 nt long. In some embodiments, the minimum tracrRNA sequence is approximately 9 nucleotides in length. In some embodiments, the minimum tracrRNA sequence is approximately 12 nucleotides. In some embodiments, the minimum tracrRNA consists of tracrRNA nt 23-48 described in Jinek *et al., supra.*

In some embodiments, the minimum tracrRNA sequence is at least about 60% identical to a reference minimum tracrRNA (*e.g.,* wild type, tracrRNA from *S. pyogenes*) sequence over a stretch of at least 6, 7, or 8 contiguous nucleotides. For example, the minimum tracrRNA sequence is at least about 65% identical, about 70% identical, about 75% identical, about 80% identical, about 85% identical, about 90% identical, about 95% identical, about 98% identical, about 99% identical or 100% identical to a reference minimum tracrRNA sequence over a stretch of at least 6, 7, or 8 contiguous nucleotides.

In some embodiments, the duplex between the minimum CRISPR RNA and the minimum tracrRNA comprises a double helix. In some embodiments, the duplex between the minimum CRISPR RNA and the minimum tracrRNA comprises at least about 1, 2, 3, 4, 5, 6, 7, 8, 9, or 10 or more nucleotides. In some embodiments, the duplex between the minimum CRISPR RNA and the minimum tracrRNA comprises at most about 1, 2, 3, 4, 5, 6, 7, 8, 9, or 10 or more nucleotides.

In some embodiments, the duplex comprises a mismatch (*i.e.,* the two strands of the duplex are not 100% complementary). In some embodiments, the duplex comprises at least about 1, 2, 3, 4, or 5 or mismatches. In some embodiments, the duplex comprises at most about 1, 2, 3, 4, or 5 or mismatches. In some embodiments, the duplex comprises no more than 2 mismatches.

### Bulges

In some embodiments, there is a "bulge" in the duplex between the minimum CRISPR RNA and the minimum tracrRNA. The bulge is an unpaired region of nucleotides within the duplex. In some embodiments, the bulge contributes to the binding of the duplex to the site-directed polypeptide. A bulge comprises, on one side of the duplex, an unpaired 5'-XXXY-3' where X is any purine and Y comprises a nucleotide that can form a wobble pair with a nucleotide on the opposite strand, and an unpaired nucleotide region on the other side of the duplex. The number of unpaired nucleotides on the two sides of the duplex can be different.

In one example, the bulge comprises an unpaired purine (*e.g.,* adenine) on the minimum CRISPR repeat strand of the bulge. In some embodiments, a bulge comprises an unpaired 5'-AAGY-3' of the minimum tracrRNA sequence strand of the bulge, where Y comprises a nucleotide that can form a wobble pairing with a nucleotide on the minimum CRISPR repeat strand.

In some embodiments, a bulge on the minimum CRISPR repeat side of the duplex comprises at least 1, 2, 3, 4, or 5 or more unpaired nucleotides. In some embodiments, a bulge on the minimum CRISPR repeat side of the duplex comprises at most 1, 2, 3, 4, or 5 or more unpaired nucleotides. In some embodiments, a bulge on the minimum CRISPR repeat side of the duplex comprises 1 unpaired nucleotide.

In some embodiments, a bulge on the minimum tracrRNA sequence side of the duplex comprises at least 1, 2, 3, 4, 5, 6, 7, 8, 9, or 10 or more unpaired nucleotides. In some embodiments, a bulge on the minimum tracrRNA sequence side of the duplex comprises at most 1, 2, 3, 4, 5, 6, 7, 8, 9, or 10 or more unpaired nucleotides. In some embodiments, a bulge on a second side of the duplex (*e.g.,* the minimum tracrRNA sequence side of the duplex) comprises 4 unpaired nucleotides.

In some embodiments, a bulge comprises at least one wobble pairing. In some embodiments, a bulge comprises at most one wobble pairing. In some embodiments, a bulge comprises at least one purine nucleotide. In some embodiments, a bulge comprises at least 3 purine nucleotides. In some embodiments, a bulge sequence comprises at least 5 purine nucleotides. In some embodiments, a bulge sequence comprises at least one guanine nucleotide. In some embodiments, a bulge sequence comprises at least one adenine nucleotide.

### Hairpins

In various embodiments, one or more hairpins are located 3' to the minimum tracrRNA in the 3' tracrRNA sequence.

In some embodiments, the hairpin starts at least about 1, 2, 3, 4, 5, 6, 7, 8, 9, 10, 15, or 20 or more nucleotides 3' from the last paired nucleotide in the minimum CRISPR repeat and minimum tracrRNA sequence duplex. In some embodiments, the hairpin can start at most about 1, 2, 3, 4, 5, 6, 7, 8, 9 or 10 or more nucleotides 3' of the last paired nucleotide in the minimum CRISPR repeat and minimum tracrRNA sequence duplex.

In some embodiments, a hairpin comprises at least about 1, 2, 3, 4, 5, 6, 7, 8, 9, 10, 15, or 20 or more consecutive nucleotides. In some embodiments, a hairpin comprises at most about 1, 2, 3, 4, 5, 6, 7, 8, 9, 10, 15, or more consecutive nucleotides.

In some embodiments, a hairpin comprises a CC dinucleotide (*i.e.,* two consecutive cytosine nucleotides).

In some embodiments, a hairpin comprises duplexed nucleotides (*e.g.,* nucleotides in a hairpin, hybridized together). For example, a hairpin comprises a CC dinucleotide that is hybridized to a GG dinucleotide in a hairpin duplex of the 3' tracrRNA sequence.

One or more of the hairpins can interact with guide RNA-interacting regions of a site-directed polypeptide.

In some embodiments, there are two or more hairpins, and in some embodiments there are three or more hairpins.

### 3' tracrRNA sequence

In some embodiments, a 3' tracrRNA sequence comprises a sequence with at least about 30%, about 40%, about 50%, about 60%, about 65%, about 70%, about 75%, about 80%, about 85%, about 90%, about 95%, or 100% sequence identity to a reference tracrRNA sequence (*e.g.,* a tracrRNA from *S. pyogenes).*

In some embodiments, the 3' tracrRNA sequence has a length from about 6 nucleotides to about 100 nucleotides. For example, the 3' tracrRNA sequence can have a length from about 6 nucleotides (nt) to about 50 nt, from about 6 nt to about 40 nt, from about 6 nt to about 30 nt, from about 6 nt to about 25 nt, from about 6 nt to about 20 nt, from about 6 nt to about 15 nt, from about 8 nt to about 40 nt, from about 8 nt to about 30 nt, from about 8 nt to about 25 nt, from about 8 nt to about 20 nt, from about 8 nt to about 15 nt, from about 15 nt to about 100 nt, from about 15 nt to about 80 nt, from about 15 nt to about 50 nt, from about 15 nt to about 40 nt, from about 15 nt to about 30 nt, or from about 15 nt to about 25 nt. In some embodiments, the 3' tracrRNA sequence has a length of approximately 14 nucleotides.

In some embodiments, the 3' tracrRNA sequence is at least about 60% identical to a reference 3' tracrRNA sequence (*e.g.,* wild type 3' tracrRNA sequence from *S. pyogenes)* over a stretch of at least 6, 7, or 8 contiguous nucleotides. For example, the 3' tracrRNA sequence is at least about 60% identical, about 65% identical, about 70% identical, about 75% identical, about 80% identical, about 85% identical, about 90% identical, about 95% identical, about 98% identical, about 99% identical, or 100% identical, to a reference 3' tracrRNA sequence (*e.g.,* wild type 3' tracrRNA sequence from *S. pyogenes)* over a stretch of at least 6, 7, or 8 contiguous nucleotides.

In some embodiments, a 3' tracrRNA sequence comprises more than one duplexed region (*e.g.,* hairpin, hybridized region). In some embodiments, a 3' tracrRNA sequence comprises two duplexed regions.

In some embodiments, the 3' tracrRNA sequence comprises a stem loop structure. In some embodiments, a stem loop structure in the 3' tracrRNA comprises at least 1, 2, 3, 4, 5, 6, 7, 8, 9, 10, 15 or 20 or more nucleotides. In some embodiments, the stem loop structure in the 3' tracrRNA comprises at most 1, 2, 3, 4, 5, 6, 7, 8, 9 or 10 or more nucleotides. In some embodiments, the stem loop structure comprises a functional moiety. For example, the stem loop structure may comprise an aptamer, a ribozyme, a protein-interacting hairpin, a CRISPR array, an intron, or an exon. In some embodiments, the stem loop structure comprises at least about 1, 2, 3, 4, or 5 or more functional moieties. In some embodiments, the stem loop structure comprises at most about 1, 2, 3, 4, or 5 or more functional moieties.

In some embodiments, the hairpin in the 3' tracrRNA sequence comprises a P-domain. In some embodiments, the P-domain comprises a double-stranded region in the hairpin.

### tracrRNA Extension Sequence

A tracrRNA extension sequence may be provided whether the tracrRNA is in the context of single-molecule guides or double-molecule guides. In some embodiments, a tracrRNA extension sequence has a length from about 1 nucleotide to about 400 nucleotides. In some embodiments, a tracrRNA extension sequence has a length of more than 1, 5, 10, 15, 20, 25, 30, 35, 40, 45, 50, 60, 70, 80, 90, 100, 120, 140, 160, 180, 200, 220, 240, 260, 280, 300, 320, 340, 360, 380, or 400 nucleotides. In some embodiments, a tracrRNA extension sequence has a length from about 20 to about 5000 or more nucleotides. In some embodiments, a tracrRNA extension sequence has a length of more than 1000 nucleotides. In some embodiments, a tracrRNA extension sequence has a length of less than 1, 5, 10, 15, 20, 25, 30, 35, 40, 45, 50, 60, 70, 80, 90, 100, 120, 140, 160, 180, 200, 220, 240, 260, 280, 300, 320, 340, 360, 380, 400 or more nucleotides. In some embodiments, a tracrRNA extension sequence can have a length of less than 1000 nucleotides. In some embodiments, a tracrRNA extension sequence comprises less than 10 nucleotides in length. In some embodiments, a tracrRNA extension sequence is 10-30 nucleotides in length. In some embodiments, tracrRNA extension sequence is 30-70 nucleotides in length.

In some embodiments, the tracrRNA extension sequence comprises a functional moiety (*e.g.,* a stability control sequence, ribozyme, endoribonuclease binding sequence). In some embodiments, the functional moiety comprises a transcriptional terminator segment (*i.e.,* a transcription termination sequence). In some embodiments, the functional moiety has a total length from about 10 nucleotides (nt) to about 100 nucleotides, from about 10 nt to about 20 nt, from about 20 nt to about 30 nt, from about 30 nt to about 40 nt, from about 40 nt to about 50 nt, from about 50 nt to about 60 nt, from about 60 nt to about 70 nt, from about 70 nt to about 80 nt, from about 80 nt to about 90 nt, or from about 90 nt to about 100 nt, from about 15 nt to about 80 nt, from about 15 nt to about 50 nt, from about 15 nt to about 40 nt, from about 15 nt to about 30 nt, or from about 15 nt to about 25 nt. In some embodiments, the functional moiety functions in a eukaryotic cell. In some embodiments, the functional moiety functions in a prokaryotic cell. In some embodiments, the functional moiety functions in both eukaryotic and prokaryotic cells.

Non-limiting examples of suitable tracrRNA extension functional moieties include a 3' poly-adenylated tail, a riboswitch sequence (*e.g.,* to allow for regulated stability and/or regulated accessibility by proteins and protein complexes), a sequence that forms a dsRNA duplex (*i.e.,* a hairpin), a sequence that targets the RNA to a subcellular location (*e.g.,* nucleus, mitochondria, chloroplasts, and the like), a modification or sequence that provides for tracking (*e.g.,* direct conjugation to a fluorescent molecule, conjugation to a moiety that facilitates fluorescent detection, a sequence that allows for fluorescent detection, etc.), and/or a modification or sequence that provides a binding site for proteins (*e.g.,* proteins that act on DNA, including transcriptional activators, transcriptional repressors, DNA methyltransferases, DNA demethylases, histone acetyltransferases, histone deacetylases, and the like). In some embodiments, a tracrRNA extension sequence comprises a primer binding site or a molecular index (*e.g.,* barcode sequence). In some embodiments, the tracrRNA extension sequence comprises one or more affinity tags.

### Single-Molecule Guide Linker Sequence

In some embodiments, the linker sequence of a single-molecule guide nucleic acid has a length from about 3 nucleotides to about 100 nucleotides. In Jinek *et al., supra,* for example, a simple 4 nucleotide "tetraloop" (-GAAA-) was used, Science, 337(6096):816-821 (2012). An illustrative linker has a length from about 3 nucleotides (nt) to about 90 nt, from about 3 nt to about 80 nt, from about 3 nt to about 70 nt, from about 3 nt to about 60 nt, from about 3 nt to about 50 nt, from about 3 nt to about 40 nt, from about 3 nt to about 30 nt, from about 3 nt to about 20 nt, from about 3 nt to about 10 nt. For example, the linker can have a length from about 3 nt to about 5 nt, from about 5 nt to about 10 nt, from about 10 nt to about 15 nt, from about 15 nt to about 20 nt, from about 20 nt to about 25 nt, from about 25 nt to about 30 nt, from about 30 nt to about 35 nt, from about 35 nt to about 40 nt, from about 40 nt to about 50 nt, from about 50 nt to about 60 nt, from about 60 nt to about 70 nt, from about 70 nt to about 80 nt, from about 80 nt to about 90 nt, or from about 90 nt to about 100 nt. In some embodiments, the linker of a single-molecule guide nucleic acid is between 4 and 40 nucleotides. In some embodiments, a linker is at least about 100, 500, 1000, 1500, 2000, 2500, 3000, 3500, 4000, 4500, 5000, 5500, 6000, 6500, or 7000 or more nucleotides. In some embodiments, a linker is at most about 100, 500, 1000, 1500, 2000, 2500, 3000, 3500, 4000, 4500, 5000, 5500, 6000, 6500, or 7000 or more nucleotides.

Linkers can comprise any of a variety of sequences, although preferably the linker will not comprise sequences that have extensive regions of homology with other portions of the guide RNA, which might cause intramolecular binding that could interfere with other functional regions of the guide. In Jinek *et al., supra,* a simple 4 nucleotide sequence -GAAA- was used, Science, 337(6096):816-821 (2012), but numerous other sequences, including longer sequences can likewise be used.

In some embodiments, the linker sequence comprises a functional moiety. For example, the linker sequence may comprise one or more features, including an aptamer, a ribozyme, a protein-interacting hairpin, a CRISPR array, an intron, or an exon. In some embodiments, the linker sequence comprises at least about 1, 2, 3, 4, or 5 or more functional moieties. In some embodiments, the linker sequence comprises at most about 1, 2, 3, 4, or 5 or more functional moieties.

### Genome engineering strategies to correct cells by deletion or replacement of the expanded hexanucleotide repeat in the C9ORF72 gene, or by knocking-in C9ORF72 cDNA into the locus of the corresponding gene or safe harbor site

A step of the *ex vivo* methods of the disclosure involves editing/correcting the patient specific iPS cells using genome engineering. Alternatively, a step of the *ex vivo* methods of the disclosure involves editing/correcting the white blood cell, mesenchymal stem cell, or hematopoietic progenitor cell. Likewise, a step of the *in vivo* methods of the disclosure involves editing/correcting the cells in an ALS or FTLD patient using genome engineering. Similarly, a step in the cellular methods of the disclosure involves editing/correcting the C9ORF72 gene in a human cell by genome engineering.

ALS and/or FTLD patients exhibit an expanded hexanucleotide repeat in the C9ORF72 gene. Therefore, different patients will generally require similar correction strategies. Any CRISPR endonuclease may be used in the methods of the invention, each CRISPR endonuclease having its own associated PAM, which may or may not be disease specific. For example, gRNA spacer sequences for targeting the C9ORF72 gene with a CRISPR/Cas9 endonuclease from *S. pyogenes* have been identified in SEQ ID NOs: 1-6148. gRNA spacer sequences for targeting the C9ORF72 gene with a CRISPR/Cas9 endonuclease from *S*. *aureus* have been identified in SEQ ID NOs: 6149-6892. gRNA spacer sequences for targeting the C9ORF72 gene with a CRISPR/Cas9 endonuclease from *S. thermophilus* have been identified in SEQ ID NOs: 7760-8097. gRNA spacer sequences for targeting the C9ORF72 gene with a CRISPR/Cas9 endonuclease from *T. denticola* have been identified in SEQ ID NOs: 8098-8261. gRNA spacer sequences for targeting the C9ORF72 gene with a CRISPR/Cas9 endonuclease from *N. meningitides* have been identified in SEQ ID NOs: 6893-7759. gRNA spacer sequences for targeting the C9ORF72 gene with a CRISPR/Cpf1 endonuclease from *Acidominococcus* and *Lachnospiraceae* have been identified in SEQ ID NOs: 8262-18807. gRNA spacer sequences for targeting, e.g., targeting exon 1-2 of, AAVS1 (PPP1R12C), ALB, Angptl3, ApoC3, ASGR2, CCR5, FIX (F9), Gys2, HGD, Lp(a), Pcsk9, Serpina1, TF, and TTR with a CRISPR/Cas9 endonuclease from *S. pyogenes* have been identified in Examples 7, 13, 19, 25, 31, 37, 43, 49, 55, 61, 67, 73, 79, 85, and 91, respectively. gRNA spacer sequences for targeting, e.g., targeting exon 1-2 of, AAVS1 (PPP1R12C), ALB, Angptl3, ApoC3, ASGR2, CCR5, FIX (F9), Gys2, HGD, Lp(a), Pcsk9, Serpina1, TF, and TTR with a CRISPR/Cas9 endonuclease from S. *aureus* have been identified in Examples 8, 14, 20, 26, 32, 38, 44, 50, 56, 62, 68, 74, 80, and 86, respectively. gRNA spacer sequences for targeting, *e.g.,* targeting exon 1-2 of, AAVS1 (PPP1R12C), ALB, Angptl3, ApoC3, ASGR2, CCR5, FIX (F9), Gys2, HGD, Lp(a), Pcsk9, Serpina1, TF, and TTR with a CRISPR/Cas9 endonuclease from *S. thermophilus* have been identified in Examples 9, 15, 21, 27, 33, 39, 45, 51, 57,63, 69, 75, 81, and 87, respectively. gRNA spacer sequences for targeting, *e.g.,* targeting exon 1-2 of, AAVS1 (PPP1R12C), ALB, Angptl3, ApoC3, ASGR2, CCR5, FIX (F9), Gys2, HGD, Lp(a), Pcsk9, Serpina1, TF, and TTR with a CRISPR/Cas9 endonuclease from *T. denticola* have been identified in Examples 10, 16, 22, 28, 34, 40, 46, 52, 58, 64, 70, 76, 82, and 88, respectively. gRNA spacer sequences for targeting, *e.g.,* targeting exon 1-2 of, AAVS1 (PPP1R12C), ALB, Angptl3, ApoC3, ASGR2, CCR5, FIX (F9), Gys2, HGD, Lp(a), Pcsk9, Serpina1, TF, and TTR with a CRISPR/Cas9 endonuclease from *N. meningitides* have been identified in Examples 11, 17, 23, 29, 35, 41, 47, 53, 59, 65, 71, 77, 83, and 89, respectively. gRNA spacer sequences for targeting, *e.g.,* targeting exon 1-2 of, AAVS1 (PPP1R12C), ALB, Angptl3, ApoC3, ASGR2, CCR5, FIX (F9), Gys2, HGD, Lp(a), Pcsk9, Serpina1, TF, and TTR with a CRISPR/Cas9 endonuclease from *Acidominococcus, Lachnospiraceae, and Francisella novicida* have been identified in Examples 12, 18, 24, 30, 36, 42, 48, 54, 60, 66, 72, 78, 84, and 90, respectively.

For example, the mutation can be corrected by the insertions or deletions that arise due to the imprecise NHEJ repair pathway. If the patient's C9ORF72 gene has an inserted or deleted base, a targeted cleavage can result in a NHEJ-mediated insertion or deletion that restores the frame. Missense mutations can also be corrected through NHEJ-mediated correction using one or more guide RNA. The ability or likelihood of the cut(s) to correct the mutation can be designed or evaluated based on the local sequence and micro-homologies. NHEJ can also be used to delete segments of the gene, either directly or by altering splice donor or acceptor sites through cleavage by one gRNA targeting several locations, or several gRNAs. This may be useful if an amino acid, domain or exon contains the mutations and can be removed or inverted, or if the deletion otherwise restored function to the protein. Pairs of guide strands have been used for deletions and corrections of inversions.

Alternatively, the donor for correction by HDR contains the corrected sequence with small or large flanking homology arms to allow for annealing. HDR is essentially an error-free mechanism that uses a supplied homologous DNA sequence as a template during DSB repair. The rate of homology directed repair (HDR) is a function of the distance between the mutation and the cut site so choosing overlapping or nearest target sites is important. Templates can include extra sequences flanked by the homologous regions or can contain a sequence that differs from the genomic sequence, thus allowing sequence editing.

In addition to correcting mutations by NHEJ or HDR, a range of other options are possible. If there are small or large deletions or multiple mutations, a cDNA can be knocked in that contains the exons affected. A full length cDNA can be knocked into any "safe harbor", but must use a supplied or other promoter. If this construct is knocked into the correct location, it will have physiological control, similar to the normal gene. Pairs of nucleases can be used to delete mutated gene regions, though a donor would usually have to be provided to restore function. In this case two gRNA would be supplied and one donor sequence.

Some genome engineering strategies involve replacement of the expanded hexanucleotide repeat with a wild-type or similar number of hexanucleotide repeats in or near the C9ORF72 gene, or deleting the expanded hexanucleotide repeat and knocking-in C9ORF72 cDNA into the locus of the corresponding gene or a safe harbor locus by homology directed repair (HDR), which is also known as homologous recombination (HR). Homology directed repair is one strategy for treating patients that have expanded hexanucleotides in the C9ORF72 gene. These strategies will restore the C9ORF72 gene and completely reverse the diseased state. This strategy will not likely require a more custom approach based on the length of the patient's expanded hexanucleotide repeat. Donor nucleotides for correcting mutations are small (< 300 bp). This is advantageous, as HDR efficiencies may be inversely related to the size of the donor molecule. Also, it is expected that the donor templates can fit into size constrained viral vector molecules, e.g., including, by way of non-limiting example, size-constrained adeno-associated virus (AAV) molecules, which have been shown to be an effective means of donor template delivery. Also, it is expected that the donor templates can fit into other size constrained molecules, including, by way of non-limiting example, platelets and/or exosomes or other microvesicles.

Homology direct repair is a cellular mechanism for repairing double-stranded breaks (DSBs). The most common form is homologous recombination. There are additional pathways for HDR, including single-strand annealing and alternative-HDR. Genome engineering tools allow researchers to manipulate the cellular homologous recombination pathways to create site-specific modifications to the genome. It has been found that cells can repair a double-stranded break using a synthetic donor molecule provided in trans. Therefore, by introducing a double-stranded break near a specific mutation and providing a suitable donor, targeted changes can be made in the genome. Specific cleavage increases the rate of HDR more than 1,000 fold above the rate of 1 in 10⁶ cells receiving a homologous donor alone. The rate of homology directed repair (HDR) at a particular nucleotide is a function of the distance to the cut site, so choosing overlapping or nearest target sites is important. Gene editing offers the advantage over gene addition, as correcting in situ leaves the rest of the genome unperturbed.

Supplied donors for editing by HDR vary markedly but generally contain the intended sequence with small or large flanking homology arms to allow annealing to the genomic DNA. The homology regions flanking the introduced genetic changes can be 30 bp or smaller, or as large as a multi-kilobase cassette that can contain promoters, cDNAs, etc. Both single-stranded and double-stranded oligonucleotide donors have been used. These oligonucleotides range in size from less than 100 nt to over many kb, though longer ssDNA can also be generated and used. Double-stranded donors are often used, including PCR amplicons, plasmids, and mini-circles. In general, it has been found that an AAV vector is a very effective means of delivery of a donor template, though the packaging limits for individual donors is <5kb. Active transcription of the donor increased HDR three-fold, indicating the inclusion of promoter may increase conversion. Conversely, CpG methylation of the donor decreased gene expression and HDR.

In addition to wildtype endonucleases, such as Cas9, nickase variants exist that have one or the other nuclease domain inactivated resulting in cutting of only one DNA strand. HDR can be directed from individual Cas nickases or using pairs of nickases that flank the target area. Donors can be single-stranded, nicked, or dsDNA.

The donor DNA can be supplied with the nuclease or independently by a variety of different methods, for example by transfection, nano-particle, micro-injection, or viral transduction. A range of tethering options have been proposed to increase the availability of the donors for HDR. Examples include attaching the donor to the nuclease, attaching to DNA binding proteins that bind nearby, or attaching to proteins that are involved in DNA end binding or repair.

The repair pathway choice can be guided by a number of culture conditions, such as those that influence cell cycling, or by targeting of DNA repair and associated proteins. For example, to increase HDR, key NHEJ molecules can be suppressed, such as KU70, KU80 or DNA ligase IV.

Without a donor present, the ends from a DNA break or ends from different breaks can be joined using the several nonhomologous repair pathways in which the DNA ends are joined with little or no base-pairing at the junction. In addition to canonical NHEJ, there are similar repair mechanisms, such as alt-NHEJ. If there are two breaks, the intervening segment can be deleted or inverted. NHEJ repair pathways can lead to insertions, deletions or mutations at the joints.

NHEJ was used to insert a 15-kb inducible gene expression cassette into a defined locus in human cell lines after nuclease cleavage. Maresca, M., Lin, V.G., Guo, N. & Yang, Y., Genome Res 23, 539-546 (2013).

In addition to genome editing by NHEJ or HDR, site-specific gene insertions have been conducted that use both the NHEJ pathway and HR. A combination approach may be applicable in certain settings, possibly including intron/exon borders. NHEJ may prove effective for ligation in the intron, while the error-free HDR may be better suited in the coding region.

As stated previously, the mutation of the C9ORF72 gene is a hexanucleotide repeat expansion of the six letter string of nucleotides GGGGCC. In healthy individuals, there are few repeats of this hexanucleotide, typically about or fewer than 30. In people with the diseases phenotype, the repeat can occur in the order of hundreds. One or more hexanucleotide repeats may be deleted or corrected in order to restore the gene to a wild-type or similar number of hexanucleotide repeats. Alternatively, all of the hexanucleotide repeats may be deleted. As a further alternative, C9ORF72 cDNA may be knocked-in to the locus of the corresponding gene or knocked-in to a safe harbor site, such as a safe harbor locus selected from the group consisting of: AAVS1 (PPP1 R12C), ALB, Angptl3, ApoC3, ASGR2, CCR5, FIX (F9), G6PC, Gys2, HGD, Lp(a), Pcsk9, Serpina1, TF, and TTR. In some embodiments, the methods provide one gRNA or a pair of gRNAs that can be used to facilitate incorporation of a new sequence from a polynucleotide donor template to replace one or more mutations or to knock-in the entire C9ORF72 gene or cDNA.

Some embodiments of the methods provide gRNA pairs that make a deletion by cutting the gene twice, one gRNA cutting at the 5' end of one or more hexanucleotide repeats and the other gRNA cutting at the 3' end of one or more hexanucleotide repeats that facilitates insertion of a new sequence from a polynucleotide donor template to replace the one or more hexanucleotide repeats. The cutting may be accomplished by a pair of DNA endonucleases that each makes a DSB in the genome, or by multiple nickases that together make a DSB in the genome.

Alternatively, some embodiments of the methods provide one gRNA to make one double-strand cut around one or more hexanucleotide repeats that facilitates insertion of a new sequence from a polynucleotide donor template to replace the one or more hexanucleotide repeats. The double-strand cut may be made by a single DNA endonuclease or multiple nickases that together make a DSB in the genome.

Illustrative modifications within the C9ORF72 gene include replacements within or proximal to the mutations referred to above, such as within the region of less than 3 kb, less than 2kb, less than 1 kb, less than 0.5 kb upstream or downstream of the specific mutation. Given the relatively wide variations of hexanucleotide repeats in the C9ORF72 gene, it will be appreciated that numerous variations of the replacements referenced above (including without limitation larger as well as smaller deletions), would be expected to result in restoration of the C9ORF72 gene.

Such variants include replacements that are larger in the 5' and/or 3' direction than the specific mutation in question, or smaller in either direction. Accordingly, by "proximal" with respect to specific replacements, it is intended that the DSB locus associated with a desired replacement boundary (also referred to herein as an endpoint) may be within a region that is less than about 3 kb from the reference locus noted. In some embodiments, the DSB locus is more proximal and within 2 kb, within 1 kb, within 0.5 kb, or within 0.1 kb. In the case of small replacement, the desired endpoint is at or "adjacent to" the reference locus, by which it is intended that the endpoint is within 100 bp, within 50 bp, within 25 bp, or less than about 10 bp to 5 bp from the reference locus.

Embodiments comprising larger or smaller replacements are expected to provide the same benefit, as long as the expanded hexanucleotide repeat is reduced to wild-type levels. It is thus expected that many variations of the replacements described and illustrated herein will be effective for ameliorating ALS and/or FTLD.

Another genome engineering strategy involves exon deletion. Targeted deletion of specific exons is an attractive strategy for treating a large subset of patients with a single therapeutic cocktail. Deletions can either be single hexanucleotide repeat deletions or multi-hexanucleotide repeat deletions. While multi-repeat deletions, including complete deletion of the expanded hexanucleotide repeat, can reach a larger number of patients, for larger deletions the efficiency of deletion greatly decreases with increased size. Therefore, preferred deletions range from 40 to 10,000 base pairs (bp) in size. For example, deletions may range from 40-100; 100-300; 300-500; 500-1,000; 1,000-2,000; 2,000-3,000; 3,000-5,000; or 5,000-10,000 base pairs in size.

In order to ensure that the pre-mRNA is properly processed following deletion, it is important to also delete the surrounding splicing signals. Splicing donor and acceptors are generally within 100 base pairs of the neighboring intron. Therefore, in some embodiments, methods provide all gRNAs that cut approximately +/- 100-3100 bp with respect to each exon/intron junction of interest.

For any of the genome editing strategies, gene editing can be confirmed by sequencing or PCR analysis.

### Target Sequence Selection

Preferentially, shifts in the location of the 5' boundary and/or the 3' boundary relative to particular reference loci are used to facilitate or enhance particular applications of gene editing, which depend in part on the endonuclease system selected for the editing, as further described and illustrated herein.

In a first aspect of such target sequence selection, many endonuclease systems have rules or criteria that guide the initial selection of potential target sites for cleavage, such as the requirement of a PAM sequence motif in a particular position adjacent to the DNA cleavage sites in the case of CRISPR Type II or Type V endonucleases.

In another aspect of target sequence selection or optimization, the frequency of "off-target" activity for a particular combination of target sequence and gene editing endonuclease (*i.e.* the frequency of DSBs occurring at sites other than the selected target sequence) is assessed relative to the frequency of on-target activity. In some cases, cells that have been correctly edited at the desired locus may have a selective advantage relative to other cells. Illustrative, but nonlimiting, examples of a selective advantage include the acquisition of attributes such as enhanced rates of replication, persistence, resistance to certain conditions, enhanced rates of successful engraftment or persistence *in vivo* following introduction into a patient, and other attributes associated with the maintenance or increased numbers or viability of such cells. In other cases, cells that have been correctly edited at the desired locus may be positively selected for by one or more screening methods used to identify, sort or otherwise select for cells that have been correctly edited. Both selective advantage and directed selection methods may take advantage of the phenotype associated with the correction. In some embodiments, cells may be edited two or more times in order to create a second modification that creates a new phenotype that is used to select or purify the intended population of cells. Such a second modification could be created by adding a second gRNA for a selectable or screenable marker. In some embodiments, cells can be correctly edited at the desired locus using a DNA fragment that contains the cDNA and also a selectable marker.

Whether any selective advantage is applicable or any directed selection is to be applied in a particular case, target sequence selection is also guided by consideration of off-target frequencies in order to enhance the effectiveness of the application and/or reduce the potential for undesired alterations at sites other than the desired target. As described further and illustrated herein and in the art, the occurrence of off-target activity is influenced by a number of factors including similarities and dissimilarities between the target site and various off target sites, as well as the particular endonuclease used. In many cases, bioinformatics tools are available that assist in the prediction of off-target activity, and frequently such tools can also be used to identify the most likely sites of off-target activity, which can then be assessed in experimental settings to evaluate relative frequencies of off-target to on-target activity, thereby allowing the selection of sequences that have higher relative on-target activities. Illustrative examples of such techniques are provided herein, and others are known in the art.

Another aspect of target sequence selection relates to homologous recombination events. It is well known that sequences sharing regions of homology can serve as focal points for homologous recombination events that result in deletion of intervening sequences. Such recombination events occur during the normal course of replication of chromosomes and other DNA sequences, and also at other times when DNA sequences are being synthesized, such as in the case of repairs of double-strand breaks (DSBs), which occur on a regular basis during the normal cell replication cycle but may also be enhanced by the occurrence of various events (such as UV light and other inducers of DNA breakage) or the presence of certain agents (such as various chemical inducers). Many such inducers cause DSBs to occur indiscriminately in the genome, and DSBs are regularly being induced and repaired in normal cells. During repair, the original sequence may be reconstructed with complete fidelity, however, in some cases, small insertions or deletions (referred to as "indels") are introduced at the DSB site.

DSBs may also be specifically induced at particular locations, as in the case of the endonucleases systems described herein, which can be used to cause directed or preferential gene modification events at selected chromosomal locations. The tendency for homologous sequences to be subject to recombination in the context of DNA repair (as well as replication) can be taken advantage of in a number of circumstances, and is the basis for one application of gene editing systems, such as CRISPR, in which homology directed repair is used to insert a sequence of interest, provided through use of a "donor" polynucleotide, into a desired chromosomal location.

Regions of homology between particular sequences, which can be small regions of "microhomology" that may comprise as few as ten basepairs or less, can also be used to bring about desired deletions. For example, a single DSB is introduced at a site that exhibits microhomology with a nearby sequence. During the normal course of repair of such DSB, a result that occurs with high frequency is the deletion of the intervening sequence as a result of recombination being facilitated by the DSB and concomitant cellular repair process.

In some circumstances, however, selecting target sequences within regions of homology can also give rise to much larger deletions, including gene fusions (when the deletions are in coding regions), which may or may not be desired given the particular circumstances.

The examples provided herein further illustrate the selection of various target regions for the creation of DSBs designed to induce replacements that result in restoration of wild-type or similar levels of hexanucleotide repeats, as well as the selection of specific target sequences within such regions that are designed to minimize off-target events relative to on-target events.

### Nucleic acid modifications

In some embodiments, polynucleotides introduced into cells comprise one or more modifications that can be used, for example, to enhance activity, stability or specificity, alter delivery, reduce innate immune responses in host cells, or for other enhancements, as further described herein and known in the art.

In certain embodiments, modified polynucleotides are used in the CRISPR/Cas9/Cpf1 system, in which case the guide RNAs (either single-molecule guides or double-molecule guides) and/or a DNA or an RNA encoding a Cas or Cpf1 endonuclease introduced into a cell can be modified, as described and illustrated below. Such modified polynucleotides can be used in the CRISPR/Cas9/Cpf1 system to edit any one or more genomic loci.

Using the CRISPR/Cas9/Cpf1 system for purposes of nonlimiting illustrations of such uses, modifications of guide RNAs can be used to enhance the formation or stability of the CRISPR/Cas9/Cpf1 genome editing complex comprising guide RNAs, which may be single-molecule guides or double-molecule, and a Cas or Cpf1 endonuclease. Modifications of guide RNAs can also or alternatively be used to enhance the initiation, stability or kinetics of interactions between the genome editing complex with the target sequence in the genome, which can be used, for example, to enhance on-target activity. Modifications of guide RNAs can also or alternatively be used to enhance specificity, *e.g.,* the relative rates of genome editing at the on-target site as compared to effects at other (off-target) sites.

Modifications can also or alternatively be used to increase the stability of a guide RNA, *e.g.,* by increasing its resistance to degradation by ribonucleases (RNases) present in a cell, thereby causing its half-life in the cell to be increased. Modifications enhancing guide RNA half-life can be particularly useful in embodiments in which a Cas or Cpf1 endonuclease is introduced into the cell to be edited via an RNA that needs to be translated in order to generate endonuclease, because increasing the half-life of guide RNAs introduced at the same time as the RNA encoding the endonuclease can be used to increase the time that the guide RNAs and the encoded Cas or Cpf1 endonuclease co-exist in the cell.

Modifications can also or alternatively be used to decrease the likelihood or degree to which RNAs introduced into cells elicit innate immune responses. Such responses, which have been well characterized in the context of RNA interference (RNAi), including small-interfering RNAs (siRNAs), as described below and in the art, tend to be associated with reduced half-life of the RNA and/or the elicitation of cytokines or other factors associated with immune responses.

One or more types of modifications can also be made to RNAs encoding an endonuclease that are introduced into a cell, including, without limitation, modifications that enhance the stability of the RNA (such as by increasing its degradation by RNAses present in the cell), modifications that enhance translation of the resulting product (*i.e.* the endonuclease), and/or modifications that decrease the likelihood or degree to which the RNAs introduced into cells elicit innate immune responses.

Combinations of modifications, such as the foregoing and others, can likewise be used. In the case of CRISPR/Cas9/Cpf1, for example, one or more types of modifications can be made to guide RNAs (including those exemplified above), and/or one or more types of modifications can be made to RNAs encoding Cas or Cpf1 endonuclease (including those exemplified above).

By way of illustration, guide RNAs used in the CRISPR/Cas9/Cpf1 system, or other smaller RNAs can be readily synthesized by chemical means, enabling a number of modifications to be readily incorporated, as illustrated below and described in the art. While chemical synthetic procedures are continually expanding, purifications of such RNAs by procedures such as high performance liquid chromatography (HPLC, which avoids the use of gels such as PAGE) tends to become more challenging as polynucleotide lengths increase significantly beyond a hundred or so nucleotides. One approach used for generating chemically-modified RNAs of greater length is to produce two or more molecules that are ligated together. Much longer RNAs, such as those encoding a Cas9 endonuclease, are more readily generated enzymatically. While fewer types of modifications are generally available for use in enzymatically produced RNAs, there are still modifications that can be used to, *e.g.,* enhance stability, reduce the likelihood or degree of innate immune response, and/or enhance other attributes, as described further below and in the art; and new types of modifications are regularly being developed.

By way of illustration of various types of modifications, especially those used frequently with smaller chemically synthesized RNAs, modifications can comprise one or more nucleotides modified at the 2' position of the sugar, in some embodiments a 2'-O-alkyl, 2'-O-alkyl-O-alkyl, or 2'-fluoro-modified nucleotide. In some embodiments, RNA modifications include 2'-fluoro, 2'-amino or 2' O-methyl modifications on the ribose of pyrimidines, abasic residues, or an inverted base at the 3' end of the RNA. Such modifications are routinely incorporated into oligonucleotides and these oligonucleotides have been shown to have a higher Tm (*i.e.,* higher target binding affinity) than 2'-deoxyoligonucleotides against a given target.

A number of nucleotide and nucleoside modifications have been shown to make the oligonucleotide into which they are incorporated more resistant to nuclease digestion than the native oligonucleotide; these modified oligos survive intact for a longer time than unmodified oligonucleotides. Specific examples of modified oligonucleotides include those comprising modified backbones, for example, phosphorothioates, phosphotriesters, methyl phosphonates, short chain alkyl or cycloalkyl intersugar linkages or short chain heteroatomic or heterocyclic intersugar linkages. Some oligonucleotides are oligonucleotides with phosphorothioate backbones and those with heteroatom backbones, particularly CH2 -NH-O-CH2, CH,∼N(CH3)∼O∼CH2 (known as a methylene(methylimino) or MMI backbone), CH2 --O--N (CH3)-CH2, CH2 -N (CH3)-N (CH3)-CH2 and O-N (CH3)- CH2 -CH2 backbones, wherein the native phosphodiester backbone is represented as O- P-- O-CH,); amide backbones [see De Mesmaeker et al., Ace. Chem. Res., 28:366-374 (1995)]; morpholino backbone structures (see Summerton and Weller, U.S. Pat. No. 5,034,506); peptide nucleic acid (PNA) backbone (wherein the phosphodiester backbone of the oligonucleotide is replaced with a polyamide backbone, the nucleotides being bound directly or indirectly to the aza nitrogen atoms of the polyamide backbone, see Nielsen et al., Science 1991, 254, 1497). Phosphorus-containing linkages include, but are not limited to, phosphorothioates, chiral phosphorothioates, phosphorodithioates, phosphotriesters, aminoalkylphosphotriesters, methyl and other alkyl phosphonates comprising 3'alkylene phosphonates and chiral phosphonates, phosphinates, phosphoramidates comprising 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'; see US patent nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050.

Morpholino-based oligomeric compounds are described in Braasch and David Corey, Biochemistry, 41(14): 4503-4510 (2002); Genesis, Volume 30, Issue 3, (2001); Heasman, Dev. Biol., 243: 209-214 (2002); Nasevicius et al., Nat. Genet., 26:216-220 (2000); Lacerra et al., Proc. Natl. Acad. Sci., 97: 9591-9596 (2000); and U.S. Pat. No. 5,034,506, issued Jul. 23, 1991.

Cyclohexenyl nucleic acid oligonucleotide mimetics are described in Wang et al., J. Am. Chem. Soc., 122: 8595-8602 (2000).

Modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These comprise those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S, and CH2 component parts; see US patent nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439.

One or more substituted sugar moieties can also be included, e.g., one of the following at the 2' position: OH, SH, SCH3, F, OCN, OCH3 OCH3, OCH3 O(CH2)n CH3, O(CH2)n NH2, or O(CH2)n CH3, where n is from 1 to about 10; C1 to C10 lower alkyl, alkoxyalkoxy, substituted lower alkyl, alkaryl or aralkyl; Cl; Br; CN; CF3; OCF3; O-, S-, or N-alkyl; O-, S-, or N-alkenyl; SOCH3; SO2 CH3; ONO2; NO2; N3; NH2; heterocycloalkyl; heterocycloalkaryl; aminoalkylamino; polyalkylamino; substituted silyl; an RNA cleaving group; a reporter group; an intercalator; a group for improving the pharmacokinetic properties of an oligonucleotide; or a group for improving the pharmacodynamic properties of an oligonucleotide and other substituents having similar properties. In some embodiments, a modification includes 2'-methoxyethoxy (2'-0-CH2CH2OCH3, also known as 2'-O-(2-methoxyethyl)) (Martin et al, Helv. Chim. Acta, 1995, 78, 486). Other modifications include 2'-methoxy (2'-0-CH3), 2'-propoxy (2'-OCH2 CH2CH3) and 2'-fluoro (2'-F). Similar modifications may also be made at other positions on the oligonucleotide, particularly the 3' position of the sugar on the 3' terminal nucleotide and the 5' position of 5' terminal nucleotide. Oligonucleotides may also have sugar mimetics, such as cyclobutyls in place of the pentofuranosyl group.

In some embodiments, both a sugar and an internucleoside linkage, *i.e.,* the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, for example, an aminoethylglycine backbone. The nucleobases are retained and are bound directly or indirectly to aza nitrogen atoms of the amide portion of the backbone. Representative United States patents that teach the preparation of PNA compounds comprise, but are not limited to, US patent nos. 5,539,082; 5,714,331; and 5,719,262. Further teaching of PNA compounds can be found in Nielsen et al, Science, 254: 1497-1500 (1991).

Guide RNAs can also include, additionally or alternatively, nucleobase (often referred to in the art simply as "base") modifications or substitutions. As used herein, "unmodified" or "natural" nucleobases include adenine (A), guanine (G), thymine (T), cytosine (C), and uracil (U). Modified nucleobases include nucleobases found only infrequently or transiently in natural nucleic acids, *e.g.,* hypoxanthine, 6-methyladenine, 5-Me pyrimidines, particularly 5-methylcytosine (also referred to as 5-methyl-2' deoxycytosine and often referred to in the art as 5-Me-C), 5-hydroxymethylcytosine (HMC), glycosyl HMC and gentobiosyl HMC, as well as synthetic nucleobases, *e.g.,* 2-aminoadenine, 2-(methylamino)adenine, 2-(imidazolylalkyl)adenine, 2-(aminoalklyamino)adenine or other heterosubstituted alkyladenines, 2-thiouracil, 2-thiothymine, 5-bromouracil, 5-hydroxymethyluracil, 8-azaguanine, 7-deazaguanine, N6 (6-aminohexyl)adenine, and 2,6-diaminopurine. Kornberg, A., DNA Replication, W. H. Freeman & Co., San Francisco, pp75-77 (1980); Gebeyehu et al., Nucl. Acids Res. 15:4513 (1997). A "universal" base known in the art, *e.g*., inosine, can also be included. 5-Me-C substitutions have been shown to increase nucleic acid duplex stability by 0.6-1.2 ºC. (Sanghvi, Y. S., in Crooke, S. T. and Lebleu, B., eds., Antisense Research and Applications, CRC Press, Boca Raton, 1993, pp. 276-278) and are embodiments of base substitutions.

Modified nucleobases comprise other synthetic and natural nucleobases, such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudo-uracil), 4-thiouracil, 8-halo, 8-amino, 8-thiol, 8- thioalkyl, 8-hydroxyl and other 8-substituted adenines and guanines, 5-halo particularly 5- bromo, 5-trifluoromethyl and other 5-substituted uracils and cytosines, 7-methylquanine and 7-methyladenine, 8-azaguanine and 8-azaadenine, 7-deazaguanine and 7-deazaadenine, and 3-deazaguanine and 3-deazaadenine.

Further, nucleobases comprise those disclosed in United States Patent No. 3,687,808, those disclosed in 'The Concise Encyclopedia of Polymer Science And Engineering', pages 858-859, Kroschwitz, J.I., ed. John Wiley & Sons, 1990, those disclosed by Englisch et al., Angewandle Chemie, International Edition', 1991, 30, page 613, and those disclosed by Sanghvi, Y. S., Chapter 15, Antisense Research and Applications', pages 289- 302, Crooke, S.T. and Lebleu, B. ea., CRC Press, 1993. Certain of these nucleobases are particularly useful for increasing the binding affinity of the oligomeric compounds of the invention. These include 5-substituted pyrimidines, 6-azapyrimidines and N-2, N-6 and 0-6 substituted purines, comprising 2-aminopropyladenine, 5-propynyluracil and 5-propynylcytosine. 5-methylcytosine substitutions have been shown to increase nucleic acid duplex stability by 0.6-1.2ºC (Sanghvi, Y.S., Crooke, S.T. and Lebleu, B., eds, 'Antisense Research and Applications', CRC Press, Boca Raton, 1993, pp. 276-278) and are embodiments of base substitutions, even more particularly when combined with 2'-O-methoxyethyl sugar modifications. Modified nucleobases are described in US patent nos. 3,687,808, as well as 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,596,091; 5,614,617; 5,681,941; 5,750,692; 5,763,588; 5,830,653; 6,005,096; and U.S. Patent Application Publication 2003/0158403.

Thus, the term "modified" refers to a non-natural sugar, phosphate, or base that is incorporated into a guide RNA, an endonuclease, or both a guide RNA and an endonuclease. It is not necessary for all positions in a given oligonucleotide to be uniformly modified, and in fact more than one of the aforementioned modifications may be incorporated in a single oligonucleotide, or even in a single nucleoside within an oligonucleotide.

In some embodiments, the guide RNAs and/or mRNA (or DNA) encoding an endonuclease are chemically linked to one or more moieties or conjugates that enhance the activity, cellular distribution, or cellular uptake of the oligonucleotide. Such moieties comprise, but are not limited to, lipid moieties such as a cholesterol moiety [Letsinger et al., Proc. Natl. Acad. Sci. USA, 86: 6553-6556 (1989)]; cholic acid [Manoharan et al., Bioorg. Med. Chem. Let., 4: 1053-1060 (1994)]; a thioether, *e.g.,* hexyl-S- tritylthiol [Manoharan et al, Ann. N. Y. Acad. Sci., 660: 306-309 (1992) and Manoharan et al., Bioorg. Med. Chem. Let., 3: 2765-2770 (1993)]; a thiocholesterol [Oberhauser et al., Nucl. Acids Res., 20: 533-538 (1992)]; an aliphatic chain, *e.g.,* dodecandiol or undecyl residues [Kabanov et al., FEBS Lett., 259: 327-330 (1990) and Svinarchuk et al., Biochimie, 75: 49- 54 (1993)]; a phospholipid, *e.g.,* di-hexadecyl-rac-glycerol or triethylammonium 1 ,2-di-O-hexadecyl- rac-glycero-3-H-phosphonate [Manoharan et al., Tetrahedron Lett., 36: 3651-3654 (1995) and Shea et al., Nucl. Acids Res., 18: 3777-3783 (1990)]; a polyamine or a polyethylene glycol chain [Mancharan et al., Nucleosides & Nucleotides, 14: 969-973 (1995)]; adamantane acetic acid [Manoharan et al., Tetrahedron Lett., 36: 3651-3654 (1995)]; a palmityl moiety [(Mishra et al., Biochim. Biophys. Acta, 1264: 229-237 (1995)]; or an octadecylamine or hexylamino-carbonyl-t oxycholesterol moiety [Crooke et al., J. Pharmacol. Exp. Ther., 277: 923-937 (1996)]. See also US Patent Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599, 928 and 5,688,941.

Sugars and other moieties can be used to target proteins and complexes comprising nucleotides, such as cationic polysomes and liposomes, to particular sites. For example, hepatic cell directed transfer can be mediated via asialoglycoprotein receptors (ASGPRs); see, *e.g.,* Hu, et al., Protein Pept Lett. 21(10):1025-30 (2014). Other systems known in the art and regularly developed can be used to target biomolecules of use in the present case and/or complexes thereof to particular target cells of interest.

These targeting moieties or conjugates can include conjugate groups covalently bound to functional groups, such as primary or secondary hydroxyl groups. Conjugate groups of the disclosure include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugate groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve uptake, enhance resistance to degradation, and/or strengthen sequence-specific hybridization with the target nucleic acid. Groups that enhance the pharmacokinetic properties, in the context of this invention, include groups that improve uptake, distribution, metabolism or excretion of the compounds of the present invention. Representative conjugate groups are disclosed in International Patent Application No. PCT/US92/09196, filed Oct. 23, 1992, and U.S. Pat. No. 6,287,860. Conjugate moieties include, but are not limited to, lipid moieties such as a cholesterol moiety, cholic acid, a thioether, *e.g.,* hexyl-5-tritylthiol, a thiocholesterol, an aliphatic chain, e.g., dodecandiol or undecyl residues, a phospholipid, *e.g.,* di-hexadecyl-rac- glycerol or triethylammonium I,2-di-O-hexadecyl-rac-glycero-3-H-phosphonate, a polyamine or a polyethylene glycol chain, or adamantane acetic acid, a palmityl moiety, or an octadecylamine or hexylamino-carbonyl-oxy cholesterol moiety. See, e.g., U.S. Pat. Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

Longer polynucleotides that are less amenable to chemical synthesis and are typically produced by enzymatic synthesis can also be modified by various means. Such modifications can include, for example, the introduction of certain nucleotide analogs, the incorporation of particular sequences or other moieties at the 5' or 3' ends of molecules, and other modifications. By way of illustration, the mRNA encoding Cas9 is approximately 4 kb in length and can be synthesized by *in vitro* transcription. Modifications to the mRNA can be applied to, *e.g.,* increase its translation or stability (such as by increasing its resistance to degradation with a cell), or to reduce the tendency of the RNA to elicit an innate immune response that is often observed in cells following introduction of exogenous RNAs, particularly longer RNAs such as that encoding Cas9.

Numerous such modifications have been described in the art, such as polyA tails, 5' cap analogs (*e.g.,* Anti Reverse Cap Analog (ARCA) or m7G(5')ppp(5')G (mCAP)), modified 5' or 3' untranslated regions (UTRs), use of modified bases (such as Pseudo-UTP, 2-Thio-UTP, 5-Methylcytidine-5'-Triphosphate (5-Methyl-CTP) or N6-Methyl-ATP), or treatment with phosphatase to remove 5' terminal phosphates. These and other modifications are known in the art, and new modifications of RNAs are regularly being developed.

There are numerous commercial suppliers of modified RNAs, including for example, TriLink Biotech, AxoLabs, Bio-Synthesis Inc., Dharmacon and many others. As described by TriLink, for example, 5-Methyl-CTP can be used to impart desirable characteristics, such as increased nuclease stability, increased translation or reduced interaction of innate immune receptors with *in vitro* transcribed RNA. 5-Methylcytidine-5'-Triphosphate (5-Methyl-CTP), N6-Methyl-ATP, as well as Pseudo-UTP and 2-Thio-UTP, have also been shown to reduce innate immune stimulation in culture and *in vivo* while enhancing translation, as illustrated in publications by Kormann *et al.* and Warren *et al.* referred to below.

It has been shown that chemically modified mRNA delivered *in vivo* can be used to achieve improved therapeutic effects; see, *e.g.,* Kormann et al., Nature Biotechnology 29, 154-157 (2011). Such modifications can be used, for example, to increase the stability of the RNA molecule and/or reduce its immunogenicity. Using chemical modifications such as Pseudo-U, N6-Methyl-A, 2-Thio-U and 5-Methyl-C, it was found that substituting just one quarter of the uridine and cytidine residues with 2-Thio-U and 5-Methyl-C respectively resulted in a significant decrease in toll-like receptor (TLR) mediated recognition of the mRNA in mice. By reducing the activation of the innate immune system, these modifications can be used to effectively increase the stability and longevity of the mRNA *in vivo*; see, *e.g.,* Kormann *et al., supra.*

It has also been shown that repeated administration of synthetic messenger RNAs incorporating modifications designed to bypass innate anti-viral responses can reprogram differentiated human cells to pluripotency. See, *e.g.,* Warren, et al., Cell Stem Cell, 7(5):618-30 (2010). Such modified mRNAs that act as primary reprogramming proteins can be an efficient means of reprogramming multiple human cell types. Such cells are referred to as induced pluripotency stem cells (iPSCs), and it was found that enzymatically synthesized RNA incorporating 5-Methyl-CTP, Pseudo-UTP and an Anti Reverse Cap Analog (ARCA) could be used to effectively evade the cell's antiviral response; see, *e.g.,* Warren *et al., supra.*

Other modifications of polynucleotides described in the art include, for example, the use of polyA tails, the addition of 5' cap analogs (such as m7G(5')ppp(5')G (mCAP)), modifications of 5' or 3' untranslated regions (UTRs), or treatment with phosphatase to remove 5' terminal phosphates - and new approaches are regularly being developed.

A number of compositions and techniques applicable to the generation of modified RNAs for use herein have been developed in connection with the modification of RNA interference (RNAi), including small-interfering RNAs (siRNAs). siRNAs present particular challenges *in vivo* because their effects on gene silencing via mRNA interference are generally transient, which can require repeat administration. In addition, siRNAs are double-stranded RNAs (dsRNA) and mammalian cells have immune responses that have evolved to detect and neutralize dsRNA, which is often a by-product of viral infection. Thus, there are mammalian enzymes such as PKR (dsRNA-responsive kinase), and potentially retinoic acid-inducible gene I (RIG-I), that can mediate cellular responses to dsRNA, as well as Toll-like receptors (such as TLR3, TLR7 and TLR8) that can trigger the induction of cytokines in response to such molecules; see, *e.g.,* the reviews by Angart et al., Pharmaceuticals (Basel) 6(4): 440-468 (2013); Kanasty et al., Molecular Therapy 20(3): 513-524 (2012); Burnett et al., Biotechnol J. 6(9):1130-46 (2011); Judge and MacLachlan, Hum Gene Ther 19(2):111-24 (2008); and references cited therein.

A large variety of modifications have been developed and applied to enhance RNA stability, reduce innate immune responses, and/or achieve other benefits that can be useful in connection with the introduction of polynucleotides into human cells, as described herein; see, *e.g.,* the reviews by Whitehead KA et al., Annual Review of Chemical and Biomolecular Engineering, 2: 77-96 (2011); Gaglione and Messere, Mini Rev Med Chem, 10(7):578-95 (2010); Chernolovskaya et al, Curr Opin Mol Ther., 12(2):158-67 (2010); Deleavey et al., Curr Protoc Nucleic Acid Chem Chapter 16:Unit 16.3 (2009); Behlke, Oligonucleotides 18(4):305-19 (2008); Fucini et al., Nucleic Acid Ther 22(3): 205-210 (2012); Bremsen et al., Front Genet 3:154 (2012).

As noted above, there are a number of commercial suppliers of modified RNAs, many of which have specialized in modifications designed to improve the effectiveness of siRNAs. A variety of approaches are offered based on various findings reported in the literature. For example, Dharmacon notes that replacement of a non-bridging oxygen with sulfur (phosphorothioate, PS) has been extensively used to improve nuclease resistance of siRNAs, as reported by Kole, Nature Reviews Drug Discovery 11:125-140 (2012). Modifications of the 2'-position of the ribose have been reported to improve nuclease resistance of the internucleotide phosphate bond while increasing duplex stability (Tm), which has also been shown to provide protection from immune activation. A combination of moderate PS backbone modifications with small, well-tolerated 2'-substitutions (2'-O-Methyl, 2'-Fluoro, 2'-Hydro) have been associated with highly stable siRNAs for applications *in vivo,* as reported by Soutschek et al. Nature 432:173-178 (2004); and 2'-O-Methyl modifications have been reported to be effective in improving stability as reported by Volkov, Oligonucleotides 19:191-202 (2009). With respect to decreasing the induction of innate immune responses, modifying specific sequences with 2'-O-Methyl, 2'-Fluoro, 2'-Hydro have been reported to reduce TLR7/TLR8 interaction while generally preserving silencing activity; see, *e.g.,* Judge et al., Mol. Ther. 13:494-505 (2006); and Cekaite et al., J. Mol. Biol. 365:90-108 (2007). Additional modifications, such as 2-thiouracil, pseudouracil, 5-methylcytosine, 5-methyluracil, and N6-methyladenosine have also been shown to minimize the immune effects mediated by TLR3, TLR7, and TLR8; see, *e.g.,* Kariko, K. et al., Immunity 23:165-175 (2005).

As is also known in the art, and commercially available, a number of conjugates can be applied to polynucleotides, such as RNAs, for use herein that can enhance their delivery and/or uptake by cells, including for example, cholesterol, tocopherol and folic acid, lipids, peptides, polymers, linkers and aptamers; see, e.g., the review by Winkler, Ther. Deliv. 4:791-809 (2013), and references cited therein.

### Codon-Optimization

In some embodiments, a polynucleotide encoding a site-directed polypeptide is codon-optimized according to methods standard in the art for expression in the cell containing the target DNA of interest. For example, if the intended target nucleic acid is in a human cell, a human codon-optimized polynucleotide encoding Cas9 is contemplated for use for producing the Cas9 polypeptide.

### Complexes of a Genome-targeting Nucleic Acid and a Site-Directed Polypeptide

A genome-targeting nucleic acid interacts with a site-directed polypeptide *(e.g.,* a nucleic acid-guided nuclease such as Cas9), thereby forming a complex. The genome-targeting nucleic acid guides the site-directed polypeptide to a target nucleic acid.

### RNPs

As stated previously, the site-directed polypeptide and genome-targeting nucleic acid may each be administered separately to a cell or a patient. On the other hand, the site-directed polypeptide may be pre-complexed with one or more guide RNAs, or one or more crRNA together with a tracrRNA. The pre-complexed material may then be administered to a cell or a patient. Such pre-complexed material is known as a ribonucleoprotein particle (RNP).

### Nucleic Acids Encoding System Components

In another aspect, the present disclosure provides a nucleic acid comprising a nucleotide sequence encoding a genome-targeting nucleic acid of the disclosure, a site-directed polypeptide of the disclosure, and/or any nucleic acid or proteinaceous molecule necessary to carry out the embodiments of the methods of the disclosure.

In some embodiments, the nucleic acid encoding a genome-targeting nucleic acid of the disclosure, a site-directed polypeptide of the disclosure, and/or any nucleic acid or proteinaceous molecule necessary to carry out the embodiments of the methods of the disclosure comprises a vector (*e.g.,* a recombinant expression vector).

The term "vector" refers to a nucleic acid molecule capable of transporting another nucleic acid to which it has been linked. One type of vector is a "plasmid", which refers to a circular double-stranded DNA loop into which additional nucleic acid segments can be ligated. Another type of vector is a viral vector, wherein additional nucleic acid segments can be ligated into the viral genome. Certain vectors are capable of autonomous replication in a host cell into which they are introduced (*e.g.,* bacterial vectors having a bacterial origin of replication and episomal mammalian vectors). Other vectors (*e.g.,* non-episomal mammalian vectors) are integrated into the genome of a host cell upon introduction into the host cell, and thereby are replicated along with the host genome.

In some embodiments, vectors are capable of directing the expression of nucleic acids to which they are operatively linked. Such vectors are referred to herein as "recombinant expression vectors", or more simply "expression vectors", which serve equivalent functions.

The term "operably linked" means that the nucleotide sequence of interest is linked to regulatory sequence(s) in a manner that allows for expression of the nucleotide sequence. The term "regulatory sequence" is intended to include, for example, promoters, enhancers and other expression control elements (*e.g.,* polyadenylation signals). Such regulatory sequences are well known in the art and are described, for example, in Goeddel; Gene Expression Technology: Methods in Enzymology 185, Academic Press, San Diego, CA (1990). Regulatory sequences include those that direct constitutive expression of a nucleotide sequence in many types of host cells, and those that direct expression of the nucleotide sequence only in certain host cells (*e.g.,* tissue-specific regulatory sequences). It will be appreciated by those skilled in the art that the design of the expression vector can depend on such factors as the choice of the target cell, the level of expression desired, and the like.

Expression vectors contemplated include, but are not limited to, viral vectors based on vaccinia virus, poliovirus, adenovirus, adeno-associated virus, SV40, herpes simplex virus, human immunodeficiency virus, retrovirus (*e.g.,* Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus) and other recombinant vectors. Other vectors contemplated for eukaryotic target cells include, but are not limited to, the vectors pXT1, pSG5, pSVK3, pBPV, pMSG, and pSVLSV40 (Pharmacia). Other vectors may be used so long as they are compatible with the host cell.

In some embodiments, a vector comprises one or more transcription and/or translation control elements. Depending on the host/vector system utilized, any of a number of suitable transcription and translation control elements, including constitutive and inducible promoters, transcription enhancer elements, transcription terminators, etc. may be used in the expression vector. In some embodiments, the vector is a self-inactivating vector that either inactivates the viral sequences or the components of the CRISPR machinery or other elements.

Non-limiting examples of suitable eukaryotic promoters (*i.e.,* promoters functional in a eukaryotic cell) include those from cytomegalovirus (CMV) immediate early, herpes simplex virus (HSV) thymidine kinase, early and late SV40, long terminal repeats (LTRs) from retrovirus, human elongation factor-1 promoter (EF1), a hybrid construct comprising the cytomegalovirus (CMV) enhancer fused to the chicken beta-actin promoter (CAG), murine stem cell virus promoter (MSCV), phosphoglycerate kinase-1 locus promoter (PGK), and mouse metallothionein-I.

For expressing small RNAs, including guide RNAs used in connection with Cas or Cpf1 endonuclease, various promoters such as RNA polymerase III promoters, including for example U6 and H1, can be advantageous. Descriptions of and parameters for enhancing the use of such promoters are known in art, and additional information and approaches are regularly being described; see, *e.g.,* Ma, H. et al., Molecular Therapy - Nucleic Acids 3, e161 (2014) doi:10.1038/mtna.2014.12.

The expression vector may also contain a ribosome binding site for translation initiation and a transcription terminator. The expression vector may also include appropriate sequences for amplifying expression. The expression vector may also include nucleotide sequences encoding non-native tags (*e.g.,* histidine tag, hemagglutinin tag, green fluorescent protein, etc.) that are fused to the site-directed polypeptide, thus resulting in a fusion protein.

In some embodiments, a promoter is an inducible promoter (*e.g.,* a heat shock promoter, tetracycline-regulated promoter, steroid-regulated promoter, metal-regulated promoter, estrogen receptor-regulated promoter, etc.). In some embodiments, a promoter is a constitutive promoter (*e.g.,* CMV promoter, UBC promoter). In some embodiments, the promoter is a spatially restricted and/or temporally restricted promoter (*e.g.,* a tissue specific promoter, a cell type specific promoter, etc.).

In some embodiments, the nucleic acid encoding a genome-targeting nucleic acid of the disclosure and/or a site-directed polypeptide are packaged into or on the surface of delivery vehicles for delivery to cells. Delivery vehicles contemplated include, but are not limited to, nanospheres, liposomes, quantum dots, nanoparticles, polyethylene glycol particles, hydrogels, and micelles. As described in the art, a variety of targeting moieties can be used to enhance the preferential interaction of such vehicles with desired cell types or locations.

Introduction of the complexes, polypeptides, and nucleic acids of the disclosure into cells can occur by viral or bacteriophage infection, transfection, conjugation, protoplast fusion, lipofection, electroporation, nucleofection, calcium phosphate precipitation, polyethyleneimine (PEI)-mediated transfection, DEAE-dextran mediated transfection, liposome-mediated transfection, particle gun technology, calcium phosphate precipitation, direct micro-injection, nanoparticle-mediated nucleic acid delivery, and the like.

### Delivery

Guide RNA polynucleotides (RNA or DNA) and/or endonuclease polynucleotide(s) (RNA or DNA) can be delivered by viral or non-viral delivery vehicles known in the art. Alternatively, endonuclease polypeptide(s) may be delivered by non-viral delivery vehicles known in the art, such as electroporation or lipid nanoparticles. In further alternative embodiments, the DNA endonuclease may be delivered as one or more polypeptides, either alone or pre-complexed with one or more guide RNAs, or one or more crRNA together with a tracrRNA.

Polynucleotides may be delivered by non-viral delivery vehicles including, but not limited to, nanoparticles, liposomes, ribonucleoproteins, positively charged peptides, small molecule RNA-conjugates, aptamer-RNA chimeras, and RNA-fusion protein complexes. Some exemplary non-viral delivery vehicles are described in Peer and Lieberman, Gene Therapy, 18: 1127-1133 (2011) (which focuses on non-viral delivery vehicles for siRNA that are also useful for delivery of other polynucleotides).

Polynucleotides, such as guide RNA, sgRNA, and mRNA encoding an endonuclease, may be delivered to a cell or a patient by a lipid nanoparticle (LNP).

A LNP refers to any particle having a diameter of less than 1000 nm, 500 nm, 250 nm, 200 nm, 150 nm, 100 nm, 75 nm, 50 nm, or 25 nm. Alternatively, a nanoparticle may range in size from 1-1000 nm, 1-500 nm, 1-250 nm, 25-200 nm, 25-100 nm, 35-75 nm, or 25-60 nm.

LNPs may be made from cationic, anionic, or neutral lipids. Neutral lipids, such as the fusogenic phospholipid DOPE or the membrane component cholesterol, may be included in LNPs as 'helper lipids' to enhance transfection activity and nanoparticle stability. Limitations of cationic lipids include low efficacy owing to poor stability and rapid clearance, as well as the generation of inflammatory or antiinflammatory responses.

LNPs may also be comprised of hydrophobic lipids, hydrophilic lipids, or both hydrophobic and hydrophilic lipids.

Any lipid or combination of lipids that are known in the art may be used to produce a LNP. Examples of lipids used to produce LNPs are: DOTMA, DOSPA, DOTAP, DMRIE, DC-cholesterol, DOTAP-cholesterol, GAP-DMORIE-DPyPE, and GL67A-DOPE-DMPE-polyethylene glycol (PEG). Examples of cationic lipids are: 98N12-5, C12-200, DLin-KC2-DMA (KC2), DLin-MC3-DMA (MC3), XTC, MD1, and 7C1. Examples of neutral lipids are: DPSC, DPPC, POPC, DOPE, and SM. Examples of PEG-modified lipids are: PEG-DMG, PEG-CerC14, and PEG-CerC20.

The lipids may be combined in any number of molar ratios to produce a LNP. In addition, the polynucleotide(s) may be combined with lipid(s) in a wide range of molar ratios to produce a LNP.

As stated previously, the site-directed polypeptide and genome-targeting nucleic acid may each be administered separately to a cell or a patient. On the other hand, the site-directed polypeptide may be pre-complexed with one or more guide RNAs, or one or more crRNA together with a tracrRNA. The pre-complexed material may then be administered to a cell or a patient. Such pre-complexed material is known as a ribonucleoprotein particle (RNP).

RNA is capable of forming specific interactions with RNA or DNA. While this property is exploited in many biological processes, it also comes with the risk of promiscuous interactions in a nucleic acid-rich cellular environment. One solution to this problem is the formation of ribonucleoprotein particles (RNPs), in which the RNA is pre-complexed with an endonuclease. Another benefit of the RNP is protection of the RNA from degradation.

The endonuclease in the RNP may be modified or unmodified. Likewise, the gRNA, crRNA, tracrRNA, or sgRNA may be modified or unmodified. Numerous modifications are known in the art and may be used.

The endonuclease and sgRNA can be generally combined in a 1:1 molar ratio. Alternatively, the endonuclease, crRNA and tracrRNA can be generally combined in a 1:1:1 molar ratio. However, a wide range of molar ratios may be used to produce a RNP.

A viral vector may be used for delivery, such as an adeno virus, HPV, HSV, lenti virus, or other viral vector.

A recombinant adeno-associated virus (AAV) vector may be used for delivery. Techniques to produce rAAV particles, in which an AAV genome to be packaged that includes the polynucleotide to be delivered, rep and cap genes, and helper virus functions are provided to a cell are standard in the art. Production of rAAV requires that the following components are present within a single cell (denoted herein as a packaging cell): a rAAV genome, AAV rep and cap genes separate from (*i.e.,* not in) the rAAV genome, and helper virus functions. The AAV rep and cap genes may be from any AAV serotype for which recombinant virus can be derived, and may be from a different AAV serotype than the rAAV genome ITRs, including, but not limited to, AAV serotypes AAV-1, AAV-2, AAV-3, AAV-4, AAV-5, AAV-6, AAV-7, AAV-8, AAV-9, AAV-10, AAV-11, AAV-12, AAV-13 and AAV rh.74. Production of pseudotyped rAAV is disclosed in, for example, international patent application publication number WO 01/83692. See Table 1.

**Table 1**

| **AAV Serotype** | **Genbank Accession No.** |
|---|---|
| AAV-1 | NC_002077.1 |
| AAV-2 | NC_001401.2 |
| AAV-3 | NC_001729.1 |
| AAV-3B | AF028705.1 |
| AAV-4 | NC_001829.1 |
| AAV-5 | NC_006152.1 |
| AAV-6 | AF028704.1 |
| AAV-7 | NC_006260.1 |
| AAV-8 | NC_006261.1 |
| AAV-9 | AX753250.1 |
| AAV-10 | AY631965.1 |
| AAV-11 | AY631966.1 |
| AAV-12 | DQ813647.1 |
| AAV-13 | EU285562.1 |

A method of generating a packaging cell involves creating a cell line that stably expresses all of the necessary components for AAV particle production. For example, a plasmid (or multiple plasmids) comprising a rAAV genome lacking AAV rep and cap genes, AAV rep and cap genes separate from the rAAV genome, and a selectable marker, such as a neomycin resistance gene, are integrated into the genome of a cell. AAV genomes have been introduced into bacterial plasmids by procedures such as GC tailing (Samulski et al., 1982, Proc. Natl. Acad. S6. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Biol. Chem., 259:4661-4666). The packaging cell line is then infected with a helper virus, such as adenovirus. The advantages of this method are that the cells are selectable and are suitable for large-scale production of rAAV. Other examples of suitable methods employ adenovirus or baculovirus, rather than plasmids, to introduce rAAV genomes and/or rep and cap genes into packaging cells.

General principles of rAAV production are reviewed in, for example, Carter, 1992, Current Opinions in Biotechnology, 1533-539; and Muzyczka, 1992, Curr. Topics in Microbial. and Immunol., 158:97-129). Various approaches are described in Ratschin et al., Mol. Cell. Biol. 4:2072 (1984); Hermonat et al., Proc. Natl. Acad. Sci. USA, 81:6466 (1984); Tratschin et al., Mo1. Cell. Biol. 5:3251 (1985); McLaughlin et al., J. Virol., 62:1963 (1988); and Lebkowski et al., 1988 Mol. Cell. Biol., 7:349 (1988). Samulski et al. (1989, J. Virol., 63:3822-3828); U.S. Patent No. 5,173,414; WO 95/13365 and corresponding U.S. Patent No. 5,658.776 ; WO 95/13392; WO 96/17947; PCT/US98/18600; WO 97/09441 (PCT/US96/14423); WO 97/08298 (PCT/US96/13872); WO 97/21825 (PCT/US96/20777); WO 97/06243 (PCT/FR96/01064); WO 99/11764; Perrin et al. (1995) Vaccine 13:1244-1250; Paul et al. (1993) Human Gene Therapy 4:609-615; Clark et al. (1996) Gene Therapy 3:1124-1132; U.S. Patent. No. 5,786,211; U.S. Patent No. 5,871,982; and U.S. Patent. No. 6,258,595.

AAV vector serotypes can be matched to target cell types. For example, the following exemplary cell types may be transduced by the indicated AAV serotypes among others. See Table 2.

**Table 2**

| **Tissue/Cell Type** | **Serotype** |
|---|---|
| Liver | AAV3, AAV5, AAV8, AAV9 |
| Skeletal muscle | AAV1, AAV7, AAV6, AAV8, AAV9 |
| Central nervous system | AAV5, AAV1, AAV4 |
| RPE | AAV5, AAV4 |
| Photoreceptor cells | AAV5 |
| Lung | AAV9 |
| Heart | AAV8 |
| Pancreas | AAV8 |
| Kidney | AAV2, AAV8 |

In addition to adeno-associated viral vectors, other viral vectors may be used in the practice of the invention. Such viral vectors include, but are not limited to, lentivirus, alphavirus, enterovirus, pestivirus, baculovirus, herpesvirus, Epstein Barr virus, papovavirusr, poxvirus, vaccinia virus, and herpes simplex virus.

In some embodiments, Cas9 mRNA, sgRNA targeting one or two loci in C9ORF72 genes, and donor DNA are each separately formulated into lipid nanoparticles, or are all co-formulated into one lipid nanoparticle, or co-formulated into two or more lipid nanoparticles.

In some embodiments, Cas9 mRNA is formulated in a lipid nanoparticle, while sgRNA and donor DNA are delivered in an AAV vector. In some embodiments, Cas9 mRNA and sgRNA are co-formulated in a lipid nanoparticle, while donor DNA is delivered in an AAV vector.

Options are available to deliver the Cas9 nuclease as a DNA plasmid, as mRNA or as a protein. The guide RNA can be expressed from the same DNA, or can also be delivered as an RNA. The RNA can be chemically modified to alter or improve its half-life, or decrease the likelihood or degree of immune response. The endonuclease protein can be complexed with the gRNA prior to delivery. Viral vectors allow efficient delivery; split versions of Cas9 and smaller orthologs of Cas9 can be packaged in AAV, as can donors for HDR. A range of non-viral delivery methods also exist that can deliver each of these components, or non-viral and viral methods can be employed in tandem. For example, nano-particles can be used to deliver the protein and guide RNA, while AAV can be used to deliver a donor DNA.

### Genetically Modified Cells

The term "genetically modified cell" refers to a cell that comprises at least one genetic modification introduced by genome editing (*e.g.,* using the CRISPR/Cas9/Cpf1 system). In some *ex vivo* embodiments herein, the genetically modified cell is a genetically modified progenitor cell. In some *in vivo* embodiments herein, the genetically modified cell is a genetically modified neural cell. A genetically modified cell comprising an exogenous genome-targeting nucleic acid and/or an exogenous nucleic acid encoding a genome-targeting nucleic acid is contemplated herein.

The term "control treated population" describes a population of cells that has been treated with identical media, viral induction, nucleic acid sequences, temperature, confluency, flask size, pH, etc., with the exception of the addition of the genome editing components. Any method known in the art can be used to measure length of the hexanucleotide repeat in the C9ORF72 gene, for example Northern Blot analysis of the or quantifying C9ORF72 mRNA.

The term "isolated cell" refers to a cell that has been removed from an organism in which it was originally found, or a descendant of such a cell. Optionally, the cell has been cultured *in vitro, e.g.,* under defined conditions or in the presence of other cells. Optionally, the cell is later introduced into a second organism or reintroduced into the organism from which it (or the cell from which it is descended) was isolated.

The term "isolated population" with respect to an isolated population of cells refers to a population of cells that has been removed and separated from a mixed or heterogeneous population of cells. In some embodiments, an isolated population is a substantially pure population of cells, as compared to the heterogeneous population from which the cells were isolated or enriched. In some embodiments, the isolated population is an isolated population of human progenitor cells, e.g., a substantially pure population of human progenitor cells, as compared to a heterogeneous population of cells comprising human progenitor cells and cells from which the human progenitor cells were derived.

The term "substantially enhanced," with respect to a particular cell population, refers to a population of cells in which the occurrence of a particular type of cell is increased relative to pre-existing or reference levels, by at least 2-fold, at least 3-, at least 4-, at least 5-, at least 6-, at least 7-, at least 8-, at least 9, at least 10-, at least 20-, at least 50-, at least 100-, at least 400-, at least 1000-, at least 5000-, at least 20000-, at least 100000- or more fold depending, e.g., on the desired levels of such cells for ameliorating ALS and/or FTLD.

The term "substantially enriched" with respect to a particular cell population, refers to a population of cells that is at least about 10%, about 20%, about 30%, about 40%, about 50%, about 60%, about 70% or more with respect to the cells making up a total cell population.

The terms "substantially enriched" or "substantially pure" with respect to a particular cell population, refers to a population of cells that is at least about 75%, at least about 85%, at least about 90%, or at least about 95% pure, with respect to the cells making up a total cell population. That is, the terms "substantially pure" or "essentially purified," with regard to a population of progenitor cells, refers to a population of cells that contain fewer than about 20%, about 15%, about 10%, about 9%, about 8%, about 7%, about 6%, about 5%, about 4%, about 3%, about 2%, about 1%, or less than 1%, of cells that are not progenitor cells as defined by the terms herein.

### Differentiation of genome edited iPSCs into hematopoietic progenitor cells, neural progenitor cells, or neural cells

Another step of the *ex vivo* methods of the disclosure involves differentiating the genome edited iPSCs into hematopoietic progenitor cells, neural progenitor cells, or neural cells. The differentiating step may be performed according to any method known in the art. For example, iPSCs are differentiated into neurons using a combination of small molecules or by delivery of master transcription factors such as, but not limited to, Brn2, ASCL1, MYT1L, Sox2 and Foxa2,*Hes1*, *Hes3, Klf4, Myc, Notchl,* (*NICD*), *PLAGL1,* and *Rfx4.* iPSCs can be differentiated into neurons, expressing βIII-tubulin, tyrosine hydroxylase, AADC, DAT, ChAT, LMX1B, and MAP2. The presence of catecholamine-associated enzymes may indicate that iPSCs, like hESCs, may be differentiable into dopaminergic neurons.

### Differentiation of genome edited mesenchymal stem cells into hematopoietic progenitor cells, neural progenitor cells, or neural cells

Another step of the *ex vivo* methods of the disclosure involves differentiating the genome edited mesenchymal stem cells into neural cells. The differentiating step may be performed according to any method known in the art. For example, mesenchymal stem cells are differentiated into neurons using a combination of small molecules or by delivery of master transcription factors such as, but not limited to, Brn2, ASCL1, MYT1L, Sox2 and Foxa2,Hes1, Hes3, Klf4, Myc, Notch1, (NICD), PLAGL1, and Rfx4. Mesenchymal stem cells can be differentiated into neurons, expressing βIII-tubulin, tyrosine hydroxylase, AADC, DAT, ChAT, LMX1B, and MAP2. The presence of catecholamine-associated enzymes may indicate that mesenchymal stem cells, like hESCs, may be differentiable into dopaminergic neurons.

### Implanting cells into patients

Another step of the *ex vivo* methods of the disclosure involves implanting the cells into patients. This implanting step may be accomplished using any method of implantation known in the art. For example, the genetically modified cells may be injected directly in the patient's central nervous system or otherwise administered to the patient. The genetically modified cells may be purified *ex vivo* using a selected marker.

### Pharmaceutically Acceptable Carriers

The *ex vivo* methods of administering progenitor cells to a subject contemplated herein involve the use of therapeutic compositions comprising progenitor cells.

Therapeutic compositions contain a physiologically tolerable carrier together with the cell composition, and optionally at least one additional bioactive agent as described herein, dissolved or dispersed therein as an active ingredient. In some embodiments, the therapeutic composition is not substantially immunogenic when administered to a mammal or human patient for therapeutic purposes, unless so desired.

In general, the progenitor cells described herein are administered as a suspension with a pharmaceutically acceptable carrier. One of skill in the art will recognize that a pharmaceutically acceptable carrier to be used in a cell composition will not include buffers, compounds, cryopreservation agents, preservatives, or other agents in amounts that substantially interfere with the viability of the cells to be delivered to the subject. A formulation comprising cells can include *e.g.,* osmotic buffers that permit cell membrane integrity to be maintained, and optionally, nutrients to maintain cell viability or enhance engraftment upon administration. Such formulations and suspensions are known to those of skill in the art and/or can be adapted for use with the progenitor cells, as described herein, using routine experimentation.

A cell composition can also be emulsified or presented as a liposome composition, provided that the emulsification procedure does not adversely affect cell viability. The cells and any other active ingredient can be mixed with excipients that are pharmaceutically acceptable and compatible with the active ingredient, and in amounts suitable for use in the therapeutic methods described herein.

Additional agents included in a cell composition can include pharmaceutically acceptable salts of the components therein. Pharmaceutically acceptable salts include the acid addition salts (formed with the free amino groups of the polypeptide) that are formed with inorganic acids, such as, for example, hydrochloric or phosphoric acids, or such organic acids as acetic, tartaric, mandelic and the like. Salts formed with the free carboxyl groups can also be derived from inorganic bases, such as, for example, sodium, potassium, ammonium, calcium or ferric hydroxides, and such organic bases as isopropylamine, trimethylamine, 2-ethylamino ethanol, histidine, procaine and the like.

Physiologically tolerable carriers are well known in the art. Exemplary liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes. Liquid compositions can also contain liquid phases in addition to and to the exclusion of water. Exemplary of such additional liquid phases are glycerin, vegetable oils such as cottonseed oil, and water-oil emulsions. The amount of an active compound used in the cell compositions that is effective in the treatment of a particular disorder or condition will depend on the nature of the disorder or condition, and can be determined by standard clinical techniques.

### Administration & Efficacy

The terms "administering," "introducing" and "transplanting" are used interchangeably in the context of the placement of cells, e.g., progenitor cells, into a subject, by a method or route that results in at least partial localization of the introduced cells at a desired site, such as a site of injury or repair, such that a desired effect(s) is produced. The cells e.g., progenitor cells, or their differentiated progeny can be administered by any appropriate route that results in delivery to a desired location in the subject where at least a portion of the implanted cells or components of the cells remain viable. The period of viability of the cells after administration to a subject can be as short as a few hours, e.g., twenty-four hours, to a few days, to as long as several years, or even the life time of the patient, *i.e.,* long-term engraftment. For example, in some embodiments described herein, an effective amount of myogenic progenitor cells is administered via a systemic route of administration, such as an intraperitoneal or intravenous route.

The terms "individual", "subject," "host" and "patient" are used interchangeably herein and refer to any subject for whom diagnosis, treatment or therapy is desired. In some embodiments, the subject is a mammal. In some embodiments, the subject is a human being.

When provided prophylactically, progenitor cells described herein can be administered to a subject in advance of any symptom of ALS and/or FTLD, e.g., prior to the development of dementia, difficulty walking, weakness in the legs, hand weakness, clumsiness, slurring of speech, trouble swallowing, muscle cramps, twitching in the arms or shoulders or tongue, difficulty holding the head up or keeping good posture. Accordingly, the prophylactic administration of a neural progenitor cell population serves to prevent ALS and/or FTLD.

When provided therapeutically, neural progenitor cells are provided at (or after) the onset of a symptom or indication of ALS and/or FTLD, e.g., upon the onset of disease.

In some embodiments described herein, the neural progenitor cell population being administered according to the methods described herein comprises allogeneic neural progenitor cells obtained from one or more donors. "Allogeneic" refers to a neural progenitor cell or biological samples comprising neural progenitor cells obtained from one or more different donors of the same species, where the genes at one or more loci are not identical. For example, a neural progenitor cell population being administered to a subject can be derived from one more unrelated donor subjects, or from one or more non-identical siblings. In some embodiments, syngeneic neural progenitor cell populations can be used, such as those obtained from genetically identical animals, or from identical twins. In other embodiments, the neural progenitor cells are autologous cells; that is, the neural progenitor cells are obtained or isolated from a subject and administered to the same subject, *i.e.,* the donor and recipient are the same.

In one embodiment, the term "effective amount" refers to the amount of a population of progenitor cells or their progeny needed to prevent or alleviate at least one or more signs or symptoms of ALS and/or FTLD, and relates to a sufficient amount of a composition to provide the desired effect, e.g., to treat a subject having ALS and/or FTLD. The term "therapeutically effective amount" therefore refers to an amount of progenitor cells or a composition comprising progenitor cells that is sufficient to promote a particular effect when administered to a typical subject, such as one who has or is at risk for ALS and/or FTLD. An effective amount would also include an amount sufficient to prevent or delay the development of a symptom of the disease, alter the course of a symptom of the disease (for example but not limited to, slow the progression of a symptom of the disease), or reverse a symptom of the disease. It is understood that for any given case, an appropriate "effective amount" can be determined by one of ordinary skill in the art using routine experimentation.

For use in the various embodiments described herein, an effective amount of progenitor cells comprises at least 10² progenitor cells, at least 5 X 10² progenitor cells, at least 10³ progenitor cells, at least 5 X 10³ progenitor cells, at least 10⁴ progenitor cells, at least 5 X 10⁴ progenitor cells, at least 10⁵ progenitor cells, at least 2 X 10⁵ progenitor cells, at least 3 X 10⁵ progenitor cells, at least 4 X 10⁵ progenitor cells, at least 5 X 10⁵ progenitor cells, at least 6 X 10⁵ progenitor cells, at least 7 X 10⁵ progenitor cells, at least 8 X 10⁵ progenitor cells, at least 9 X 10⁵ progenitor cells, at least 1 X 10⁶ progenitor cells, at least 2 X 10⁶ progenitor cells, at least 3 X 10⁶ progenitor cells, at least 4 X 10⁶ progenitor cells, at least 5 X 10⁶ progenitor cells, at least 6 X 10⁶ progenitor cells, at least 7 X 10⁶ progenitor cells, at least 8 X 10⁶ progenitor cells, at least 9 X 10⁶ progenitor cells, or multiples thereof. The progenitor cells are derived from one or more donors, or are obtained from an autologous source. In some embodiments described herein, the progenitor cells are expanded in culture prior to administration to a subject in need thereof.

Reduction of the expanded hexanucleotide repeats in the C9ORF72 gene in cells of patients having ALS and/or FTLD can be beneficial for ameliorating one or more symptoms of the disease, for increasing long-term survival, and/or for reducing side effects associated with other treatments. Upon administration of such cells to human patients, the presence of neural progenitors that have wild-type or similar levels of the hexanucleotide repeat is beneficial. In some embodiments, effective treatment of a subject gives rise to at least about 3%, 5% or 7% wild-type or similar levels relative to total hexanucleotide repeat in the treated subject. In some embodiments, wild-type or similar levels will be at least about 10% of total hexanucleotide repeat. In some embodiments, wild-type or similar levels will be at least about 20% to 30% of total hexanucleotide repeat. Similarly, the introduction of even relatively limited subpopulations of cells having wild-type levels of hexanucleotide repeat can be beneficial in various patients because in some situations normalized cells will have a selective advantage relative to diseased cells. However, even modest levels of neural progenitors with wild-type or similar levels of hexanucleotide repeat can be beneficial for ameliorating one or more aspects of ALS and/or FTLD in patients. In some embodiments, about 10%, about 20%, about 30%, about 40%, about 50%, about 60%, about 70%, about 80%, about 90% or more of the neural progenitors in patients to whom such cells are administered are producing wild-type or similar levels of hexanucleotide repeat.

"Administered" refers to the delivery of a progenitor cell composition into a subject by a method or route that results in at least partial localization of the cell composition at a desired site. A cell composition can be administered by any appropriate route that results in effective treatment in the subject, *i.e.* administration results in delivery to a desired location in the subject where at least a portion of the composition delivered, *i.e.* at least 1 x 10⁴ cells are delivered to the desired site for a period of time. Modes of administration include injection, infusion, instillation, or ingestion. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In some embodiments, the route is intravenous. For the delivery of cells, administration by injection or infusion is generally preferred.

In one embodiment, the cells are administered systemically. The phrases "systemic administration," "administered systemically", "peripheral administration" and "administered peripherally" refer to the administration of a population of progenitor cells other than directly into a target site, tissue, or organ, such that it enters, instead, the subject's circulatory system and, thus, is subject to metabolism and other like processes.

The efficacy of a treatment comprising a composition for the treatment of ALS and/or FTLD can be determined by the skilled clinician. However, a treatment is considered "effective treatment," if any one or all of the signs or symptoms of, as but one example, levels of hexanucleotide repeat are altered in a beneficial manner (e.g., increased by at least 10%), or other clinically accepted symptoms or markers of disease are improved or ameliorated. Efficacy can also be measured by failure of an individual to worsen as assessed by hospitalization or need for medical interventions (e.g., chronic obstructive pulmonary disease, or progression of the disease is halted or at least slowed). Methods of measuring these indicators are known to those of skill in the art and/or described herein. Treatment includes any treatment of a disease in an individual or an animal (some non-limiting examples include a human, or a mammal) and includes: (1) inhibiting the disease, e.g., arresting, or slowing the progression of symptoms; or (2) relieving the disease, e.g., causing regression of symptoms; and (3) preventing or reducing the likelihood of the development of symptoms.

The treatment according to the present invention ameliorates one or more symptoms associated with ALS and/or FTLD by reducing the amount of hexanucleotide repeat in the individual. Early signs typically associated with ALS and/or FTLD include for example, dementia, difficulty walking, weakness in the legs, hand weakness, clumsiness, slurring of speech, trouble swallowing, muscle cramps, twitching in the arms or shoulders or tongue, difficulty holding the head up or keeping good posture.

### Kits

The present disclosure provides kits for carrying out the methods of the invention. A kit can include one or more of a genome-targeting nucleic acid, a polynucleotide encoding a genome-targeting nucleic acid, a site-directed polypeptide, a polynucleotide encoding a site-directed polypeptide, and/or any nucleic acid or proteinaceous molecule necessary to carry out the embodiments of the methods of the invention, or any combination thereof.

In some embodiments, a kit comprises: (1) a vector comprising a nucleotide sequence encoding a genome-targeting nucleic acid, and (2) a vector comprising a nucleotide sequence encoding the site-directed polypeptide or the site-directed polypeptide and (3) a reagent for reconstitution and/or dilution of the vector(s) and or polypeptide.

In some embodiments, a kit comprises: (1) a vector comprising (i) a nucleotide sequence encoding a genome-targeting nucleic acid, and (ii) a nucleotide sequence encoding the site-directed polypeptide and (2) a reagent for reconstitution and/or dilution of the vector.

In some embodiments of any of the above kits, the kit comprises a single-molecule guide genome-targeting nucleic acid. In some embodiments of any of the above kits, the kit comprises a double-molecule genome-targeting nucleic acid. In some embodiments of any of the above kits, the kit comprises two or more double-molecule guides or single-molecule guides. In some embodiments, the kits comprise a vector that encodes the nucleic acid targeting nucleic acid.

In some embodiments of any of the above kits, the kit can further comprise a polynucleotide to be inserted to effect the desired genetic modification.

Components of a kit may be in separate containers, or combined in a single container.

In some embodiments, a kit described above further comprises one or more additional reagents, where such additional reagents are selected from a buffer, a buffer for introducing a polypeptide or polynucleotide into a cell, a wash buffer, a control reagent, a control vector, a control RNA polynucleotide, a reagent for *in vitro* production of the polypeptide from DNA, adaptors for sequencing and the like. A buffer can be a stabilization buffer, a reconstituting buffer, a diluting buffer, or the like. In some embodiments, a kit can also include one or more components that may be used to facilitate or enhance the on-target binding or the cleavage of DNA by the endonuclease, or improve the specificity of targeting.

In addition to the above-mentioned components, a kit can further include instructions for using the components of the kit to practice the methods. The instructions for practicing the methods are generally recorded on a suitable recording medium. For example, the instructions may be printed on a substrate, such as paper or plastic, etc. The instructions may be present in the kits as a package insert, in the labeling of the container of the kit or components thereof (*i.e.,* associated with the packaging or subpackaging), etc. The instructions can be present as an electronic storage data file present on a suitable computer readable storage medium, e.g. CD-ROM, diskette, flash drive, etc. In some instances, the actual instructions are not present in the kit, but means for obtaining the instructions from a remote source (e.g. via the Internet), can be provided. An example of this embodiment is a kit that includes a web address where the instructions can be viewed and/or from which the instructions can be downloaded. As with the instructions, this means for obtaining the instructions can be recorded on a suitable substrate.

### Guide RNA Formulation

Guide RNAs of the invention are formulated with pharmaceutically acceptable excipients such as carriers, solvents, stabilizers, adjuvants, diluents, etc., depending upon the particular mode of administration and dosage form. Guide RNA compositions are generally formulated to achieve a physiologically compatible pH, and range from a pH of about 3 to a pH of about 11, about pH 3 to about pH 7, depending on the formulation and route of administration. In alternative embodiments, the pH is adjusted to a range from about pH 5.0 to about pH 8. In some embodiments, the compositions comprise a therapeutically effective amount of at least one compound as described herein, together with one or more pharmaceutically acceptable excipients. Optionally, the compositions comprise a combination of the compounds described herein, or may include a second active ingredient useful in the treatment or prevention of bacterial growth (for example and without limitation, anti-bacterial or anti-microbial agents), or may include a combination of reagents of the invention.

Suitable excipients include, for example, carrier molecules that include large, slowly metabolized macromolecules such as proteins, polysaccharides, polylactic acids, polyglycolic acids, polymeric amino acids, amino acid copolymers, and inactive virus particles. Other exemplary excipients include antioxidants (for example and without limitation, ascorbic acid), chelating agents (for example and without limitation, EDTA), carbohydrates (for example and without limitation, dextrin, hydroxyalkylcellulose, and hydroxyalkylmethylcellulose), stearic acid, liquids (for example and without limitation, oils, water, saline, glycerol and ethanol), wetting or emulsifying agents, pH buffering substances, and the like.

### Other Possible Therapeutic Approaches

Gene editing can be conducted using nucleases engineered to target specific sequences. To date there are four major types of nucleases: meganucleases and their derivatives, zinc finger nucleases (ZFNs), transcription activator like effector nucleases (TALENs), and CRISPR-Cas9 nuclease systems. The nuclease platforms vary in difficulty of design, targeting density and mode of action, particularly as the specificity of ZFNs and TALENs is through protein-DNA interactions, while RNA-DNA interactions primarily guide Cas9. Cas9 cleavage also requires an adjacent motif, the PAM, which differs between different CRISPR systems. Cas9 from *Streptococcus pyogenes* cleaves using a NRG PAM, CRISPR from *Neisseria meningitidis* can cleave at sites with PAMs including NNNNGATT, NNNNNGTTT and NNNNGCTT. A number of other Cas9 orthologs target protospacer adjacent to alternative PAMs.

It is preferable to use CRISPR endonucleases, such as Cas9, in the methods of the disclosure. However, the teachings described herein, such as therapeutic target sites, could be applied to other forms of endonucleases, such as ZFNs, TALENs, HEs, or MegaTALs, or using combinations of nucleases. However, in order to apply the teachings of the present invention to such endonucleases, one would need to, among other things, engineer proteins directed to the specific target sites.

Additional binding domains can be fused to the Cas9 protein to increase specificity. The target sites of these constructs would map to the identified gRNA specified site, but would require additional binding motifs, such as for a zinc finger domain. In the case of Mega-TAL, a meganuclease can be fused to a TALE DNA-binding domain. The meganuclease domain can increase specificity and provide the cleavage. Similarly, inactivated or dead Cas9 (dCas9) can be fused to a cleavage domain and require the sgRNA/Cas9 target site and adjacent binding site for the fused DNA-binding domain. This likely would require some protein engineering of the dCas9, in addition to the catalytic inactivation, to decrease binding without the additional binding site.

### Zinc Finger Nucleases

Zinc finger nucleases (ZFNs) are modular proteins comprised of an engineered zinc finger DNA binding domain linked to the catalytic domain of the type II endonuclease Fokl. Because Fokl functions only as a dimer, a pair of ZFNs must be engineered to bind to cognate target "half-site" sequences on opposite DNA strands and with precise spacing between them to enable the catalytically active Fokl dimer to form. Upon dimerization of the Fokl domain, which itself has no sequence specificity per se, a DNA double-strand break is generated between the ZFN half-sites as the initiating step in genome editing.

The DNA binding domain of each ZFN is typically comprised of 3-6 zinc fingers of the abundant Cys2-His2 architecture, with each finger primarily recognizing a triplet of nucleotides on one strand of the target DNA sequence, although cross-strand interaction with a fourth nucleotide also can be important. Alteration of the amino acids of a finger in positions that make key contacts with the DNA alters the sequence specificity of a given finger. Thus, a four-finger zinc finger protein will selectively recognize a 12 bp target sequence, where the target sequence is a composite of the triplet preferences contributed by each finger, although triplet preference can be influenced to varying degrees by neighboring fingers. An important aspect of ZFNs is that they can be readily re-targeted to almost any genomic address simply by modifying individual fingers, although considerable expertise is required to do this well. In most applications of ZFNs, proteins of 4-6 fingers are used, recognizing 12-18 bp respectively. Hence, a pair of ZFNs will typically recognize a combined target sequence of 24-36 bp, not including the 5-7 bp spacer between half-sites. The binding sites can be separated further with larger spacers, including 15-17 bp. A target sequence of this length is likely to be unique in the human genome, assuming repetitive sequences or gene homologs are excluded during the design process. Nevertheless, the ZFN protein-DNA interactions are not absolute in their specificity so off-target binding and cleavage events do occur, either as a heterodimer between the two ZFNs, or as a homodimer of one or the other of the ZFNs. The latter possibility has been effectively eliminated by engineering the dimerization interface of the Fokl domain to create "plus" and "minus" variants, also known as obligate heterodimer variants, which can only dimerize with each other, and not with themselves. Forcing the obligate heterodimer prevents formation of the homodimer. This has greatly enhanced specificity of ZFNs, as well as any other nuclease that adopts these Fokl variants.

A variety of ZFN-based systems have been described in the art, modifications thereof are regularly reported, and numerous references describe rules and parameters that are used to guide the design of ZFNs; see, *e.g.,* Segal et al., Proc Natl Acad Sci USA 96(6):2758-63 (1999); Dreier B et al., J Mol Biol. 303(4):489-502 (2000); Liu Q et al., J Biol Chem. 277(6):3850-6 (2002); Dreier et al., J Biol Chem 280(42):35588-97 (2005); and Dreier et al., J Biol Chem. 276(31):29466-78 (2001).

### Transcription Activator-Like Effector Nucleases (TALENs)

TALENs represent another format of modular nucleases whereby, as with ZFNs, an engineered DNA binding domain is linked to the Fokl nuclease domain, and a pair of TALENs operate in tandem to achieve targeted DNA cleavage. The major difference from ZFNs is the nature of the DNA binding domain and the associated target DNA sequence recognition properties. The TALEN DNA binding domain derives from TALE proteins, which were originally described in the plant bacterial pathogen *Xanthomonas sp.* TALEs are comprised of tandem arrays of 33-35 amino acid repeats, with each repeat recognizing a single basepair in the target DNA sequence that is typically up to 20 bp in length, giving a total target sequence length of up to 40 bp. Nucleotide specificity of each repeat is determined by the repeat variable diresidue (RVD), which includes just two amino acids at positions 12 and 13. The bases guanine, adenine, cytosine and thymine are predominantly recognized by the four RVDs: Asn-Asn, Asn-Ile, His-Asp and Asn-Gly, respectively. This constitutes a much simpler recognition code than for zinc fingers, and thus represents an advantage over the latter for nuclease design. Nevertheless, as with ZFNs, the protein-DNA interactions of TALENs are not absolute in their specificity, and TALENs have also benefitted from the use of obligate heterodimer variants of the Fokl domain to reduce off-target activity.

Additional variants of the Fokl domain have been created that are deactivated in their catalytic function. If one half of either a TALEN or a ZFN pair contains an inactive Fokl domain, then only single-strand DNA cleavage (nicking) will occur at the target site, rather than a DSB. The outcome is comparable to the use of CRISPR/Cas9/Cpf1 "nickase" mutants in which one of the Cas9 cleavage domains has been deactivated. DNA nicks can be used to drive genome editing by HDR, but at lower efficiency than with a DSB. The main benefit is that off-target nicks are quickly and accurately repaired, unlike the DSB, which is prone to NHEJ-mediated mis-repair.

A variety of TALEN-based systems have been described in the art, and modifications thereof are regularly reported; see, *e.g.,* Boch, Science 326(5959):1509-12 (2009); Mak et al., Science 335(6069):716-9 (2012); and Moscou et al., Science 326(5959):1501 (2009). The use of TALENs based on the "Golden Gate" platform has been described by multiple groups; see, *e.g.,* Cermak et al., Nucleic Acids Res. 39(12):e82 (2011); Li et al., Nucleic Acids Res. 39(14):6315-25(2011); Weber et al., PLoS One. 6(2):e16765 (2011); Wang et al., J Genet Genomics 41(6):339-47, Epub 2014 May 17 (2014); and Cermak T et al., Methods Mol Biol. 1239:133-59 (2015).

### Homing Endonucleases

Homing endonucleases (HEs) are sequence-specific endonucleases that have long recognition sequences (14-44 base pairs) and cleave DNA with high specificity - often at sites unique in the genome. There are at least six known families of HEs as classified by their structure, including LAGLIDADG (SEQ ID NO: 18809), GIY-YIG, His-Cis box, H-N-H, PD-(D/E)xK, and Vsr-like that are derived from a broad range of hosts, including eukarya, protists, bacteria, archaea, cyanobacteria and phage. As with ZFNs and TALENs, HEs can be used to create a DSB at a target locus as the initial step in genome editing. In addition, some natural and engineered HEs cut only a single strand of DNA, thereby functioning as site-specific nickases. The large target sequence of HEs and the specificity that they offer have made them attractive candidates to create site-specific DSBs.

A variety of HE-based systems have been described in the art, and modifications thereof are regularly reported; see, *e.g.,* the reviews by Steentoft et al., Glycobiology 24(8):663-80 (2014); Belfort and Bonocora, Methods Mol Biol. 1123:1-26 (2014); Hafez and Hausner, Genome 55(8):553-69 (2012); and references cited therein.

### MegaTAL / Tev-mTALEN / MegaTev

As further examples of hybrid nucleases, the MegaTAL platform and Tev-mTALEN platform use a fusion of TALE DNA binding domains and catalytically active HEs, taking advantage of both the tunable DNA binding and specificity of the TALE, as well as the cleavage sequence specificity of the HE; see, *e.g.,* Boissel et al., NAR 42: 2591-2601 (2014); Kleinstiver et al., G3 4:1155-65 (2014); and Boissel and Scharenberg, Methods Mol. Biol. 1239: 171-96 (2015).

In a further variation, the MegaTev architecture is the fusion of a meganuclease (Mega) with the nuclease domain derived from the GIY-YIG homing endonuclease I-Tevl (Tev). The two active sites are positioned -30 bp apart on a DNA substrate and generate two DSBs with non-compatible cohesive ends; see, *e.g.,* Wolfs et al., NAR 42, 8816-29 (2014). It is anticipated that other combinations of existing nuclease-based approaches will evolve and be useful in achieving the targeted genome modifications described herein.

### dCas9-Fokl or dCpf1-Fok1 and Other Nucleases

Combining the structural and functional properties of the nuclease platforms described above offers a further approach to genome editing that can potentially overcome some of the inherent deficiencies. As an example, the CRISPR genome editing system typically uses a single Cas9 endonuclease to create a DSB. The specificity of targeting is driven by a 20 or 24 nucleotide sequence in the guide RNA that undergoes Watson-Crick base-pairing with the target DNA (plus an additional 2 bases in the adjacent NAG or NGG PAM sequence in the case of Cas9 from S. *pyogenes*). Such a sequence is long enough to be unique in the human genome, however, the specificity of the RNA/DNA interaction is not absolute, with significant promiscuity sometimes tolerated, particularly in the 5' half of the target sequence, effectively reducing the number of bases that drive specificity. One solution to this has been to completely deactivate the Cas9 or Cpf1 catalytic function - retaining only the RNA-guided DNA binding function - and instead fusing a Fokl domain to the deactivated Cas9; see, *e.g.,* Tsai et al., Nature Biotech 32: 569-76 (2014); and Guilinger et al., Nature Biotech. 32:577-82 (2014). Because Fokl must dimerize to become catalytically active, two guide RNAs are required to tether two Fokl fusions in close proximity to form the dimer and cleave DNA. This essentially doubles the number of bases in the combined target sites, thereby increasing the stringency of targeting by CRISPR-based systems.

As further example, fusion of the TALE DNA binding domain to a catalytically active HE, such as l-Tevl, takes advantage of both the tunable DNA binding and specificity of the TALE, as well as the cleavage sequence specificity of l-Tevl, with the expectation that off-target cleavage may be further reduced.

### Methods and Compositions

Accordingly, the present disclosure relates in particular to the following methods and compositions: In a first method, Method 1, the present disclosure provides a method for editing the C9ORF72 gene in a human cell by genome editing, the method comprising introducing into the cell one or more deoxyribonucleic acid (DNA) endonucleases to effect one or more single-strand breaks (SSBs) or double-strand breaks (DSBs) within or near the C9ORF72 gene or other DNA sequences that encode regulatory elements of the C9ORF72 gene that results in permanent deletion, insertion, or correction of the expanded hexanucleotide repeat within or near the C9ORF72 gene and results in restoration of C9orf72 protein activity.

In another method, Method 2, the present disclosure provides a method for inserting the C9ORF72 gene in a human cell by genome editing, the method comprising the step of: introducing into the human cell one or more deoxyribonucleic acid (DNA) endonucleases to effect one or more single-strand breaks (SSBs) or double-strand breaks (DSBs) within or near a safe harbor locus that results in a permanent insertion of the C9ORF72 gene or minigene, and results in restoration of the C9orf72 protein activity.

In another method, Method 3, present disclosure provides an *ex vivo* method for treating a patient with amyotrophic lateral sclerosis (ALS) or frontotemporal lobular dementia (FTLD) comprising the steps of: i) creating a patient specific induced pluripotent stem cell (iPSC); ii) editing within or near the C9ORF72 gene of the iPSC or other DNA sequences that encode regulatory elements of the C9ORF72 gene of the iPSC, or editing within or near a safe harbor locus of the iPSC; iii) differentiating the genome edited iPSC into a hematopoietic progenitor cell, a neural progenitor cell, or neural cell; and iv) implanting the hematopoietic progenitor cell, neural progenitor cell, or neural cell into the patient.

In another method, Method 4, the present disclosure provides the method of Method 3, wherein the creating step comprises: a) isolating a somatic cell from the patient; and b) introducing a set of pluripotency-associated genes into the somatic cell to induce the cell to become a pluripotent stem cell.

In another method, Method 5, the present disclosure provides the method of Method 4, wherein the somatic cell is a fibroblast.

In another method, Method 6, the present disclosure provides the method of Method 4, wherein the set of pluripotency-associated genes is one or more of the genes selected from the group consisting of OCT4, SOX2, KLF4, Lin28, NANOG and cMYC.

In another method, Method 7, the present disclosure provides the method of any one of Methods 3-6, wherein the editing step comprises introducing into the iPSC one or more deoxyribonucleic acid (DNA) endonucleases to effect one or more single-stranded breaks (SSBs) or double-strand breaks (DSBs) within or near the C9ORF72 gene or other DNA sequences that encode regulatory elements of the C9ORF72 gene that results in permanent deletion, insertion, or correction of the expanded hexanucleotide repeat within or near the C9ORF72 gene or other DNA sequences that encode regulatory elements of the C9ORF72 gene, or within or near a safe harbor locus that results in permanent insertion of the C9ORF72 gene or minigene and restoration of C9orf72 protein activity.

In another method, Method 8, the present disclosure provides the method of Method 7, wherein the safe harbor locus is a safe harbor locus selected from the group consisting of: AAVS1 (PPP1R12C), ALB, Angptl3, ApoC3, ASGR2, CCR5, FIX (F9), G6PC, Gys2, HGD, Lp(a), Pcsk9, Serpina1, TF, and TTR.

In another method, Method 9, the present disclosure provides the method of any one of Methods 3-8, wherein the differentiating step comprises one or more of the following to differentiate the genome edited iPSC into a hematopoietic progenitor cell, a neural progenitor cell, or neural cell: treatment with a combination of small molecules or delivery of master transcription factors.

In another method, Method 10, the present disclosure provides the method of any one of Methods 3-9, wherein the implanting step comprises implanting the hematopoietic progenitor cell, neural progenitor cell, or neural cell into the patient by transplantation, local injection, systemic infusion, or combinations thereof.

In another method, Method 11, the present disclosure provides an *ex vivo* method for treating a patient with amyotrophic lateral sclerosis (ALS) or frontotemporal lobular dementia (FTLD) comprising the steps of: i) isolating a white blood cell from the patient; ii) editing within or near the C9ORF72 gene or other DNA sequences that encode regulatory elements of the C9ORF72 gene of the white blood cell, or editing within or near a safe harbor locus of the white blood cell; and iii) implanting the edited white blood cell into the patient.

In another method, Method 12, the present disclosure provides the method of Method 11, wherein the isolating step comprises: cell differential centrifugation, cell culturing, or combinations thereof.

In another method, Method 13, the present disclosure provides the method of any one of Methods 11-12, wherein the editing step comprises introducing into the neural cell one or more deoxyribonucleic acid (DNA) endonucleases to effect one or more single-strand breaks (SSBs) or one or more double-strand breaks (DSBs) within or near the C9ORF72 gene or other DNA sequences that encode regulatory elements of the C9ORF72 gene that results in permanent deletion, insertion, or correction of the expanded hexanucleotide repeat within or near the C9ORF72 gene or other DNA sequences that encode regulatory elements of the C9ORF72 gene, or within or near a safe harbor locus that results in restoration of C9orf72 protein activity.

In another method, Method 14, the present disclosure provides the method of Method 13, wherein the safe harbor locus is a safe harbor locus selected from the group consisting of: AAVS1 (PPP1R12C), ALB, Angptl3, ApoC3, ASGR2, CCR5, FIX (F9), G6PC, Gys2, HGD, Lp(a), Pcsk9, Serpina1, TF, and TTR.

In another method, Method 15, the present disclosure provides the method of any one of Methods 11-14, wherein the implanting step comprises implanting the edited white blood cell into the patient by transplantation, local injection, systemic infusion, or combinations thereof.

In another method, Method 16, the present disclosure provides an *ex vivo* method for treating a patient with amyotrophic lateral sclerosis (ALS) or frontotemporal lobular dementia (FTLD) comprising the steps of: i) optionally, performing a biopsy of the patient's bone marrow; ii) isolating a mesenchymal stem cell; iii) editing within or near the C9ORF72 gene of the stem cell or other DNA sequences that encode regulatory elements of the C9ORF72 gene of the stem cell, or editing within or near a safe harbor locus of the stem cell; Iv) differentiating the stem cell into a hematopoietic progenitor cell, neural progenitor cell, or neural cell; and v) implanting the hematopoietic progenitor cell, neural progenitor cell, or neural cell into the patient.

In another method, Method 17, the present disclosure provides the method of Method 16, wherein the isolating step comprises: aspiration of bone marrow and isolation of mesenchymal cells by density centrifugation using Percoll™.

In another method, Method 18, the present disclosure provides the method of any one of Methods 16-17, wherein the editing step comprises introducing into the stem cell one or more deoxyribonucleic acid (DNA) endonucleases to effect one or more single-strand breaks (SSBs) or one or more double-strand breaks (DSBs) within or near the C9ORF72 gene or other DNA sequences that encode regulatory elements of the C9ORF72 gene that results in permanent deletion, insertion, or correction of the expanded hexanucleotide repeat within or near the C9ORF72 gene or other DNA sequences that encode regulatory elements of the C9ORF72 gene, or within or near a safe harbor locus that results in restoration of C9orf72 protein activity.

In another method, Method 19, the present disclosure provides the method of Method 18, wherein the safe harbor locus is a safe harbor locus selected from the group consisting of: AAVS1 (PPP1 R12C), ALB, Angptl3, ApoC3, ASGR2, CCR5, FIX (F9), G6PC, Gys2, HGD, Lp(a), Pcsk9, Serpina1, TF, and TTR.

In another method, Method 20, the present disclosure provides the method of any one of Methods 16-19, wherein the differentiating step comprises one or more of the following to differentiate the genome edited stem cell into a hematopoietic progenitor cell, neural progenitor cell, or neural cell: treatment with a combination of small molecules or delivery of master transcription factors.

In another method, Method 21, the present disclosure provides the method of any one of Methods 16-20, wherein the implanting step comprises implanting the cell into the patient by transplantation, local injection, systemic infusion, or combinations thereof.

In another method, Method 22, the present disclosure provides an *ex vivo* method for treating a patient with amyotrophic lateral sclerosis (ALS) or frontotemporal lobular dementia (FTLD) comprising the steps of: i) optionally, treating the patient with granulocyte colony stimulating factor (GCSF); ii) isolating a hematopoietic progenitor cell from the patient; iii) editing within or near the C9ORF72 gene of the hematopoietic progenitor cell or other DNA sequences that encode regulatory elements of the C9ORF72 gene of the hematopoietic progenitor cell, or editing within or near a safe harbor locus of the hematopoietic progenitor cell; and iv) implanting the cell into the patient.

In another method, Method 23, the present disclosure provides the method of Method 22, wherein the treating step is performed in combination with Plerixaflor.

In another method, Method 24, the present disclosure provides the method of any one of Methods 22-23, wherein the isolating step comprises isolating CD34+ cells.

In another method, Method 25, the present disclosure provides the method of any one of Methods 22-24, wherein the editing step comprises introducing into the stem cell one or more deoxyribonucleic acid (DNA) endonucleases to effect one or more single-strand breaks (SSBs) or one or more double-strand breaks (DSBs) within or near the C9ORF72 gene or other DNA sequences that encode regulatory elements of the C9ORF72 gene that results in permanent deletion, insertion, or correction of the expanded hexanucleotide repeat within or near the C9ORF72 gene or other DNA sequences that encode regulatory elements of the C9ORF72 gene, or within or near a safe harbor locus that results in restoration of C9orf72 protein activity.

In another method, Method 26, the present disclosure provides the method of Method 25, wherein the safe harbor locus is a safe harbor locus selected from the group consisting of: AAVS1 (PPP1 R12C), ALB, Angptl3, ApoC3, ASGR2, CCR5, FIX (F9), G6PC, Gys2, HGD, Lp(a), Pcsk9, Serpina1, TF, and TTR.

In another method, Method 27, the present disclosure provides the method of any one of Methods 22-26, wherein the implanting step comprises implanting the neural cell into the patient by transplantation, local injection, systemic infusion, or combinations thereof.

In another method, Method 28, the present disclosure provides an *in vivo* method for treating a patient with amyotrophic lateral sclerosis (ALS) or frontotemporal lobular dementia (FTLD) comprising the step of editing within or near the C9ORF72 gene in a cell of the patient or other DNA sequences that encode regulatory elements of the C9ORF72 gene, or editing within or near a safe harbor locus.

In another method, Method 29, the present disclosure provides the method of Method 28, wherein the editing step comprises introducing into the cell one or more deoxyribonucleic acid (DNA) endonucleases to effect one or more single-strand breaks (SSBs) or one or more double-strand breaks (DSBs) within or near the C9ORF72 gene or other DNA sequences that encode regulatory elements of the C9ORF72 gene that results in permanent deletion, insertion, or correction of the expanded hexanucleotide repeat within or near the C9ORF72 gene or other DNA sequences that encode regulatory elements of the C9ORF72 gene, or within or near a safe harbor locus that results in restoration of C9orf72 protein activity.

In another method, Method 30, the present disclosure provides the method of Method 29, wherein the safe harbor locus is a safe harbor locus selected from the group consisting of: AAVS1 (PPP1 R12C), ALB, Angptl3, ApoC3, ASGR2, CCR5, FIX (F9), G6PC, Gys2, HGD, Lp(a), Pcsk9, Serpina1, TF, and TTR.

In another method, Method 31, the present disclosure provides the method of any one of Methods 28-30, wherein the cell a neural cell, a bone marrow cell, a hematopoietic progenitor cell, or a CD34+ cell.

In another method, Method 32, the present disclosure provides the method of any one of Methods 1, 7, 13, 18, 25 or 29, wherein the one or more DNA endonucleases is a Cas1, Cas1B, Cas2, Cas3, Cas4, Cas5, Cas6, Cas7, Cas8, Cas9 (also known as Csn1 and Csx12), Cas100, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, or Cpf1 endonuclease; or a homolog thereof, recombination of the naturally occurring molecule, codon-optimized, or modified version thereof, and combinations thereof.

In another method, Method 33, the present disclosure provides the method of Method 32, wherein the method comprises introducing into the cell one or more polynucleotides encoding the one or more DNA endonucleases.

In another method, Method 34, the present disclosure provides the method of Method 32, wherein the method comprises introducing into the cell one or more ribonucleic acids (RNAs) encoding the one or more DNA endonucleases.

In another method, Method 35, the present disclosure provides the method of any one of Methods 33 or 34, wherein the one or more polynucleotides or one or more RNAs is one or more modified polynucleotides or one or more modified RNAs.

In another method, Method 36, the present disclosure provides the method of Method 32, wherein the DNA endonuclease is a protein or polypeptide.

In another method, Method 37, the present disclosure provides the method of any one of the preceding Methods, wherein the method further comprises introducing into the cell one or more guide ribonucleic acids (gRNAs).

In another method, Method 38, the present disclosure provides the method of Method 37, wherein the one or more gRNAs are single-molecule guide RNA (sgRNAs).

In another method, Method 39, the present disclosure provides the method of any one of Methods 37-38, wherein the one or more gRNAs or one or more sgRNAs is one or more modified gRNAs or one or more modified sgRNAs.

In another method, Method 40, the present disclosure provides the method of any one of Methods 37-39, wherein the one or more DNA endonucleases is pre-complexed with one or more gRNAs or one or more sgRNAs.

In another method, Method 41, the present disclosure provides the method of any one of the preceding Methods, wherein the method further comprises introducing into the cell a polynucleotide donor template comprising a part of the wild-type C9ORF72 gene or minigene or cDNA.

In another method, Method 42, the present disclosure provides the method of Method 41, wherein the part of the wild-type C9ORF72 gene or cDNA is the entire C9ORF72 gene or cDNA, or the cDNA of natural Variant 1, Variant 2, or Variant 3.

In another method, Method 43, the present disclosure provides the method of any one of Methods 41-42, wherein the donor template is either single or double stranded.

In another method, Method 44, the present disclosure provides the method of any one of Methods 41-43, wherein the donor template has homologous arms to the 9p21.2 region.

In another method, Method 45, the present disclosure provides the method of any one of Methods 41-43, wherein the donor template has homologous arms to a safe harbor locus selected from the group consisting of exon 1-2 of AAVS1 (PPP1R12C), exon 1-2 of ALB, exon 1-2 of Angptl3, exon 1-2 of ApoC3, exon 1-2 of ASGR2, exon 1-2 of CCR5, exon 1-2 of FIX (F9), exon 1-2 of G6PC, exon 1-2 of Gys2, exon 1-2 of HGD, exon 1-2 of Lp(a), exon 1-2 of Pcsk9, exon 1-2 of Serpina1, exon 1-2 of TF, and exon 1-2 of TTR.

In another method, Method 46, the present disclosure provides the method of any one of Methods 1, 7, 13, 18, 25 or 29, wherein the method further comprises introducing into the cell one guide ribonucleic acid (gRNA) and a polynucleotide donor template comprising at least a portion of the wild-type C9ORF72 gene, and wherein the one or more DNA endonucleases is one or more Cas9 or Cpf1 endonucleases that effect one single-strand break (SSB) or double-strand break (DSB) at a DSB locus within or near the C9ORF72 gene or other DNA sequences that encode regulatory elements of the C9ORF72 gene, or within or near a safe harbor locus that facilitates insertion of a new sequence from the polynucleotide donor template into the chromosomal DNA at the locus or safe harbor that results in permanent insertion or correction of a part of the chromosomal DNA of the C9ORF72 gene or other DNA sequences that encode regulatory elements of the C9ORF72 gene proximal to the locus or safe harbor locus and restoration of C9orf72 protein activity, and wherein the gRNA comprises a spacer sequence that is complementary to a segment of the locus or locus.

In another method, Method 47, the present disclosure provides the method of Method 46, wherein proximal means nucleotides both upstream and downstream of the locus or locus.

In another method, Method 48, the present disclosure provides the method of any one of Methods 1, 7, 13, 18, 25 or 29, wherein the method further comprises introducing into the cell two guide ribonucleic acid (gRNAs) and a polynucleotide donor template comprising at least a portion of the wild-type C9ORF72 gene, and wherein the one or more DNA endonucleases is two or more Cas9 or Cpf1 endonucleases that effect a pair of single-strand break (SSB) or double-strand breaks (DSBs), the first at a 5' locus and the second at a 3' locus, within or near the C9ORF72 gene or other DNA sequences that encode regulatory elements of the C9ORF72 gene, or within or near a safe harbor locus that facilitates insertion of a new sequence from the polynucleotide donor template into the chromosomal DNA between the 5' locus and the 3' locus that results in permanent insertion or correction of the chromosomal DNA between the 5' locus and the 3' locus within or near the C9ORF72 gene or other DNA sequences that encode regulatory elements of the C9ORF72 gene or safe harbor locus and restoration of C9orf72 protein activity, and wherein the first guide RNA comprises a spacer sequence that is complementary to a segment of the 5' locus and the second guide RNA comprises a spacer sequence that is complementary to a segment of the 3' locus.

In another method, Method 49, the present disclosure provides the method of any one of Methods 46-48, wherein the one or two gRNAs are one or two single-molecule guide RNA (sgRNAs).

In another method, Method 50, the present disclosure provides the method of any one of Methods 46-49, wherein the one or two gRNAs or one or two sgRNAs is one or two modified gRNAs or one or two modified sgRNAs.

In another method, Method 51, the present disclosure provides the method of any one of Methods 46-50, wherein the one or more DNA endonucleases is pre-complexed with one or two gRNAs or one or two sgRNAs.

In another method, Method 52, the present disclosure provides the method of any one of Methods 46-51, wherein the part of the wild-type C9ORF72 gene or cDNA is the entire C9ORF72 gene or cDNA; the cDNA of natural Variant 1, Variant 2, Variant 3; or about 30 hexanucleotide repeats.

In another method, Method 53, the present disclosure provides the method of any one of Methods 46-52, wherein the donor template is either single or double stranded polynucleotide.

In another method, Method 54, the present disclosure provides the method of any one of Methods 46-53, wherein the donor template has homologous arms to the 9p21.2 region.

In another method, Method 55, the present disclosure provides the method of any one of Methods 46-44, wherein the donor template has homologous arms to a safe harbor locus selected from the group consisting of exon 1-2 of AAVS1 (PPP1 R12C), exon 1-2 of ALB, exon 1-2 of Angptl3, exon 1-2 of ApoC3, exon 1-2 of ASGR2, exon 1-2 of CCR5, exon 1-2 of FIX (F9), exon 1-2 of G6PC, exon 1-2 of Gys2, exon 1-2 of HGD, exon 1-2 of Lp(a), exon 1-2 of Pcsk9, exon 1-2 of Serpina1, exon 1-2 of TF, and exon 1-2 of TTR.

In another method, Method 56, the present disclosure provides the method of Method 44, wherein the DSB, or 5' DSB and 3' DSB are in the first intron of the C9ORF72 gene.

In another method, Method 57, the present disclosure provides the method of any one of Methods 1, 7, 13, 18, 25 or 29-56, wherein the insertion or correction is by homology directed repair (HDR).

In another method, Method 58, the present disclosure provides the method of any one of Methods 1, 7, 13, 18, 25 or 29, wherein the method further comprises introducing into the cell two guide ribonucleic acid (gRNAs), and wherein the one or more DNA endonucleases is two or more Cas9 or Cpf1 endonucleases that effect a pair of double-strand breaks (DSBs), the first at a 5' DSB locus and the second at a 3' DSB locus, within or near the C9ORF72 gene that causes a deletion of the chromosomal DNA between the 5' DSB locus and the 3' DSB locus that results in permanent deletion of the chromosomal DNA between the 5' DSB locus and the 3' DSB locus within or near the C9ORF72 gene, and wherein the first guide RNA comprises a spacer sequence that is complementary to a segment of the 5' DSB locus and the second guide RNA comprises a spacer sequence that is complementary to a segment of the 3' DSB locus.

In another method, Method 59, the present disclosure provides the method of Method 58, wherein the two gRNAs are two single-molecule guide RNA (sgRNAs).

In another method, Method 60, the present disclosure provides the method of any one of Methods 58-59, wherein the two gRNAs or two sgRNAs are two modified gRNAs or two modified sgRNAs.

In another method, Method 61, the present disclosure provides the method of any one of Methods 58-60, wherein the one or more DNA endonucleases is pre-complexed with one or two gRNAs or one or two sgRNAs.

In another method, Method 62, the present disclosure provides the method of any one of Methods 58-61, wherein both the 5' DSB and 3' DSB are in or near either the first exon, first intron, or second exon of the C9ORF72 gene.

In another method, Method 63, the present disclosure provides the method of any one of Method 58-62, wherein the deletion is a deletion of the expanded hexanucleotide repeat.

In another method, Method 64, the present disclosure provides the method of any one of Methods 1, 7, 13, 18, 25 or 29-63, wherein the Cas9 or Cpf1 mRNA, gRNA, and donor template are either each formulated separately into lipid nanoparticles or all co-formulated into a lipid nanoparticle.

In another method, Method 65, the present disclosure provides the method of any one of Methods 1, 7, 13, 18, 25 or 29-63, wherein the Cas9 or Cpf1 mRNA is formulated into a lipid nanoparticle, and both the gRNA and donor template are delivered by a viral vector.

In another method, Method 66, the present disclosure provides the method of Method 65, wherein the viral vector is an adeno-associated virus (AAV) vector.

In another method, Method 67, the present disclosure provides the method of Method 66, wherein the AAV vector is an AAV6 vector.

In another method, Method 68, the present disclosure provides the method of any one of Methods 1, 7, 13, 18, 25 or 29-63, wherein the Cas9 or Cpf1 mRNA, gRNA, and donor template are either each formulated into separate exosomes or all co-formulated into an exosome.

In another method, Method 69, the present disclosure provides the method of any one of Methods 1, 7, 13, 18, 25 or 29-63, wherein the Cas9 or Cpf1 mRNA is formulated into a lipid nanoparticle, and the gRNA is delivered to the cell by electroporation and donor template is delivered to the cell by a viral vector.

In another method, Method 70, the present disclosure provides the method of Methods 69, wherein the gRNA is delivered to the cell by electroporation and donor template is delivered to the cell by an adeno-associated virus (AAV) vector.

In another method, Method 71, the present disclosure provides the method of Method 70, wherein the AAV vector is an AAV6 vector.

In another method, Method 72, the present disclosure provides the method of any one of the preceding Methods, wherein the C9ORF72 gene is located on Chromosome 9: 27,546,542 - 27,573,863 (Genome Reference Consortium - GRCh38/hg38).

In another method, Method 73, the present disclosure provides the method of any one of Methods 1, 7, 13, 18, 25 or 29, wherein the restoration of C9orf72 protein activity is compared to wild-type or normal C9orf72 protein activity.

In another method, Method 74, the present disclosure provides a method for treating a patient with amyotrophic lateral sclerosis (ALS) or frontotemporal lobular dementia (FTLD) comprising transplanting the bone marrow from a donor to the patient.

The present disclosure also provides a composition, Composition 1, of one or more guide ribonucleic acids (gRNAs) comprising a spacer sequence selected from the group consisting of the nucleic acid sequences in SEQ ID NOs: 1 - 18807 for editing the C9ORF72 gene in a cell from a patient with amyotrophic lateral sclerosis (ALS) or frontotemporal lobular dementia (FTLD).

In another composition, Composition 2, the present disclosure provides the composition of Composition 1, wherein the one or more gRNAs are one or more single-molecule guide RNAs (sgRNAs).

In another composition, Composition 3, the present disclosure provides the composition of Composition 1 or Composition 2, wherein the one or more gRNAs or one or more sgRNAs is one or more modified gRNAs or one or more modified sgRNAs.

The present disclosure also provides a composition, Composition 4, of one or more guide ribonucleic acids (gRNAs) comprising a spacer sequence selected from the group consisting of the nucleic acid sequences in SEQ ID NOs: 18810-73668 for editing the C9ORF72 gene in a cell from a patient with amyotrophic lateral sclerosis (ALS) or frontotemporal lobular dementia (FTLD).

In another composition, Composition 5, the present disclosure provides the composition of Composition 4, wherein the one or more gRNAs are one or more single-molecule guide RNAs (sgRNAs).

In another composition, Composition 6, the present disclosure provides the composition of Composition 4 or Composition 5, wherein the one or more gRNAs or one or more sgRNAs is one or more modified gRNAs or one or more modified sgRNAs.

### Definitions

The term "comprising" or "comprises" is used in reference to compositions, methods, and respective component(s) thereof, that are essential to the invention, yet open to the inclusion of unspecified elements, whether essential or not.

The term "consisting essentially of" refers to those elements required for a given embodiment. The term permits the presence of additional elements that do not materially affect the basic and novel or functional characteristic(s) of that embodiment of the invention.

The term "consisting of" refers to compositions, methods, and respective components thereof as described herein, which are exclusive of any element not recited in that description of the embodiment.

The singular forms "a," "an," and "the" include plural references, unless the context clearly dictates otherwise.

Certain numerical values presented herein are preceded by the term "about." The term "about" is used to provide literal support for the numerical value the term "about" precedes, as well as a numerical value that is approximately the numerical value, that is the approximating unrecited numerical value may be a number which, in the context it is presented, is the substantial equivalent of the specifically recited numerical value. The term "about" means numerical values within + 10% of the recited numerical value.

When a range of numerical values is presented herein, it is contemplated that each intervening value between the lower and upper limit of the range, the values that are the upper and lower limits of the range, and all stated values with the range are encompassed within the disclosure. All the possible sub-ranges within the lower and upper limits of the range are also contemplated by the disclosure.

### Examples

The invention will be more fully understood by reference to the following examples, which provide illustrative non-limiting embodiments of the invention.

The examples describe the use of the CRISPR system as an illustrative genome editing technique to create defined therapeutic genomic replacements, termed "genomic modifications" herein, in the C9ORF72 gene that lead to permanent correction of expanded hexanucleotide repeats in the genomic locus, or expression at a heterologous locus, that restore wild-type or similar levels of hexanucleotide repeats. Introduction of the defined therapeutic modifications represents a novel therapeutic strategy for the potential amelioration of ALS and/or FTLD, as described and illustrated herein.

### Example 1 - CRISPR/SpCas9 target sites for the C9ORF72 gene

Regions of the C9ORF72 gene were scanned for target sites. Each area was scanned for a protospacer adjacent motif (PAM) having the sequence NRG. gRNA 20 bp spacer sequences corresponding to the PAM were identified, as provided in SEQ ID NOs: 1-6148.

### Example 2 - CRISPR/SaCas9 target sites for the C9ORF72 gene

Regions of the C9ORF72 gene were scanned for target sites. Each area was scanned for a protospacer adjacent motif (PAM) having the sequence NNGRRT. gRNA 20 bp spacer sequences corresponding to the PAM were identified, as provided in SEQ ID NOs: 6149-6892.

### Example 3 - CRISPR/StCas9 target sites for the C9ORF72 gene

Regions of the C9ORF72 gene were scanned for target sites. Each area was scanned for a protospacer adjacent motif (PAM) having the sequence NNAGAAW. gRNA 24 bp spacer sequences corresponding to the PAM were identified, as provided in SEQ ID NOs: 7760-8097.

### Example 4 - CRISPR/TdCas9 target sites for the C9ORF72 gene

Regions of the C9ORF72 gene were scanned for target sites. Each area was scanned for a protospacer adjacent motif (PAM) having the sequence NAAAAC. gRNA 24 bp spacer sequences corresponding to the PAM were identified, as provided in SEQ ID NOs: 8098-8261.

### Example 5 - CRISPR/NmCas9 target sites for the C9ORF72 gene

Regions of the C9ORF72 gene were scanned for target sites. Each area was scanned for a protospacer adjacent motif (PAM) having the sequence NNNNGHTT. gRNA 24 bp spacer sequences corresponding to the PAM were identified, as provided in SEQ ID NOs: 6893-7759.

### Example 6 - CRISPR/Cpf1 target sites for the C9ORF72 gene

Regions of the C9ORF72 gene were scanned for target sites. Each area was scanned for a protospacer adjacent motif (PAM) having the sequence YTN. gRNA 24 bp spacer sequences corresponding to the PAM were identified, as provided in SEQ ID NOs: 8262-18807.

### Example 7 - CRISPR/SpCas9 target sites for the AAVS1 (PPP1R12C) gene

Exons 1-2 of the AAVS1 (PPP1 R12C) gene were scanned for target sites. Each area was scanned for a protospacer adjacent motif (PAM) having the sequence NRG. gRNA 20 bp spacer sequences corresponding to the PAM were identified, as shown in SEQ ID NOs: 18810-20841.

### Example 8 - CRISPR/SaCas9 target sites for the AAVS1 (PPP1R12C) gene

Exons 1-2 of the AAVS1 (PPP1 R12C) gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 20842-21012.

### Example 9 - CRISPR/StCas9 target sites for the AAVS1 (PPP1R12C) gene

Exons 1-2 of the AAVS1 (PPP1 R12C) gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 21013-21030.

### Example 10 - CRISPR/TdCas9 target sites for the AAVS1 (PPP1R12C) gene

Exons 1-2 of the AAVS1 (PPP1 R12C) gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 21031-21039.

### Example 11 - CRISPR/NmCas9 target sites for the AAVS1 (PPP1R12C) gene

Exons 1-2 of the AAVS1 (PPP1 R12C) gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 21040-21114.

### Example 12 - CRISPR/Cpf1 target sites for the AAVS1 (PPP1R12C) gene

Exons 1-2 of the AAVS1 (PPP1 R12C) gene were scanned for target sites. Each area was scanned for a protospacer adjacent motif (PAM) having the sequence YTN. gRNA 22 bp spacer sequences corresponding to the PAM were identified, as shown in SEQ ID NOs: 21115-22290.

### Example 13 - CRISPR/SpCas9 target sites for the ALB gene

Exons 1-2 of the ALB gene were scanned for target sites. Each area was scanned for a protospacer adjacent motif (PAM) having the sequence NRG. gRNA 20 bp spacer sequences corresponding to the PAM were identified, as shown in SEQ ID NOs: 22291-22458.

### Example 14 - CRISPR/SaCas9 target sites for the ALB gene

Exons 1-2 of the ALB gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 22459-22486.

### Example 15 - CRISPR/StCas9 target sites for the ALB gene

Exons 1-2 of the ALB gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 22487-22504.

### Example 16 - CRISPR/TdCas9 target sites for the ALB gene

Exons 1-2 of the ALB gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 22505-22509.

### Example 17 - CRISPR/NmCas9 target sites for the ALB gene

Exons 1-2 of the ALB gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 22510-22533.

### Example 18 - CRISPR/Cpf1 target sites for the ALB gene

Exons 1-2 of the ALB gene were scanned for target sites. Each area was scanned for a protospacer adjacent motif (PAM) having the sequence YTN. gRNA 22 bp spacer sequences corresponding to the PAM were identified, as shown in SEQ ID NOs: 22534-22912.

### Example 19 - CRISPR/SpCas9 target sites for the Angptl3 gene

Exons 1-2 of the Angptl3 gene were scanned for target sites. Each area was scanned for a protospacer adjacent motif (PAM) having the sequence NRG. gRNA 20 bp spacer sequences corresponding to the PAM were identified, as shown in SEQ ID NOs: 22913-23257.

### Example 20 - CRISPR/SaCas9 target sites for the Anaptl3 gene

Exons 1-2 of the Angptl3 gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 23258-23293.

### Example 21 - CRISPR/StCas9 target sites for the Anaptl3 gene

Exons 1-2 of the Angptl3 gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 23294-23316.

### Example 22 - CRISPR/TdCas9 target sites for the Anaptl3 gene

Exons 1-2 of the Angptl3 gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 23317-23329.

### Example 23 - CRISPR/NmCas9 target sites for the Anaptl3 gene

Exons 1-2 of the Angptl3 gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 23330-23392.

### Example 24 - CRISPR/Cpf1 target sites for the Anaptl3 gene

Exons 1-2 of the Angptl3 gene were scanned for target sites. Each area was scanned for a protospacer adjacent motif (PAM) having the sequence YTN. gRNA 22 bp spacer sequences corresponding to the PAM were identified, as shown in SEQ ID NOs: 23393-24240.

### Example 25 - CRISPR/SpCas9 target sites for the ApoC3 gene

Exons 1-2 of the ApoC3 gene were scanned for target sites. Each area was scanned for a protospacer adjacent motif (PAM) having the sequence NRG. gRNA 20 bp spacer sequences corresponding to the PAM were identified, as shown in SEQ ID NOs: 24241-24643.

### Example 26 - CRISPR/SaCas9 target sites for the ApoC3 gene

Exons 1-2 of the ApoC3 gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 24644-24668.

### Example 27 - CRISPR/StCas9 target sites for the ApoC3 gene

Exons 1-2 of the ApoC3 gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 24669-24671.

### Example 28 - CRISPR/TdCas9 target sites for the ApoC3 gene

Exons 1-2 of the ApoC3 gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 24672-24673.

### Example 29 - CRISPR/NmCas9 target sites for the ApoC3 gene

Exons 1-2 of the ApoC3 gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 24674-24685.

### Example 30 - CRISPR/Cpf1 target sites for the ApoC3 gene

Exons 1-2 of the ApoC3 gene were scanned for target sites. Each area was scanned for a protospacer adjacent motif (PAM) having the sequence YTN. gRNA 22 bp spacer sequences corresponding to the PAM were identified, as shown in SEQ ID NOs: 24686-24917.

### Example 31 - CRISPR/SpCas9 target sites for the ASGR2 gene

Exons 1-2 of the ASGR2 gene were scanned for target sites. Each area was scanned for a protospacer adjacent motif (PAM) having the sequence NRG. gRNA 20 bp spacer sequences corresponding to the PAM were identified, as shown in SEQ ID NOs: 24918-26685.

### Example 32 - CRISPR/SaCas9 target sites for the ASGR2 gene

Exons 1-2 of the ASGR2 gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 26686-26891.

### Example 33 - CRISPR/StCas9 target sites for the ASGR2 gene

Exons 1-2 of the ASGR2 gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 26892-26915.

### Example 34 - CRISPR/TdCas9 target sites for the ASGR2 gene

Exons 1-2 of the ASGR2 gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 26916-26927.

### Example 35 - CRISPR/NmCas9 target sites for the ASGR2 gene

Exons 1-2 of the ASGR2 gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 26928-27010.

### Example 36 - CRISPR/Cpf1 target sites for the ASGR2 gene

Exons 1-2 of the ASGR2 gene were scanned for target sites. Each area was scanned for a protospacer adjacent motif (PAM) having the sequence YTN. gRNA 22 bp spacer sequences corresponding to the PAM were identified, as shown in SEQ ID NOs: 27011-28450.

### Example 37 - CRISPR/SpCas9 target sites for the CCR5 gene

Exons 1-2 of the CCR5 gene were scanned for target sites. Each area was scanned for a protospacer adjacent motif (PAM) having the sequence NRG. gRNA 20 bp spacer sequences corresponding to the PAM were identified, as shown in SEQ ID NOs: 28451-28653.

### Example 38 - CRISPR/SaCas9 target sites for the CCR5 gene

Exons 1-2 of the CCR5 gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 28654-28685.

### Example 39 - CRISPR/StCas9 target sites for the CCR5 gene

Exons 1-2 of the CCR5 gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 28686-28699.

### Example 40 - CRISPR/TdCas9 target sites for the CCR5 gene

Exons 1-2 of the CCR5 gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 28700-28701.

### Example 41 - CRISPR/NmCas9 target sites for the CCR5 gene

Exons 1-2 of the CCR5 gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 28702-28729.

### Example 42 - CRISPR/Cpf1 target sites for the CCR5 gene

Exons 1-2 of the CCR5 gene were scanned for target sites. Each area was scanned for a protospacer adjacent motif (PAM) having the sequence YTN. gRNA 22 bp spacer sequences corresponding to the PAM were identified, as shown in SEQ ID NOs: 28730-29029.

### Example 43 - CRISPR/SpCas9 target sites for the FIX (F9) gene

Exons 1-2 of the FIX (F9) gene were scanned for target sites. Each area was scanned for a protospacer adjacent motif (PAM) having the sequence NRG. gRNA 20 bp spacer sequences corresponding to the PAM were identified, as shown in SEQ ID NOs: 29030-30495.

### Example 44 - CRISPR/SaCas9 target sites for the FIX (F9) gene

Exons 1-2 of the FIX (F9) gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 30496-30658.

### Example 45 - CRISPR/StCas9 target sites for the FIX (F9) gene

Exons 1-2 of the FIX (F9) gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 30659-30719.

### Example 46 - CRISPR/TdCas9 target sites for the FIX (F9) gene

Exons 1-2 of the FIX (F9) gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 30720-30744.

### Example 47 - CRISPR/NmCas9 target sites for the FIX (F9) gene

Exons 1-2 of the FIX (F9) gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 30745-30897.

### Example 48 - CRISPR/Cpf1 target sites for the FIX (F9) gene

Exons 1-2 of the FIX (F9) gene were scanned for target sites. Each area was scanned for a protospacer adjacent motif (PAM) having the sequence YTN. gRNA 22 bp spacer sequences corresponding to the PAM were identified, as shown in SEQ ID NOs: 30898-33038.

### Example 49 - CRISPR/SpCas9 target sites for the G6PC gene

Regions of the G6PC gene were scanned for target sites. Each area was scanned for a protospacer adjacent motif (PAM) having the sequence NRG. gRNA 20 bp spacer sequences corresponding to the PAM were identified, as shown in SEQ ID NOs: 33039-34054.

### Example 50 - CRISPR/SaCas9 target sites for the G6PC gene

Regions of the G6PC gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 34055-34171.

### Example 51 - CRISPR/StCas9 target sites for the G6PC gene

Regions of the G6PC gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 34172-34195.

### Example 52 - CRISPR/TdCas9 target sites for the G6PC gene

Regions of the G6PC gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 34196-34204.

### Example 53 - CRISPR/NmCas9 target sites for the G6PC gene

Regions of the G6PC gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 34205-34294.

### Example 54 - CRISPR/Cpf1 target sites for the G6PC gene

Regions of the G6PC gene were scanned for target sites. Each area was scanned for a protospacer adjacent motif (PAM) having the sequence YTN. gRNA 22 bp spacer sequences corresponding to the PAM were identified, as shown in SEQ ID NOs: 34295-35389.

### Example 55 - CRISPR/SpCas9 target sites for the Gvs2 gene

Exons 1-2 of the Gys2 gene were scanned for target sites. Each area was scanned for a protospacer adjacent motif (PAM) having the sequence NRG. gRNA 20 bp spacer sequences corresponding to the PAM were identified, as shown in SEQ ID NOs: 35390-40882.

### Example 56 - CRISPR/SaCas9 target sites for the Gvs2 gene

Exons 1-2 of the Gys2 gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 40883-41558.

### Example 57 - CRISPR/StCas9 target sites for the Gvs2 gene

Exons 1-2 of the Gys2 gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 41559-41836.

### Example 58 - CRISPR/TdCas9 target sites for the Gvs2 gene

Exons 1-2 of the Gys2 gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 41837-41950.

### Example 59 - CRISPR/NmCas9 target sites for the Gys2 gene

Exons 1-2 of the Gys2 gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 41951-42630.

### Example 60 - CRISPR/Cpf1 target sites for the Gvs2 gene

Exons 1-2 of the Gys2 gene were scanned for target sites. Each area was scanned for a protospacer adjacent motif (PAM) having the sequence YTN. gRNA 22 bp spacer sequences corresponding to the PAM were identified, as shown in SEQ ID NOs: 42631-51062.

### Example 61 - CRISPR/SpCas9 target sites for the HGD gene

Exons 1-2 of the HGD gene were scanned for target sites. Each area was scanned for a protospacer adjacent motif (PAM) having the sequence NRG. gRNA 20 bp spacer sequences corresponding to the PAM were identified, as shown in SEQ ID NOs: 51063-52755.

### Example 62 - CRISPR/SaCas9 target sites for the HGD gene

Exons 1-2 of the HGD gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 52756-52969.

### Example 63 - CRISPR/StCas9 target sites for the HGD gene

Exons 1-2 of the HGD gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 52970-53052.

### Example 64 - CRISPR/TdCas9 target sites for the HGD gene

Exons 1-2 of the HGD gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 53053-53071.

### Example 65 - CRISPR/NmCas9 target sites for the HGD gene

Exons 1-2 of the HGD gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 53072-53272.

### Example 66 - CRISPR/Cpf1 target sites for the HGD gene

Exons 1-2 of the HGD gene were scanned for target sites. Each area was scanned for a protospacer adjacent motif (PAM) having the sequence YTN. gRNA 22 bp spacer sequences corresponding to the PAM were identified, as shown in SEQ ID NOs: 53273-55597.

### Example 67 - CRISPR/SpCas9 target sites for the Lp(a) gene

Exons 1-2 of the Lp(a) gene were scanned for target sites. Each area was scanned for a protospacer adjacent motif (PAM) having the sequence NRG. gRNA 20 bp spacer sequences corresponding to the PAM were identified, as shown in SEQ ID NOs: 55598-59392.

### Example 68 - CRISPR/SaCas9 target sites for the Lp(a) gene

Exons 1-2 of the Lp(a) gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 59393-59802.

### Example 69 - CRISPR/StCas9 target sites for the Lp(a) gene

Exons 1-2 of the Lp(a) gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 59803-59938.

### Example 70 - CRISPR/TdCas9 target sites for the Lp(a) gene

Exons 1-2 of the Lp(a) gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 59939-59973.

### Example 71 - CRISPR/NmCas9 target sites for the Lp(a) gene

Exons 1-2 of the Lp(a) gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 59974-60341.

### Example 72 - CRISPR/Cpf1 target sites for the Lp(a) gene

Exons 1-2 of the Lp(a) gene were scanned for target sites. Each area was scanned for a protospacer adjacent motif (PAM) having the sequence YTN. gRNA 22 bp spacer sequences corresponding to the PAM were identified, as shown in SEQ ID NOs: 60342-64962.

### Example 73 - CRISPR/SpCas9 target sites for the PCSK9 gene

Exons 1-2 of the PCSK9 gene were scanned for target sites. Each area was scanned for a protospacer adjacent motif (PAM) having the sequence NRG. gRNA 20 bp spacer sequences corresponding to the PAM were identified, as shown in SEQ ID NOs: 64963-66982.

### Example 74 - CRISPR/SaCas9 target sites for the PCSK9 gene

Exons 1-2 of the PCSK9 gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 66983-67169.

### Example 75 - CRISPR/StCas9 target sites for the PCSK9 gene

Exons 1-2 of the PCSK9 gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 67170-67205.

### Example 76 - CRISPR/TdCas9 target sites for the PCSK9 gene

Exons 1-2 of the PCSK9 gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 67206-67219.

### Example 77 - CRISPR/NmCas9 target sites for the PCSK9 gene

Exons 1-2 of the PCSK9 gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 67220-67359.

### Example 78 - CRISPR/Cpf1 target sites for the PCSK9 gene

Exons 1-2 of the PCSK9 gene were scanned for target sites. Each area was scanned for a protospacer adjacent motif (PAM) having the sequence YTN. gRNA 22 bp spacer sequences corresponding to the PAM were identified, as shown in SEQ ID NOs: 67360-69153.

### Example 79 - CRISPR/SpCas9 target sites for the Serpina1 gene

Exons 1-2 of the Serpina1 gene were scanned for target sites. Each area was scanned for a protospacer adjacent motif (PAM) having the sequence NRG. gRNA 20 bp spacer sequences corresponding to the PAM were identified, as shown in SEQ ID NOs: 69154-70291.

### Example 80 - CRISPR/SaCas9 target sites for the Serpina1 gene

Exons 1-2 of the Serpina1 gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 70292-70384.

### Example 81 - CRISPR/StCas9 target sites for the Serpina1 gene

Exons 1-2 of the Serpina1 gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 70385-70396.

### Example 82 - CRISPR/TdCas9 target sites for the Serpina1 gene

Exons 1-2 of the Serpina1 gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 70397-70399.

### Example 83 - CRISPR/NmCas9 target sites for the Serpina1 gene

Exons 1-2 of the Serpina1 gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 70400-70450.

### Example 84 - CRISPR/Cpf1 target sites for the Serpina1 gene

Exons 1-2 of the Serpina1 gene were scanned for target sites. Each area was scanned for a protospacer adjacent motif (PAM) having the sequence YTN. gRNA 22 bp spacer sequences corresponding to the PAM were identified, as shown in SEQ ID NOs: 70451-71254.

### Example 85 - CRISPR/SpCas9 target sites for the TF gene

Exons 1-2 of the TF gene were scanned for target sites. Each area was scanned for a protospacer adjacent motif (PAM) having the sequence NRG. gRNA 20 bp spacer sequences corresponding to the PAM were identified, as shown in SEQ ID NOs: 71255-72086.

### Example 86 - CRISPR/SaCas9 target sites for the TF gene

Exons 1-2 of the TF gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 72087-72172.

### Example 87 - CRISPR/StCas9 target sites for the TF gene

Exons 1-2 of the TF gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 72173-72184.

### Example 88 - CRISPR/TdCas9 target sites for the TF gene

Exons 1-2 of the TF gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 72185-72191.

### Example 89 - CRISPR/NmCas9 target sites for the TF gene

Exons 1-2 of the TF gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 72192-72235.

### Example 90 - CRISPR/Cpf1 target sites for the TF gene

Exons 1-2 of the TF gene were scanned for target sites. Each area was scanned for a protospacer adjacent motif (PAM) having the sequence YTN. gRNA 22 bp spacer sequences corresponding to the PAM were identified, as shown in SEQ ID NOs: 72236-72871.

### Example 91 - CRISPR/SpCas9 target sites for the TTR gene

Exons 1-2 of the TTR gene were scanned for target sites. Each area was scanned for a protospacer adjacent motif (PAM) having the sequence NRG. gRNA 20 bp spacer sequences corresponding to the PAM were identified, as shown in SEQ ID NOs: 72872-73171.

### Example 92 - CRISPR/SaCas9 target sites for the TTR gene

Exons 1-2 of the TTR gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 73172-73212.

### Example 93 - CRISPR/StCas9 target sites for the TTR gene

Exons 1-2 of the TTR gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 73213-73229.

### Example 94 - CRISPR/TdCas9 target sites for the TTR gene

Exons 1-2 of the TTR gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 73230-73231.

### Example 95 - CRISPR/NmCas9 target sites for the TTR gene

Exons 1-2 of the TTR gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 73232-73266.

### Example 96 - CRISPR/Cpf1 target sites for the TTR gene

Exons 1-2 of the TTR gene were scanned for target sites. Each area was scanned for a protospacer adjacent motif (PAM) having the sequence YTN. gRNA 22 bp spacer sequences corresponding to the PAM were identified, as shown in SEQ ID NOs: 73267-73668.

### Example 97 - Bioinformatics analysis of the guide strands

Candidate guides will be screened and selected in a multi-step process that involves both theoretical binding and experimentally assessed activity. By way of illustration, candidate guides having sequences that match a particular on-target site, such as a site within the C9ORF72 gene, with adjacent PAM can be assessed for their potential to cleave at off-target sites having similar sequences, using one or more of a variety of bioinformatics tools available for assessing off-target binding, as described and illustrated in more detail below, in order to assess the likelihood of effects at chromosomal positions other than those intended. Candidates predicted to have relatively lower potential for off-target activity can then be assessed experimentally to measure their on-target activity, and then off-target activities at various sites. Preferred guides have sufficiently high on-target activity to achieve desired levels of gene editing at the selected locus, and relatively lower off-target activity to reduce the likelihood of alterations at other chromosomal loci. The ratio of on-target to off-target activity is often referred to as the "specificity" of a guide.

For initial screening of predicted off-target activities, there are a number of bioinformatics tools known and publicly available that can be used to predict the most likely off-target sites; and since binding to target sites in the CRISPR/Cas9/Cpf1 nuclease system is driven by Watson-Crick base pairing between complementary sequences, the degree of dissimilarity (and therefore reduced potential for off-target binding) is essentially related to primary sequence differences: mismatches and bulges, *i.e.* bases that are changed to a non-complementary base, and insertions or deletions of bases in the potential off-target site relative to the target site. An exemplary bioinformatics tool called COSMID (CRISPR Off-target Sites with Mismatches, Insertions and Deletions) (available on the web at crispr.bme.gatech.edu) compiles such similarities. Other bioinformatics tools include, but are not limited to, GUIDO, autoCOSMID, and CCtop.

Bioinformatics were used to minimize off-target cleavage in order to reduce the detrimental effects of mutations and chromosomal rearrangements. Studies on CRISPR /Cas9 systems suggested the possibility of high off-target activity due to nonspecific hybridization of the guide strand to DNA sequences with base pair mismatches and/or bulges, particularly at positions distal from the PAM region. (See e.g., Hsu, P.D. et al. DNA targeting specificity of RNA-guided Cas9 nucleases. Nat Biotechnol 31, 827-832 (2013); Fu, Y. et al. High-frequency off-target mutagenesis induced by CRISPR-Cas nucleases in human cells. Nat Biotechnol (2013); Cradick, T.J., Fine, E.J., Antico, C.J. & Bao, G. CRISPR/Cas9 systems targeting β-globin and CCR5 genes have substantial off-target activity. Nucleic Acids Research 41, 9584-9592 (2013); Lin, Y. et al. CRISPR/Cas9 systems have off-target activity with insertions or deletions between target DNA and guide RNA sequences. Nucleic Acids Res 42, 7473-7485 (2014)). Therefore, it is important to have a bioinformatics tool that can identify potential off-target sites that have insertions and/or deletions between the RNA guide strand and genomic sequences, in addition to base-pair mismatches. The bioinformatics-based tool, COSMID (CRISPR Off-target Sites with Mismatches, Insertions and Deletions) was therefore used to search genomes for potential CRISPR off-target sites (available on the web at http_crispr_bme_gatech_edu). COSMID output ranked lists of the potential off-target sites based on the number and location of mismatches, allowing more informed choice of target sites, and avoiding the use of sites with more likely off-target cleavage.

Additional bioinformatics pipelines were employed that weigh the estimated on- and/or off-target activity of gRNA targeting sites in a region. Other features that may be used to predict activity include information about the cell type in question, DNA accessibility, chromatin state, transcription factor binding sites, transcription factor binding data, and other CHIP-seq data. Additional factors are weighed that predict editing efficiency such as relative positions and directions of pairs of gRNAs, local sequence features and micro-homologies. (See e.g., Naito, Y., Hino, K., Bono, H. & Ui-Tei, K. CRISPRdirect: software for designing CRISPR/Cas guide RNA with reduced off-target sites. Bioinformatics 31, 1120-1123 (2015); Cradick, T.J., Qui, P., Lee, C.M., Fine, E.J. & Bao, G. COSMID: A Web-based Tool for Identifying and Validating CRISPR/Cas Off-target Sites. Molecular Therapy-Nucleic Acids, e214 (2014); Güell, M., Yang, L. & Church, G.M. Genome editing assessment using CRISPR Genome Analyzer (CRISPR-GA). Bioinformatics 30, 2968-2970 (2014); Prykhozhij, S.V., Rajan, V., Gaston, D. & Berman, J.N. CRISPR multitargeter: a web tool to find common and unique CRISPR single guide RNA targets in a set of similar sequences. PLoS One 10, e0119372 (2015); Montague, T.G., Cruz, J.M., Gagnon, J.A., Church, G.M. & Valen, E. CHOPCHOP: a CRISPR/Cas9 and TALEN web tool for genome editing. Nucleic Acids Res 42, W401-407 (2014); Bae, S., Kweon, J., Kim, H.S. & Kim, J.S. Microhomology-based choice of Cas9 nuclease target sites. Nat Methods 11, 705-706 (2014); Bae, S., Park, J. & Kim, J.S. Cas-OFFinder: a fast and versatile algorithm that searches for potential off-target sites of Cas9 RNA-guided endonucleases. Bioinformatics 30, 1473-1475 (2014); Stemmer, M., Thumberger, T., Del Sol Keyer, M., Wittbrodt, J. & Mateo, J.L. CCTop: An Intuitive, Flexible and Reliable CRISPR/Cas9 Target Prediction Tool. PLoS One 10, e0124633 (2015)).

### Example 98 - Testing of preferred guides in cells for on-target activity

The gRNAs predicted to have the lowest off target activity will then be tested for on-target activity in a model cell line, such as HEK293T or K562, and evaluated for indel frequency using TIDE or next generation sequencing. TIDE is a web tool to rapidly assess genome editing by CRISPR-Cas9 of a target locus determined by a guide RNA (sgRNA). Based on the quantitative sequence trace data from two standard capillary sequencing reactions, the TIDE software quantifies the editing efficacy and identifies the predominant types of insertions and deletions (indels) in the DNA of a targeted cell pool. See Brinkman et al, Nucl. Acids Res. (2014) for a detailed explanation and examples. Next-generation sequencing (NGS), also known as high-throughput sequencing, is the catch-all term used to describe a number of different modern sequencing technologies including: Illumina (Solexa) sequencing, Roche 454 sequencing, Ion torrent: Proton/PGM sequencing, and SOLiD sequencing. These recent technologies allow one to sequence DNA and RNA much more quickly and cheaply than the previously used Sanger sequencing, and as such have revolutionized the study of genomics and molecular biology.

Transfection of tissue culture cells allows screening of different constructs and a robust means of testing activity and specificity.(Cradick, T.J., Qui, P., Lee, C.M., Fine, E.J. & Bao, G. COSMID: A Web-based Tool for Identifying and Validating CRISPR/Cas Off-target Sites. Molecular Therapy-Nucleic Acids, e214 (2014)). Tissue culture cell lines, such as K-562 or HEK293T are easily transfected and result in high activity. These or other cell lines will be evaluated to determine the cell lines that match with CD34+ and provide the best surrogate. These cells will then be used for many early stage tests. For example, individual gRNAs for S. *pyogenes* Cas9 will be transfected into the cells using plasmids, which are suitable for expression in human cells. Several days later the genomic DNA is harvested and the target site amplified by PCR. The cutting activity can be measured by the rate of insertions, deletions and mutations introduced by NHEJ repair of the free DNA ends. Although this method cannot differentiate correctly repaired sequences from uncleaved DNA with this method, the level of cutting can be gauged by the amount of mis-repair. Off-target activity can be observed by amplifying identified putative off-target sites and using similar methods to detect cleavage. Translocation can also be assayed using primers flanking cut sites, to determine if specific cutting and translocations happen. Un-guided assays have been developed allowing complementary testing of off-target cleavage including guide-seq. (Kim, D. et al. Digenome-seq: genome-wide profiling of CRISPR-Cas9 off-target effects in human cells. Nat Methods 12, 237-243, 231 p following 243 (2015); Frock, R.L. et al. Genome-wide detection of DNA double-stranded breaks induced by engineered nucleases. Nat Biotechnol 33, 179-186 (2015); Wang, X. et al. Unbiased detection of off-target cleavage by CRISPR-Cas9 and TALENs using integrase-defective lentiviral vectors. Nat Biotechnol 33, 175-178 (2015); Tsai, S.Q. et al. GUIDE-seq enables genome-wide profiling of off-target cleavage by CRISPR-Cas nucleases. Nat Biotechnol (2014); Kuscu, C., Arslan, S., Singh, R., Thorpe, J. & Adli, M. Genome-wide analysis reveals characteristics of off-target sites bound by the Cas9 endonuclease. Nat Biotechnol 32, 677-683 (2014)). The gRNA or pairs of gRNA with significant activity can then be followed up in cultured cells to measure the expression level of a C9ORF72 gene. Off-target events can be followed again. Similarly CD34+ cells can be transfected and the level of gene correction and possible off-target events measured. These experiments allow optimization of nuclease and donor design and delivery.

### Example 99 - Testing of preferred guides in cells for off-target activity

The gRNAs having the best on-target activity from the TIDE and next generation sequencing studies in the above example will then be tested for off-target activity using whole genome sequencing. Candidate gRNAs will be more completely evaluated in CD34+ cells or iPSCs.

### Example 100 - Testing different approaches for HDR gene editing

After testing the gRNAs for both on-target activity and off-target activity, the mutation correction and knock-in strategies will be tested for HDR gene editing.

For the mutation correction approach, the donor DNA template will be provided as a short single-stranded oligonucleotide, a short double-stranded oligonucleotide (PAM sequence intact/PAM sequence mutated), a long single-stranded DNA molecule (PAM sequence intact/PAM sequence mutated) or a long double-stranded DNA molecule (PAM sequence intact/PAM sequence mutated). In addition, the donor DNA template will be delivered by AAV.

For some embodiments of the cDNA knock-in approach, a single-stranded or double-stranded DNA having homologous arms to a safe harbor locus (e.g., AAVS1 (PPP1R12C), ALB, Angptl3, ApoC3, ASGR2, CCR5, FIX (F9), G6PC, Gys2, HGD, Lp(a), Pcsk9, Serpina1, TF, and TTR), the 18q11.2 region, the 14q24.3 region, or the 11p15.4 region may include more than 40 nt of the first exon (the first coding exon) of the C9ORF72 gene the complete CDS of the C9ORF72 gene and 3'UTR of the C9ORF72 gene and at least 40 nt of the following intron. The single-stranded or double-stranded DNA having homologous arms to a safe harbor locus (e.g., AAVS1 (PPP1R12C), ALB, Angptl3, ApoC3, ASGR2, CCR5, FIX (F9), G6PC, Gys2, HGD, Lp(a), Pcsk9, Serpina1, TF, and TTR), the 18q11.2 region, the 14q24.3 region, or the 11p15.4 region may include more than 80 nt of the first exon of the C9ORF72 gene, the complete CDS of the C9ORF72 gene and 3'UTR of the C9ORF72 gene, and at least 80 nt of the following intron. The single-stranded or double-stranded DNA having homologous arms to a safe harbor locus (e.g., AAVS1 (PPP1R12C), ALB, Angptl3, ApoC3, ASGR2, CCR5, FIX (F9), G6PC, Gys2, HGD, Lp(a), Pcsk9, Serpina1, TF, and TTR), the 18q11.2 region, the 14q24.3 region, or the 11p15.4 region may include more than 100 nt of the first exon of the C9ORF72 gene, the complete CDS of the C9ORF72 gene and 3'UTR of the C9ORF72 gene, and at least 100 nt of the following intron. The single-stranded or double-stranded DNA having homologous arms to a safe harbor locus (e.g., AAVS1 (PPP1R12C), ALB, Angptl3, ApoC3, ASGR2, CCR5, FIX (F9), G6PC, Gys2, HGD, Lp(a), Pcsk9, Serpina1, TF, and TTR), the 18q11.2 region, the 14q24.3 region, or the 11p15.4 region may include more than 150 nt of the first exon of the C9ORF72 gene, the complete CDS of the C9ORF72 gene and 3'UTR of the C9ORF72 gene, and at least 150 nt of the following intron. The single-stranded or double-stranded DNA having homologous arms to a safe harbor locus (e.g., AAVS1 (PPP1R12C), ALB, Angptl3, ApoC3, ASGR2, CCR5, FIX (F9), G6PC, Gys2, HGD, Lp(a), Pcsk9, Serpina1, TF, and TTR), the 18q11.2 region, the 14q24.3 region, or the 11p15.4 region may include more than 300 nt of the first exon of the C9ORF72 gene, the complete CDS of the C9ORF72 gene and 3'UTR of the C9ORF72 gene, and at least 300 nt of the following intron. The single-stranded or double-stranded DNA having homologous arms to a safe harbor locus (e.g., AAVS1 (PPP1R12C), ALB, Angptl3, ApoC3, ASGR2, CCR5, FIX (F9), G6PC, Gys2, HGD, Lp(a), Pcsk9, Serpina1, TF, and TTR), the 18q11.2 region, the 14q24.3 region, or the 11p15.4 region may include more than 400 nt of the first exon of the C9ORF72 gene, the complete CDS of the C9ORF72 gene and 3'UTR of the C9ORF72 gene, and at least 400 nt of the following intron. In addition, the DNA template will be delivered by AAV.

For the cDNA or minigene knock-in approach, a single-stranded or double-stranded DNA having homologous arms to the 9p21.2, which includes more than 80 nt of the first exon (the first coding exon) of the C9ORF72 gene, the complete CDS of the C9ORF72 gene and 3'UTR of the C9ORF72 gene, and at least 80 nt of the following intron. In addition, the DNA template will be delivered by AAV.

### Example 101 - Re-assessment of lead Crispr-Cas9/DNA donor combinations

After testing the different strategies for HDR gene editing, the lead CRISPR-Cas9/DNA donor combinations will be re-assessed in primary human neurons for efficiency of deletion, recombination, and off-target specificity. Cas9 mRNA will be formulated into lipid nanoparticles for delivery, sgRNAs will be formulated into nanoparticles or delivered as AAV, and donor DNA will be formulated into nanoparticles or delivered as AAV.

### Example 102 - In vivo testing in relevant mouse model

Recently a mouse model containing the human C9ORF72 gene with either a normal hexanucleotide repeat complement or an expanded hexanucleotide repeat was generated. The Tg(C9orf72_3) line 112 mice (Jackson Laboratory, Stock No. 023099) have several tandem copies of the human C9orf72_3 transgene, with each copy having between 100-1000 repeats ([GGGGCC]100-1000). As a control, the Tg(C9orf72_2) line 8 mice (Jackson Laboratory, Stock No. 023088) have the C9orf72_2 transgene with 15 hexanucleotide repeats ([GGGGCC]15). Hemizygous mice from Tg(C9orf72_3) line 112 and Tg(C9orf72_2) line 8 are viable, fertile, born in Mendelian ratios and do not develop any locomotor or cognitive phenotype by 16 months of age.

By three months of age, however, hemizygous Tg(C9orf72_3) line 112 animals exhibit RNA foci (formed by hexanucleotide-repeat expansion-containing human C9ORF72 RNAs) and poly(GP) dipeptides (resulting from a non-ATG initiated translation of the repeat expansion) in most neuronal populations of the brain. No RNA foci or poly(GP) dipeptide phenotype is observed for hemizygous Tg(C9orf72_2) line 8. (O'Rourke (2015) Neuron Volume 88, Issue 5, p892-901, 2 December 2015 "C9orf72 BAC Transgenic Mice Display Typical Pathologic Features of ALS/FTD").

gRNA's will be tested in animals to assess their ability to alter the hexanucleotide repeat expansion in C9ORF72 and produce phenotypic changes, such as suppression or amelioration of RNA foci and repeat associated non-ATG (RAN) translated dipeptides, and suppression or amelioration of the resulting altered nucleolin distribution and nucleolar dysfunction exhibited in the hemizygous Tg(C9orf72_3) line 112 animals.

### Example 103 - In vitro transcription of C9ORF72

To identify a large spectrum of pairs of gRNAs able to edit the C9ORF72 DNA target region, an *in vitro* transcribed (IVT) gRNA screen was conducted. C9ORF72 genomic sequence was submitted for analysis using a gRNA design software. The resulting list of gRNAs were narrowed to a list of about 200 gRNAs (see Table 3) based on uniqueness of sequence (only gRNAs without a perfect match somewhere else in the genome were screened) and minimal predicted off targets.

**Table 3. gRNAs**

| **Guide Sequence (20mer)** | **Indel %** | **SEQ ID NO** |
|---|---|---|
| AGAGCAGGTG TGGGTTTAGG | 60.0 | 3330 |
| CAAGTAGTGG GGAGAGAGGG | 6.9 | 2812 |
| GAGCAAGTAG TGGGGAGAGA | 68.9 | 2811 |
| AGAGCAAGTA GTGGGGAGAG | 76.7 | 2810 |
| TACTGTGAGA GCAAGTAGTG | 55.9 | 2806 |
| GTACTGTGAG AGCAAGTAGT | 88.1 | 2805 |
| AGTACTGTGA GAGCAAGTAG | 59.5 | 2804 |
| TGCTCTCACA GTACTCGCTG | 57.5 | 3333 |
| GCTCTCACAG TACTCGCTGA | 78.4 | 3334 |
| TCTTCTGGTT AATCTTTATC | 53.0 | 2797 |
| AGATTAACCA GAAGAAAACA | 92.6 | 3341 |
| TAACCAGAAG AAAACAAGGA | 91.8 | 3344 |
| CCAGAGCTTG CTACAGGCTG | 68.3 | 2794 |
| TGAGTTCCAG AGCTTGCTAC | 71.3 | 2793 |
| CCGCAGCCTG TAGCAAGCTC | 44.3 | 3348 |
| TGTAGCAAGC TCTGGAACTC | 71.7 | 3350 |
| GAACTCAGGA GTCGCGCGCT | 69.8 | 3353 |
| AACTCAGGAG TCGCGCGCTA | 71.9 | 3354 |
| ACTCAGGAGT CGCGCGCTAG | 61.3 | 3355 |
| AGGAGTCGCG CGCTAGGGGC | 24.6 | 3356 |
| GGAGTCGCGC GCTAGGGGCC | 53.1 | 3357 |
| GAGTCGCGCG CTAGGGGCCG | 63.6 | 3358 |
| CGCGCGCTAG GGGCCGGGGC | 33.2 | 3359 |
| GGCCCGCCCC GACCACGCCC | 89.8 | 2785 |
| GGTTGCGGTG CCTGCGCCCG | 54.0 | 3385 |
| TGCGGTGCCT GCGCCCGCGG | 64.6 | 3386 |
| GGTGCCTGCG CCCGCGGCGG | 68.9 | 3387 |
| AGGCGCAGGC GGTGGCGAGT | 75.9 | 3396 |
| TGAGTGAGGA GGCGGCATCC | 85.3 | 3403 |
| GTGAGGAGGC GGCATCCTGG | 92.5 | 3404 |
| TGAGGAGGCG GCATCCTGGC | 90.4 | 3405 |
| ACCCCAAACA GCCACCCGCC | 72.7 | 2775 |
| CATCCTGGCG GGTGGCTGTT | 76.8 | 3407 |
| ATCCTGGCGG GTGGCTGTTT | 71.3 | 3408 |
| TCCTGGCGGG TGGCTGTTTG | 66.7 | 3409 |
| GCGGGTGGCT GTTTGGGGTT | 86.3 | 3410 |
| GCTGTTTGGG GTTCGGCTGC | 45.9 | 3411 |
| CTGTTTGGGG TTCGGCTGCC | 31.5 | 3412 |
| GGGGTTCGGC TGCCGGGAAG | 71.5 | 3415 |
| CTTCTACCCG CGCCTCTTCC | 90.6 | 2771 |
| TCGGCTGCCG GGAAGAGGCG | 66.2 | 3416 |
| CGGCTGCCGG GAAGAGGCGC | 61.9 | 3417 |
| GGAAGAGGCG CGGGTAGAAG | 77.2 | 3420 |
| GAAGAGGCGC GGGTAGAAGC | 80.6 | 3421 |
| AAGAGGCGCG GGTAGAAGCG | 84.2 | 3422 |
| AGAGGCGCGG GTAGAAGCGG | 68.9 | 3423 |
| TAAAAATGCG TCGAGCTCTG | 70.9 | 2768 |
| CTTCGGTCAG AGAAATGAGA | not obtainable | 2764 |
| GCTTCGGTCA GAGAAATGAG | 91.3 | 2763 |
| CTCATTTCTC TGACCGAAGC | 4.3 | 3427 |
| TCATTTCTCT GACCGAAGCT | 61.1 | 3428 |
| GAAAGCCCGA CACCCAGCTT | 81.7 | 2758 |
| CTCTGACCGA AGCTGGGTGT | 60.3 | 3429 |
| TCTGACCGAA GCTGGGTGTC | 80.5 | 3430 |
| GCAATTCCAC CAGTCGCTAG | 94.3 | 2755 |
| GGGCTTTCGC CTCTAGCGAC | 86.1 | 3432 |
| CTTTCGCCTC TAGCGACTGG | 74.9 | 3433 |
| CTGGTGGAAT TGCCTGCATC | 59.1 | 3434 |
| TGGTGGAATT GCCTGCATCC | 85.7 | 3435 |
| GAAGCCCGGG GCCCGGATGC | 27.9 | 2751 |
| AATTGCCTGC ATCCGGGCCC | 41.4 | 3436 |
| ATTGCCTGCA TCCGGGCCCC | 81.4 | 3437 |
| CCGCCGGGAA GCCCGGGGCC | 37.7 | 2749 |
| CGCCGCCGCC GGGAAGCCCG | 74.6 | 2748 |
| CCGCCGCCGC CGGGAAGCCC | 46.3 | 2747 |
| CATCCGGGCC CCGGGCTTCC | 62.3 | 3438 |
| GCCGCCGCCG CCGGGAAGCC | 21.3 | 2746 |
| CCGGGCCCCG GGCTTCCCGG | 3.1 | 3439 |
| GGCCCCGGGC TTCCCGGCGG | 74.0 | 3440 |
| CCCGGGCTTC CCGGCGGCGG | 43.2 | 3441 |
| GCGGCGGCGG CGCAGGGACA | not obtainable | 3450 |
| CGGCGGCGGC GCAGGGACAA | not obtainable | 3451 |
| GCGGCGCAGG GACAAGGGAT | 88.3 | 3453 |
| CGGCGCAGGG ACAAGGGATG | 48.7 | 3454 |
| GTACTGAGGG CGGGAAAGCA | 48.3 | 2738 |
| ACAGCTCGGG TACTGAGGGC | 26.9 | 2735 |
| GACAGCTCGG GTACTGAGGG | 69.6 | 2734 |
| GGAGACAGCT CGGGTACTGA | 89.9 | 2733 |
| AGGAGACAGC TCGGGTACTG | 73.1 | 2732 |
| CCCCGGGAAG GAGACAGCTC | 34.9 | 2730 |
| TCCCCGGGAA GGAGACAGCT | not obtainable | 2729 |
| TACCCGAGCT GTCTCCTTCC | 75.9 | 3458 |
| ACCCGAGCTG TCTCCTTCCC | 49.0 | 3459 |
| CCCGAGCTGT CTCCTTCCCG | 78.7 | 3460 |
| CTCCCAGCGG GTCCCCGGGA | 32.1 | 2726 |
| AGCGCTCCCA GCGGGTCCCC | 12.0 | 2724 |
| CAGCGCTCCC AGCGGGTCCC | 44.4 | 2723 |
| TCTCCTTCCC GGGGACCCGC | 27.7 | 3461 |
| CTCCTTCCCG GGGACCCGCT | 31.0 | 3462 |
| GCAGCGGCAG CGCTCCCAGC | 49.3 | 2722 |
| CGCAGCGGCA GCGCTCCCAG | 53.3 | 2721 |
| CGCTGGGAGC GCTGCCGCTG | 86.0 | 3464 |
| GCTGGGAGCG CTGCCGCTGC | 28.3 | 3465 |
| CCCTTTTCTC GAGCCCGCAG | 71.6 | 2718 |
| GCCGCTGCGG GCTCGAGAAA | 58.2 | 3468 |
| CCGCTGCGGG CTCGAGAAAA | 61.5 | 3469 |
| GGCTCGAGAA AAGGGAGCCT | 12.0 | 3471 |
| GCTCGAGAAA AGGGAGCCTC | 80.0 | 3472 |
| GCGAGGCCTC TCAGTACCCG | 76.4 | 2715 |
| AGGGAGCCTC GGGTACTGAG | 95.2 | 3475 |
| GGGTACTGAG AGGCCTCGCC | 10.8 | 3476 |
| GGTACTGAGA GGCCTCGCCT | 36.3 | 3477 |
| GTACTGAGAG GCCTCGCCTG | 14.2 | 3478 |
| TACTGAGAGG CCTCGCCTGG | 81.5 | 3479 |
| CTCCGGCCTT CCCCCAGGCG | 54.9 | 2712 |
| GAGAGGCCTC GCCTGGGGGA | 32.0 | 3481 |
| CCACCCTCCG GCCTTCCCCC | 73.0 | 2710 |
| GGCCTCGCCT GGGGGAAGGC | 62.6 | 3482 |
| CTCGCCTGGG GGAAGGCCGG | 18.8 | 3484 |
| TCGCCTGGGG GAAGGCCGGA | 92.7 | 3485 |
| GCCGCGCGCC GCCCACCCTC | 66.3 | 2708 |
| TGGGCGGCGC GCGGCTTCTG | 36.5 | 3490 |
| GCGGCTTCTG CGGACCAAGT | 63.8 | 3492 |
| CGGCTTCTGC GGACCAAGTC | 10.6 | 3493 |
| GGCTTCTGCG GACCAAGTCG | 78.9 | 3494 |
| GTTCCTAGCG AACCCCGACT | 67.9 | 2705 |
| GGACCAAGTC GGGGTTCGCT | 93.3 | 3496 |
| GGTTCGCTAG GAACCCGAGA | 79.6 | 3498 |
| TCGCCGGCAG GGACCGTCTC | 72.5 | 2703 |
| CTCGCCGGCA GGGACCGTCT | 47.1 | 2702 |
| GAACCCGAGA CGGTCCCTGC | 52.5 | 3499 |
| GCATGATCTC CTCGCCGGCA | 87.5 | 2701 |
| CGCATGATCT CCTCGCCGGC | 56.2 | 2700 |
| CGAGACGGTC CCTGCCGGCG | 68.1 | 3501 |
| ATCCCGCATG ATCTCCTCGC | 94.0 | 2698 |
| CTGCCGGCGA GGAGATCATG | 85.2 | 3503 |
| TGCCGGCGAG GAGATCATGC | 78.6 | 3504 |
| GGAGATCATG CGGGATGAGA | 63.1 | 3506 |
| GAGATCATGC GGGATGAGAT | 66.2 | 3507 |
| AGATCATGCG GGATGAGATG | 57.6 | 3508 |
| GATCATGCGG GATGAGATGG | 70.3 | 3509 |
| AGCTTGGGCT GAAATTGTGC | 87.1 | 2697 |
| TCACCACTCT CTAGAAGCTT | 73.9 | 2695 |
| ATCACCACTC TCTAGAAGCT | 87.6 | 2694 |
| CAGCCCAAGC TTCTAGAGAG | 48.5 | 3517 |
| GTGGTGATGA CTTGCATATG | 93.2 | 3519 |
| TGGTGATGAC TTGCATATGA | 71.5 | 3520 |
| ATGAGGGCAG CAATGCAAGT | 80.4 | 3523 |
| TCGGTGTGCT CCCCATTCTG | 84.0 | 3524 |
| AGGTCATGTC CCACAGAATG | 94.9 | 2689 |
| CGGTGTGCTC CCCATTCTGT | 77.2 | 3525 |
| CAGGTCATGT CCCACAGAAT | 63.1 | 2688 |
| CCAGGTCATG TCCCACAGAA | 83.6 | 2687 |
| CCATTCTGTG GGACATGACC | 64.7 | 3526 |
| CTCGGAGCTG TGAAGCAACC | 48.8 | 2685 |
| TAAGCAAGTC TGTGTCATCT | 92.7 | 2681 |
| GATGACACAG ACTTGCTTAA | 90.7 | 3531 |
| AGGAAGTGAC TATTGTGACT | 62.1 | 3533 |
| GGAAGTGACT ATTGTGACTT | 89.0 | 3534 |
| CTTGGGCATC ACTTGACTGA | 74.1 | 3535 |
| AGATAGACCC AATGAGCACA | 91.0 | 2673 |
| TTACATGTCC GTGTGCTCAT | 19.2 | 3540 |
| TACATGTCCG TGTGCTCATT | 48.6 | 3541 |
| GTGTGCTCAT TGGGTCTATC | 91.5 | 3542 |
| AAGCCTGGTG GTGTTCAACG | 98.5 | 2669 |
| TGGCCGCGTT GAACACCACC | 73.7 | 3544 |
| CTGTTTCTGA ATACAAAGCC | 67.0 | 2667 |
| CAGGCTTTGT ATTCAGAAAC | 62.1 | 3547 |
| GCTTTGTATT CAGAAACAGG | 84.0 | 3549 |
| TGTATTCAGA AACAGGAGGG | 38.5 | 3552 |
| CACCCCTCCT GGGAAAGTGC | 78.2 | 2665 |
| GGGAGGTCCT GCACTTTCCC | 35.7 | 3554 |
| AGGTCCTGCA CTTTCCCAGG | 88.9 | 3556 |
| GGTCCTGCAC TTTCCCAGGA | 67.5 | 3557 |
| GTCCTGCACT TTCCCAGGAG | 96.3 | 3558 |
| CTGAAAGGGC CACCCCTCCT | 47.0 | 2662 |
| TCTGAAAGGG CCACCCCTCC | 41.2 | 2661 |
| CTGCACTTTC CCAGGAGGGG | not obtainable | 3559 |
| AATCTCGATT GCATCTGAAA | not obtainable | 2660 |
| CAATCTCGAT TGCATCTGAA | not obtainable | 2659 |
| CAGATGCAAT CGAGATTGTT | not obtainable | 3563 |
| CAATCGAGAT TGTTAGGCTC | not obtainable | 3564 |
| AATCGAGATT GTTAGGCTCT | not obtainable | 3565 |
| GCTCTGGGAG AGTAGTTGCC | not obtainable | 3569 |
| GGAGAGTAGT TGCCTGGTTG | not obtainable | 3570 |
| ATTTACCAAC TGCCACAACC | not obtainable | 2654 |
| AGTTGCCTGG TTGTGGCAGT | not obtainable | 3572 |
| TGTTGTGAAC AACTGGTGCA | not obtainable | 2652 |
| GTACCCTTGT TGTGAACAAC | not obtainable | 2651 |
| TGCACCAGTT GTTCACAACA | not obtainable | 3576 |
| GCACCAGTTG TTCACAACAA | not obtainable | 3577 |
| ACAAGGGTAC GTAATCTGTC | not obtainable | 3578 |
| CAGAAACAAG ACTATCATAT | 13.1 | 3589 |
| AGAAACAAGA CTATCATATA | 65.3 | 3590 |
| GAAACAAGAC TATCATATAG | 58.4 | 3591 |
| TATAGGGGAT ATTAATAACC | 93.5 | 3592 |
| ATTTCAAGTA TTCTGACTCC | 50.2 | 2639 |
| GAGTCAGAAT ACTTGAAATA | 38.3 | 3595 |
| AAATACGGTG TCATTTGACA | 49.8 | 3596 |
| AATACGGTGT CATTTGACAC | 18.6 | 3597 |
| TGTTGTCACC ACCTCTGCCA | 72.5 | 3599 |
| TTTCCTAAAG TGGCAGGCCT | 21.4 | 2632 |

The gRNAs in Table 3 were *in vitro* transcribed, and transfected using messenger Max into HEK293T cells that constitutively express Cas9. Cells were harvested 48 hours post transfection, the genomic DNA was isolated, and cutting efficiency was evaluated using TIDE analysis. (Figure 1). It was found that about 25% of the tested gRNAs induced cutting efficiencies over 80%.

### Note Regarding Illustrative Embodiments

While the present disclosure provides descriptions of various specific aspects for the purpose of illustrating various aspects of the present invention and/or its potential applications, it is understood that variations and modifications will occur to those skilled in the art. Accordingly, the invention or inventions described herein should be understood to be at least as broad as they are claimed, and not as more narrowly defined by particular illustrative aspects provided herein.

## Claims

1. An *ex vivo* method for editing the C9ORF72 gene in a human cell by genome editing comprising introducing into the cell one or more Cas9 endonucleases and one or more guide ribonucleic acids (gRNAs) to effect one or more single-strand breaks (SSBs) or double-strand breaks (DSBs) within or near the C9ORF72 gene or other DNA sequences that encode regulatory elements of the C9ORF72 gene that results in permanent deletion or correction of an expanded hexanucleotide repeat within or near the C9ORF72 gene and results in restoration of C9ORF72 protein activity, wherein the one or more gRNAs comprise a spacer sequence selected from the group consisting of SEQ ID NOs 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558, and 3592, and wherein the gRNAs induce a cutting efficiency of 80% or higher.

2. A composition comprising one or more Cas9 endonucleases and one or more guide ribonucleic acids (gRNAs) for use in a therapeutic method for editing the C9ORF72 gene in a human cell comprising introducing the composition into the cell to effect one or more single-strand breaks (SSBs) or double-strand breaks (DSBs) within or near the C9ORF72 gene or other DNA sequences that encode regulatory elements of the C9ORF72 gene that results in permanent deletion or correction of an expanded hexanucleotide repeat within or near the C9ORF72 gene and results in restoration of C9ORF72 protein activity, wherein the one or more gRNAs comprise a spacer sequence selected from the group consisting of SEQ ID NOs 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558, and 3592, and wherein the gRNAs induce a cutting efficiency of 80% or higher.

3. The method of claim 1 or the composition for use according to claim 2, wherein the one or more Cas9 endonucleases are encoded on one or more polynucleotides.

4. The method or composition for use according to claim 3, wherein the one or more Cas9 endonucleases are encoded on one or more ribonucleic acids (RNAs).

5. The method or composition for use according to claim 3 or 4, wherein the one or more polynucleotides or one or more RNAs are one or more modified polynucleotides or one or more modified RNAs.

6. The method of claim 1 or the composition for use according to claim 2, wherein the one or more Cas9 endonucleases are proteins or polypeptides.

7. The method or composition for use according to any one of the preceding claims, wherein the one or more gRNAs are single-molecule guide RNA (sgRNAs).

8. The method or composition for use according to any one of the preceding claims, wherein the one or more gRNAs or one or more sgRNAs are one or more modified gRNAs or one or more modified sgRNAs.

9. The method or composition for use according to any one of the preceding claims, wherein the one or more Cas9 endonucleases and one or more gRNAs are either each formulated separately into lipid nanoparticles or all co-formulated into a lipid nanoparticle.

10. The method or composition for use according to any one of the preceding claims, wherein the one or more Cas9 endonucleases are formulated into a lipid nanoparticle, and the one or more gRNAs are delivered by a viral vector.

11. The method or composition for use according to claim 10, wherein the viral vector is an adeno-associated virus (AAV) vector.

12. The method or composition for use according to claim 11, wherein the AAV vector is an AAV6 vector.

13. One or more guide ribonucleic acids (gRNAs) comprising a spacer sequence selected from SEQ ID NOs: 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558, and 3592.

14. The one or more gRNAs of claim 13, wherein the one or more gRNAs are one or more single-molecule guide RNAs (sgRNAs).

15. The one or more gRNAs or sgRNAs of claim 13 or 14, wherein the one or more gRNAs or one or more sgRNAs are one or more modified gRNAs or one or more modified sgRNAs.

16. One or more gRNAs according to claims 13-15 for use in treating amyotrophic lateral sclerosis (ALS) or frontotemporal lobular dementia (FTLD) in a subject by editing the C9ORF72 gene in a cell from the subject.

## Patentansprüche

1. Ex-vivo-Verfahren zum Editieren des C9ORF72-Gens in einer menschlichen Zelle durch Genom-Editing, umfassend Einführen einer oder mehrerer Cas9-Endonukleasen und einer oder mehrerer Guide-Ribonukleinsäuren (gRNAs) in die Zelle, um einen oder mehrere Einzelstrangbrüche (Single-Strand Breaks, SSB) oder Doppelstrangbrüche (Double-Strand Breaks, DSB) in oder nahe dem C9ORF72-Gen oder anderen DNA-Sequenzen, die Regulatorelemente des C9ORF72-Gens codieren, zu bewirken, was zur permanenten Deletion oder Korrektur einer expandierten Hexanukleotid-Repeat in oder nahe dem C9ORF72-Gen und zur Wiederherstellung von C9ORF72-Protein-Aktivität führt, wobei die eine oder mehreren gRNAs eine Spacer-Sequenz ausgewählt aus der Gruppe bestehend aus SEQ ID NO 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558 und 3592 umfassen und wobei die gRNAs eine Schneideeffizienz von 80% oder höher induzieren.

2. Zusammensetzung, umfassend eine oder mehrere Cas9-Endonukleasen und eine oder mehrere Guide-Ribonukleinsäuren (gRNAs), zur Verwendung bei einem therapeutischen Verfahren zum Editieren des C9ORF72-Gens in einer menschlichen Zelle, umfassend Einführen der Zusammensetzung in die Zelle, um einen oder mehrere Einzelstrangbrüche (SSB) oder Doppelstrangbrüche (DSB) in oder nahe dem C9ORF72-Gen oder anderen DNA-Sequenzen, die Regulatorelemente des C9ORF72-Gens codieren, zu bewirken, was zur permanenten Deletion oder Korrektur einer expandierten Hexanukleotid-Repeat in oder nahe dem C9ORF72-Gen und zur Wiederherstellung von C9ORF72-Protein-Aktivität führt, wobei die eine oder mehreren gRNAs eine Spacer-Sequenz ausgewählt aus der Gruppe bestehend aus SEQ ID NO 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558 und 3592 umfassen und wobei die gRNAs eine Schneideeffizienz von 80% oder höher induzieren.

3. Verfahren nach Anspruch 1 oder Zusammensetzung zur Verwendung gemäß Anspruch 2, wobei die eine oder mehreren Cas9-Endonukleasen auf einem oder mehreren Polynukleotiden codiert sind.

4. Verfahren oder Zusammensetzung zur Verwendung gemäß Anspruch 3, wobei die eine oder mehreren Cas9-Endonukleasen auf einer oder mehreren Ribonukleinsäuren (RNAs) codiert sind.

5. Verfahren oder Zusammensetzung zur Verwendung gemäß Anspruch 3 oder 4, wobei es sich bei den ein oder mehreren Polynukleotiden bzw. eine oder mehreren RNAs um ein oder mehrere modifizierte Polynukleotide bzw. eine oder mehrere modifizierte RNAs handelt.

6. Verfahren nach Anspruch 1 oder Zusammensetzung zur Verwendung gemäß Anspruch 2, wobei es sich bei den eine oder mehreren Cas9-Endonukleasen um Proteine oder Polypeptide handelt.

7. Verfahren oder Zusammensetzung zur Verwendung gemäß einem der vorhergehenden Ansprüche, wobei es sich bei den eine oder mehreren gRNAs um Einzelmolekül-gRNAs (Single-molecule guide RNA, sgRNAs) handelt.

8. Verfahren oder Zusammensetzung zur Verwendung gemäß einem der vorhergehenden Ansprüche, wobei es sich bei den eine oder mehreren gRNAs bzw. eine oder mehreren sgRNAs um eine oder mehrere modifizierte gRNAs bzw. eine oder mehrere modifizierte sgRNAs handelt.

9. Verfahren oder Zusammensetzung zur Verwendung gemäß einem der vorhergehenden Ansprüche, wobei die eine oder mehreren Cas9-Endonukleasen und eine oder mehreren gRNAs entweder jeweils getrennt in Lipidnanopartikel oder alle zusammen in ein Lipidnanopartikel formuliert werden.

10. Verfahren oder Zusammensetzung zur Verwendung gemäß einem der vorhergehenden Ansprüche, wobei die eine oder mehreren Cas9-Endonukleasen in ein Lipidnanopartikel formuliert und die eine oder mehreren gRNAs mit einem Virusvektor zugeführt werden.

11. Verfahren oder Zusammensetzung zur Verwendung gemäß Anspruch 10, wobei es sich bei dem Virusvektor um einen AAV(Adeno-associated Virus)-Vektor handelt.

12. Verfahren oder Zusammensetzung zur Verwendung gemäß Anspruch 11, wobei es sich bei dem AAV-Vektor um einen AAV6-Vektor handelt.

13. Eine oder mehrere Guide-Ribonukleinsäuren (gRNAs), umfassend eine Spacer-Sequenz ausgewählt aus SEQ ID NO: 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558 und 3592.

14. Eine oder mehrere gRNAs nach Anspruch 13, wobei es sich bei den eine oder mehreren gRNAs um eine oder mehrere um Einzelmolekül-gRNAs (Single-molecule guide RNA, sgRNAs) handelt.

15. Eine oder mehrere gRNAs oder sgRNAs nach Anspruch 13 oder 14, wobei es sich bei den eine oder mehreren gRNAs bzw. eine oder mehreren sgRNAs um eine oder mehrere modifizierte gRNAs bzw. eine oder mehrere modifizierte sgRNAs handelt.

16. Eine oder mehrere gRNAs gemäß Anspruch 13-15 zur Verwendung bei der Behandlung von amyotropher Lateralsklerose (ALS) oder frontotemporaler lobärer Degeneration (FTLD) bei einem Individuum durch Editieren des C9ORF72-Gens in einer Zelle von dem Individuum.

## Revendications

1. Procédé *ex vivo* d'édition du gène C9ORF72 dans une cellule humaine par édition génomique comprenant l'introduction dans la cellule d'une ou plusieurs endonucléases Cas9 et d'un ou plusieurs acides ribonucléiques guides (ARNg) pour effectuer une ou plusieurs coupures simple brin (SSB) ou coupures double brin (DSB) dans ou à proximité du gène C9ORF72 ou d'autres séquences d'ADN qui codent pour des éléments régulateurs du gène C9ORF72 et conduit à la délétion ou correction permanente d'une répétition d'hexanucléotide étendue dans ou à proximité du gène C9ORF72 et conduit à la restauration de l'activité de la protéine C9ORF72, dans lequel les un ou plusieurs ARNg comprennent une séquence d'espaceur choisie dans le groupe constitué de SEQ ID NO 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558 et 3592, et dans lequel les ARNg induisent une efficacité de coupure de 80 % ou plus.

2. Composition comprenant une ou plusieurs endonucléases Cas9 et un ou plusieurs acides ribonucléiques guides (ARNg) pour utilisation dans un procédé thérapeutique pour éditer le gène C9ORF72 dans une cellule humaine comprenant l'introduction de la composition dans la cellule pour effectuer une ou plusieurs coupures simple brin (SSB) ou coupures double brin (DSB) dans ou à proximité du gène C9ORF72 ou d'autres séquences d'ADN qui codent pour des éléments régulateurs du gène C9ORF72 et conduit à la délétion ou correction permanente d'une répétition d'hexanucléotide étendue dans ou à proximité du gène C9ORF72 et conduit à la restauration de l'activité de la protéine C9ORF72, dans laquelle les un ou plusieurs ARNg comprennent une séquence d'espaceur choisie dans le groupe constitué de SEQ ID NO 2669, 2673, 2681, 2687, 2689, 2694, 2697, 2698, 2701, 2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558 et 3592, et dans laquelle les ARNg induisent une efficacité de coupure de 80 % ou plus.

3. Procédé selon la revendication 1 ou composition pour utilisation selon la revendication 2, où les une ou plusieurs endonucléases Cas9 sont codées sur un ou plusieurs polynucléotides.

4. Procédé ou composition pour utilisation selon la revendication 3, où les une ou plusieurs endonucléases Cas9 sont codées sur un ou plusieurs acides ribonucléiques (ARN).

5. Procédé ou composition pour utilisation selon la revendication 3 ou 4, où les un ou plusieurs polynucléotides ou un ou plusieurs ARN sont un ou plusieurs polynucléotides modifiés ou un ou plusieurs ARN modifiés.

6. Procédé selon la revendication 1 ou composition pour utilisation selon la revendication 2, où les une ou plusieurs endonucléases Cas9 sont des protéines ou des polypeptides.

7. Procédé ou composition pour utilisation selon l'une quelconque des revendications précédentes, où les un ou plusieurs ARNg sont des ARN guides à molécule unique (ARNgs).

8. Procédé ou composition pour utilisation selon l'une quelconque des revendications précédentes, où les un ou plusieurs ARNg ou un ou plusieurs ARNgs sont un ou plusieurs ARNg modifiés ou un ou plusieurs ARNgs modifiés.

9. Procédé ou composition pour utilisation selon l'une quelconque des revendications précédentes, où les une ou plusieurs endonucléases Cas9 et un ou plusieurs ARNg sont chacun formulés séparément dans des nanoparticules lipidiques ou tous coformulés dans une nanoparticule lipidique.

10. Procédé ou composition pour utilisation selon l'une quelconque des revendications précédentes, où les une ou plusieurs endonucléases Cas9 sont formulées dans une nanoparticule lipidique, et les un ou plusieurs ARNg sont délivrés par un vecteur viral.

11. Procédé ou composition pour utilisation selon la revendication 10, où le vecteur viral est un vecteur de virus adénoassocié (AAV).

12. Procédé ou composition pour utilisation selon la revendication 11, où le vecteur AAV est un vecteur AAV6.

13. Un ou plusieurs acides ribonucléiques guides (ARNg) comprenant une séquence d'espaceur choisie parmi SEQ ID NO : 2669, 2673, 2681, 2687, 2689, 2694, 2697, 2698, 2701,2755, 2758, 2763, 2785, 2805, 3341, 3344, 3403, 3404, 3405, 3410, 3421, 3422, 3430, 3432, 3435, 3437, 3464, 3472, 3475, 3479, 3485, 3496, 3503, 3519, 3523, 3524, 3531, 3534, 3542, 3549, 3556, 3558 et 3592.

14. Un ou plusieurs ARNg selon la revendication 13, où les un ou plusieurs ARNg sont un ou plusieurs ARN guides à molécule unique (ARNgs).

15. Un ou plusieurs ARNg ou ARNgs selon la revendication 13 ou 14, où les un ou plusieurs ARNg ou un ou plusieurs ARNgs sont un ou plusieurs ARNg modifiés ou un ou plusieurs ARNgs modifiés.

16. Un ou plusieurs ARNg selon les revendications 13 à 15 pour utilisation dans le traitement de la sclérose latérale amyotrophique (SLA) ou la démence lobaire fronto-temporale (DLFT) chez un sujet par édition du gène C9ORF72 dans une cellule du sujet.
